# EUROPEAN PATENT APPLICATION

(11) **EP 4 606 802 A1**
(43) Date of publication of application: **27.08.2025**
(21) Application number: 23879190.9
(22) Date of filing: 20.10.2023
(51) Int. Cl.: C07D 471/04, C07D 401/14, A61K 31/4353, A61K 31/495, A61P 35/00

(54) **SALT AND CRYSTAL FORM OF HETEROCYCLIC DERIVATIVE INHIBITOR, AND PREPARATION METHOD THEREFOR AND USE THEREOF**

(30) Priority: 21.10.2022 CN 202211299364
(71) Applicant: Shanghai Hansoh Biomedical Co., Ltd., Shanghai 201203 (CN); Jiangsu Hansoh Pharmaceutical Group Co., Ltd., Lianyungang, Jiangsu 222047 (CN)
(72) Inventor: ZHAN, Xiaolan, Shanghai 201203 (CN); LI, Yuanyuan, Shanghai 201203 (CN); GUO, Linsong, Shanghai 201203 (CN)
(74) Representative: Murgitroyd & Company
(86) International application number: PCT/CN2023/125513
(87) International publication number: WO 2024/083204

(57) **Abstract**

The present invention relates to a salt and a crystal form of a heterocyclic derivative inhibitor, and a preparation method therefor and the use thereof. Particularly, the present invention relates to a salt and a crystal form of the compound as represented by general formula (I), a preparation method, a pharmaceutical composition containing a therapeutically effective amount of the salt and/or crystal form, and the use thereof as an inhibitor in the treatment of cancers, wherein the definition of each substituent in general formula (I) is as defined in the description.

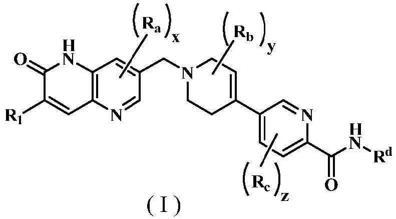

## Description

### Technical Field

The present invention belongs to the field of biomedicine and particularly relates to a salt and crystal form of a heterocyclic derivative inhibitor, a preparation method therefor, and the use thereof.

### Background Art

Poly(ADP-ribose) polymerases (PARPs) are a superfamily of proteins that catalyze ADP ribosylation of proteins in eukaryotic cells and include at least 17 protein subtypes. PARPs can catalyze the cleavage of the substrate nicotinamide adenine dinucleotide NAD+ into nicotinamide and ADP-ribose and cause polyADP-ribosylation of its target proteins. PARPs reside in the nucleus and are key enzymes for DNA damage repair in cells.

PARP1 is the earliest discovered and most extensively studied subtype of PARP, which comprises three main domains, namely, an N-terminal DNA binding region (DBD), an antomodification region (AMD), and a C-terminal catalytic region. PARP1 is an important functional protein in the process of DNA damage repair. PARP1 acts as a sensor for single-strand damage in DNA. PARP1 can be activated by DNA damage, which results in modification of its target proteins, such as histones, by poly ADP-ribosylation, and recruitment of related repair proteins to promote DNA damage repair. PARP1 is very important for the stability of a genome in a normal cell. However, in tumor therapy, the PARP1-mediated DNA repair in tumor cells damaged by radiotherapy and chemotherapy may antagonize the tumor-killing effect of the radiotherapy and chemotherapy, so PARP1 inhibitors can be developed as sensitizers for the tumor radiotherapy and chemotherapy.

Breast cancer susceptibility gene (BRCA) is an important tumor suppressor gene, which mainly has two subtypes: BRCA1 and BRCA2. BRCA plays an important role in the process of repairing double-strand breaks in DNA during homologous recombination of DNA. BRCA defects often occur in tumor cells, leading to loss of the function of repairing double-strand break damage in DNA. If the function of PARP1 is deleted or inhibited at the same time, the single-strand damage repair in DNA will also be lost, which eventually leads to the death of tumor cells and causes a "synthetic lethality" effect. Therefore, the use of a PARP1 inhibitor to block the function of repairing single-strand break damage in DNA has a selective killing effect on BRCA-deficient tumors.

PARP inhibitors have achieved great success in precise treatment in the field of tumors and have a prominent therapeutic effect, especially for tumors with BRCA mutations or defects. PARP inhibitors currently available on the market include Olaparib (AZD2281) from AstraZeneca, Rucaparib (CO-338) from Clovis, Niraparib (MK-4827) from Tesaro, and Talazoparib (BMN-673) from Pfizer. The indications are mainly ovarian cancer and breast cancer with BRCA mutations, etc., and there are also many PARP inhibitors in the clinical research phase. In the PARP family, PARP2 has the highest homology with PARP1, so most of the PARP inhibitors currently available on the market or in the clinical phase are non-selective PARP inhibitors and have a strong inhibitory effect on both PARP1 and PARP2 subtypes. Studies have shown that PARP2 plays an important role in regulating erythrocytopoiesis, and the inhibition of PARP2 is closely related to side effects, e.g., hematological toxicity such as anemia, of PARP inhibitors in clinic.

### Summary of the Invention

A patent application (Application No.: PCT/CN2022/088466) of Jiangsu Hansoh Pharmaceutical Group Co., Ltd. discloses the structures of a series of heterocyclic derivative inhibitors. In the subsequent research and development, in order to make the products easy to treat, filter, and dry, convenient to store, stable for a long time, highly bioavailable, etc., the present invention has conducted a comprehensive study on the salts and crystalline forms of the above substances and is dedicated to obtaining the most suitable crystalline form.

The full content involved in patent application PCT/CN2022/088466 is incorporated into the present invention by reference.

An object of the present invention is to provide an acid addition salt of a compound represented by general formula (I) or a stereoisomer thereof, 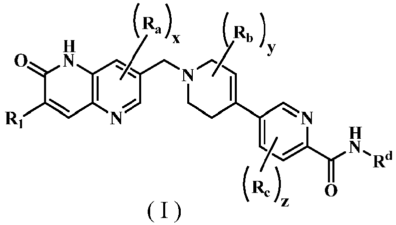 wherein
R₁ is selected from hydrogen, deuterium, halogen, nitro, hydroxyl, mercapto, cyano, C₁₋₆ alkyl, C₁₋₆ deuteroalkyl, C₁₋₆ haloalkyl, C₁₋₆ hydroxyalkyl, C₁₋₆ alkoxy, C₂₋₆ alkenyl, C₂₋₆ alkynyl, C₃₋₈ cycloalkyl, or 3- to 8-membered heterocyclyl; preferably C₁₋₃ alkyl, C₁₋₃ haloalkyl, C₂₋₄ alkynyl, or C₃₋₆ cycloalkyl;
R^{a} is independently selected from hydrogen, deuterium, halogen, nitro, hydroxyl, mercapto, cyano, C₁₋₆ alkyl, C₁₋₆ deuteroalkyl, C₁₋₆ haloalkyl, C₁₋₆ hydroxyalkyl, C₁₋₆ alkoxy, C₂₋₆ alkenyl, or C₂₋₆ alkynyl;
R^{b} is independently selected from hydrogen, deuterium, halogen, nitro, hydroxyl, mercapto, cyano, C₁₋₆ alkyl, C₁₋₆ deuteroalkyl, C₁₋₆ haloalkyl, C₁₋₆ hydroxyalkyl, C₁₋₆ alkoxy, C₂₋₆ alkenyl, or C₂₋₆ alkynyl; preferably hydrogen, deuterium, halogen, C₁₋₃ alkyl, C₁₋₃ deuteroalkyl, or C₁₋₃ haloalkyl;
R^{c} is independently selected from hydrogen, deuterium, halogen, nitro, hydroxyl, mercapto, cyano, C₁₋₆ alkyl, C₁₋₆ deuteroalkyl, C₁₋₆ haloalkyl, C₁₋₆ hydroxyalkyl, C₁₋₆ alkoxy, C₂₋₆ alkenyl, C₂₋₆ alkynyl, C₃₋₈ cycloalkyl, or 3- to 8-membered heterocyclyl; preferably selected from hydrogen, deuterium, halogen, cyano, C₁₋₃ alkyl, C₁₋₃ haloalkyl, C₁₋₃ alkoxy, C₂₋₄ alkynyl, or C₃₋₆ cycloalkyl;
R^{d} is selected from hydrogen, deuterium, halogen, nitro, hydroxyl, mercapto, cyano, C₁₋₆ alkyl, C₁₋₆ deuteroalkyl, C₁₋₆ haloalkyl, C₁₋₆ hydroxyalkyl, C₁₋₆ alkoxy, C₂₋₆ alkenyl, C₂₋₆ alkynyl, C₃₋₈ cycloalkyl, or 3- to 8-membered heterocyclyl, wherein the C₁₋₆ alkyl, C₁₋₆ deuteroalkyl, C₁₋₆ haloalkyl, C₁₋₆ hydroxyalkyl, C₁₋₆ alkoxy, C₂₋₆ alkenyl, C₂₋₆ alkynyl, C₃₋₈ cycloalkyl, or 3- to 8-membered heterocyclyl can be optionally further substituted with one or more of hydrogen, deuterium, halogen, nitro, hydroxyl, mercapto, cyano, C₁₋₃ alkyl, C₁₋₃ deuteroalkyl, C₁₋₃ haloalkyl, C₁₋₃ hydroxyalkyl, C₁₋₃ alkoxy, C₂₋₄ alkenyl, and C₂₋₄ alkynyl; R^{d} is preferably selected from cyano, C₁₋₃ alkyl, C₁₋₃ haloalkyl, C₁₋₃ alkoxy, cyano-substituted C₁₋₃ alkyl, cyano-substituted C₃₋₆ cycloalkyl, C₃₋₆ cycloalkyl, or 3- to 6-membered heterocyclyl;
x is 1, 2, or 3;
y is 1, 2, 3, or 4;
z is 1, 2, 3, or 4; and
the acid in the acid addition salt is an inorganic acid or organic acid; preferably, the inorganic acid is selected from hydrochloric acid, sulfuric acid, nitric acid, hydrobromic acid, hydrofluoric acid, hydroiodic acid, or phosphoric acid; the organic acid is selected from 2,5-dihydroxybenzoic acid, 1-hydroxy-2-naphthoic acid, acetic acid, ethanesulfonic acid, dichloroacetic acid, trichloroacetic acid, acetohydroxamic acid, adipic acid, benzenesulfonic acid, 4-chlorobenzenesulfonic acid, benzoic acid, 4-acetylaminobenzoic acid, 4-aminobenzoic acid, decanoic acid, hexanoic acid, octanoic acid, cinnamic acid, citric acid, cyclohexylsulfamic acid, camphorsulfonic acid, aspartic acid, camphanic acid, gluconic acid, glucuronic acid, glutamic acid, isoascorbic acid, lactic acid, malic acid, mandelic acid, pyroglutamic acid, tartaric acid, dodecylsulfuric acid, dibenzoyltartaric acid, ethane-1,2-disulfonic acid, ethanesulfonic acid, formic acid, fumaric acid, galactonic acid, gentisuric acid, glutaric acid, 2-ketoglutaric acid, glycolic acid, hippuric acid, hydroxyethylsulfonic acid, lactobionic acid, ascorbic acid, aspartic acid, lauric acid, camphanic acid, maleic acid, malonic acid, methanesulfonic acid, 1,5-naphthalenedisulfonic acid, naphthalene-2-sulfonic acid, nicotinic acid, oleic acid, orotic acid, oxalic acid, palmitic acid, pamoic acid, propanoic acid, salicylic acid, 4-aminosalicylic acid, sebacic acid, stearic acid, succinic acid, thiocyanic acid, undecylenoic acid, trifluoroacetic acid, benzenesulfonic acid, p-methylbenzenesulfonic acid, or L-malic acid.

In certain embodiments of the present invention, the compound is as shown below: 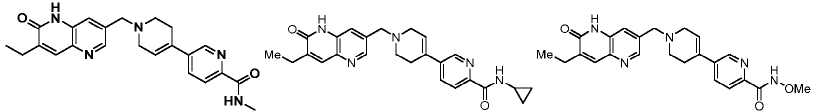 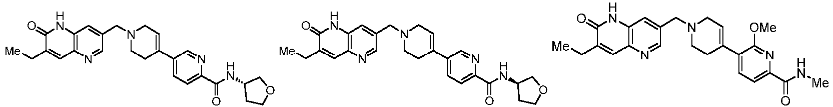 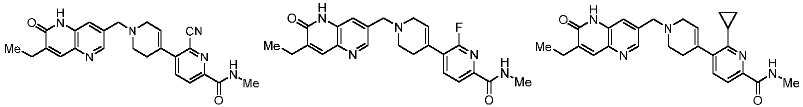 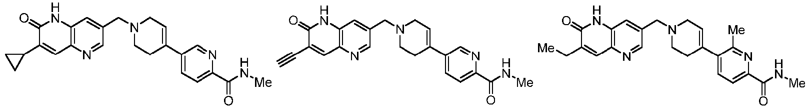 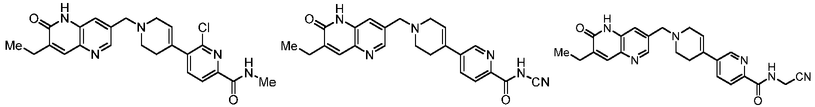 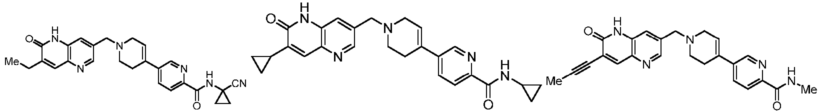 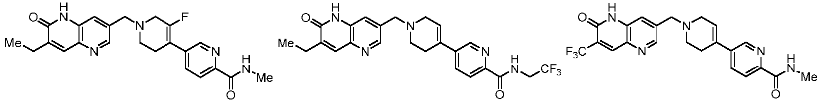 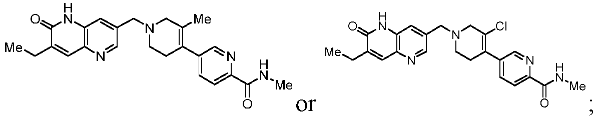 the acid in the acid addition salt is selected from hydroxyethylsulfonic acid, hydrochloric acid, sulfuric acid, 1,5-naphthalenedisulfonic acid, methanesulfonic acid, hydrobromic acid, ethanesulfonic acid, phosphoric acid, benzenesulfonic acid, oxalic acid, maleic acid, adipic acid, hydrochloric acid, citric acid, malonic acid, L-malic acid, pamoic acid, p-toluenesulfonic acid, or fumaric acid; preferably hydrochloric acid, sulfuric acid, methanesulfonic acid, hydrobromic acid, or p-toluenesulfonic acid.

In certain embodiments of the present invention, the number of acids in the acid addition salt is 0.2-3; preferably 0.2, 0.5, 1, 1.5, 2, 2.5, or 3; more preferably 0.5, 1, 2, or 3, further preferably 1.

In certain embodiments of the present invention, the acid addition salt is a hydrate or an anhydrate; when the acid addition salt is a hydrate, the water is preferably crystal water or channel water; and the number of water molecules is 0.2-3; preferably 0.2, 0.5, 1, 1.5, 2, 2.5, or 3; more preferably 2.

The present invention further provides a crystalline form of the acid addition salt of the compound or a stereoisomer thereof as described above, wherein
preferably, the crystalline form is a crystalline form of an acid addition salt of 1'-((7-ethyl-6-carbonyl-5,6-dihydro-1,5-diazanaphth-3-yl)methyl)-N-methyl-1',2',3',6'-tetrahydro-[3,4'-bipyridinyl]-6-carboxamide ( 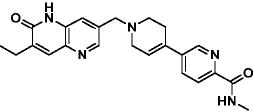 ), a crystalline form of an acid addition salt of N-cyclopropyl-1'-((7-ethyl-6-carbonyl-5,6-dihydro-1,5-diazanaphth-3-yl)methyl)-1',2',3',6'-tetrahydro-[3,4'-bipyridinyl]-6-carboxamide ( 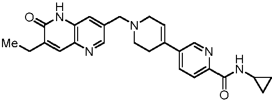 ), a crystalline form of an acid addition salt of 1'-((7-ethyl-6-carbonyl-5,6-dihydro-1,5-diazanaphth-3-yl)methyl)-2-methoxy-N-methyl-1',2',3',6'-tetrahydro-[3,4'-bipyridinyl]-6-carboxamide ( 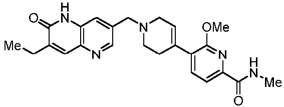 ), a crystalline form of an acid addition salt monohydrate of 1'-((7-ethyl-6-carbonyl-5,6-dihydro-1,5-diazanaphth-3-yl)methyl)-N-methyl-1',2',3',6'-tetrahydro-[3,4'-bipyridinyl]-6-carboxamide, a crystalline form of an acid addition salt monohydrate of N-cyclopropyl-1'-((7-ethyl-6-carbonyl-5,6-dihydro-1,5-diazanaphth-3-yl)methyl)-1',2',3',6'-tetrahydro-[3,4'-bipyridinyl]-6-carboxamide, a crystalline form of an acid addition salt monohydrate of 1'-((7-ethyl-6-carbonyl-5,6-dihydro-1,5-diazanaphth-3-yl)methyl)-2-methoxy-N-methyl-1',2',3',6'-tetrahydro-[3,4'-bipyridinyl]-6-carboxamide, a crystalline form of an acid addition salt dihydrate of 1'-((7-ethyl-6-carbonyl-5,6-dihydro-1,5-diazanaphth-3-yl)methyl)-N-methyl-1',2',3',6'-tetrahydro-[3,4'-bipyridinyl]-6-carboxamide, a crystalline form of an acid addition salt dihydrate of N-cyclopropyl-1'-((7-ethyl-6-carbonyl-5,6-dihydro-1,5-diazanaphth-3-yl)methyl)-1',2',3',6'-tetrahydro-[3,4'-bipyridinyl]-6-carboxamide, or a crystalline form of an acid addition salt dihydrate of 1'-((7-ethyl-6-carbonyl-5,6-dihydro-1,5-diazanaphth-3-yl)methyl)-2-methoxy-N-methyl-1',2',3',6'-tetrahydro-[3,4'-bipyridinyl]-6-carboxamide;
more preferably, the acid addition salt in the crystalline form of the acid addition salt, the crystalline form of the acid addition salt monohydrate, or the crystalline form of the acid addition salt dihydrate is a hydroxyethylsulfonate, a sulfate, a hydrochloride, a 1,5-naphthalenedisulfonate, a methanesulfonate, an ethanesulfonate, a hydrobromide, a phosphate, a benzenesulfonate, an oxalate, a maleate, an adipate, a hydrochloride, a citrate, a malonate, an L-malate, a pamoate, a p-toluenesulfonate, or a fumarate.

In certain embodiments of the present invention, the crystalline form of the acid addition salt of 1'-((7-ethyl-6-carbonyl-5,6-dihydro-1,5-diazanaphth-3-yl)methyl)-N-methyl-1',2',3',6'-tetrahydro-[3,4'-bipyridinyl]-6-carboxamide is hydrochloride crystalline Form A, sulfate crystalline Form A, methanesulfonate crystalline Form A, p-toluenesulfonate crystalline Form A, p-toluenesulfonate crystalline Form B, p-toluenesulfonate crystalline Form C, p-toluenesulfonate dihydrate crystalline Form A, and benzenesulfonate crystalline Form A.

In certain embodiments of the present invention, the X-ray powder diffraction pattern of the hydrochloride crystalline Form A has a diffraction peak at 2θ of 4.6 ± 0.2°; or has a diffraction peak at 2θ of 7.0 ± 0.2°; or has a diffraction peak at 2θ of 9.2 ± 0.2°; or has a diffraction peak at 2θ of 13.8 ± 0.2°; or has a diffraction peak at 2θ of 15.0 ± 0.2°; or has a diffraction peak at 2θ of 16.1 ± 0.2°; or has a diffraction peak at 2θ of 18.2 ± 0.2°; or has a diffraction peak at 2θ of 20.8 ± 0.2°; or has a diffraction peak at 2θ of 22.4 ± 0.2°; or has a diffraction peak at 2θ of 25.2 ± 0.2°; or has a diffraction peak at 2θ of 28.1 ± 0.2°; preferably, the X-ray powder diffraction pattern comprises any 2-5, or 3-5, or 3-6, or 3-8, or 5-8, or 6-8, or 6-9, or 8-10, or 8-11 of said peaks, more preferably, comprises any 6, 7, 8, 9, 10, or 11 of said peaks.

In certain embodiments of the present invention, the X-ray powder diffraction pattern of the hydrochloride crystalline Form A comprises at least one or more, preferably two, more preferably three of diffraction peaks at 2θ of 9.2 ± 0.2°, 13.8 ± 0.2°, and 15.0 ± 0.2°; and optionally further comprises at least one, preferably two or three of diffraction peaks at 2θ of 4.6 ± 0.2°, 7.0 ± 0.2°, and 20.8 ± 0.2°.

In certain embodiments of the present invention, the X-ray powder diffraction pattern of the hydrochloride crystalline Form A optionally further comprises one or more of diffraction peaks at 2θ of 16.1 ± 0.2°, 18.2 ± 0.2°, 22.4 ± 0.2°, 25.2 ± 0.2°, and 28.1 ± 0.2°; preferably, at least comprises any 2-3, or 4-5 of said peaks; further preferably, comprises any 2, 3, 4, or 5 of said peaks.

In certain embodiments of the present invention,the X-ray powder diffraction pattern of the hydrochloride crystalline Form A comprises one or more of diffraction peaks at 2θ of 4.6 ± 0.2°, 7.0 ± 0.2°, 9.2 ± 0.2°, 13.8 ± 0.2°, 15.0 ± 0.2°, 16.1 ± 0.2°, 18.2 ± 0.2°, 20.8 ± 0.2°, 22.4 ± 0.2°, 25.2 ± 0.2°, and 28.1 ± 0.2°; preferably, comprises any 4, 5, 6, 8, or 10 of said peaks.

In certain embodiments of the present invention, the hydrochloride crystalline Form A has X-ray characteristic diffraction peaks expressed by 2θ angle and interplanar spacing d-value by using Cu-Kα radiation, as shown in the following table:

**Table 1**

| Serial No. | XRPD data of hydrochloride crystalline Form A | | | |
|---|---|---|---|---|
| | 2θ (± 0.2°) | d -value | Peak height | Proportion (I%) |
| 1 | 4.649 | 18.9935 | 163 | 30.3 |
| 2 | 6.958 | 12.6938 | 127 | 23.6 |
| 3 | 9.209 | 9.5948 | 538 | 100 |
| 4 | 13.772 | 6.4247 | 360 | 66.9 |
| 5 | 15.009 | 5.8978 | 206 | 38.3 |
| 6 | 16.063 | 5.5131 | 74 | 13.8 |
| 7 | 18.231 | 4.8622 | 97 | 18 |
| 8 | 20.762 | 4.2748 | 128 | 23.8 |
| 9 | 22.369 | 3.9712 | 106 | 19.7 |
| 10 | 25.161 | 3.5365 | 66 | 12.3 |
| 11 | 28.124 | 3.1702 | 63 | 11.7 |

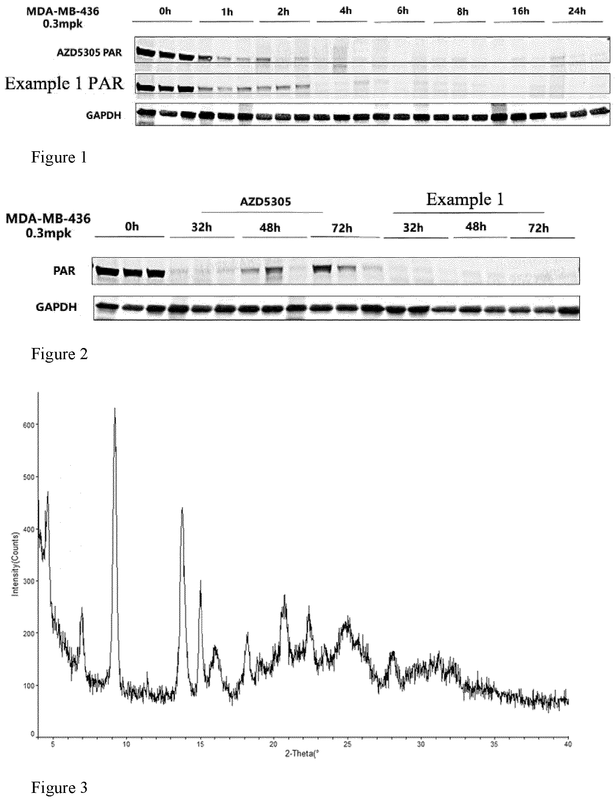

In certain embodiments of the present invention, the X-ray powder diffraction pattern of the hydrochloride crystalline Form A is substantially as shown in FIG. 3.

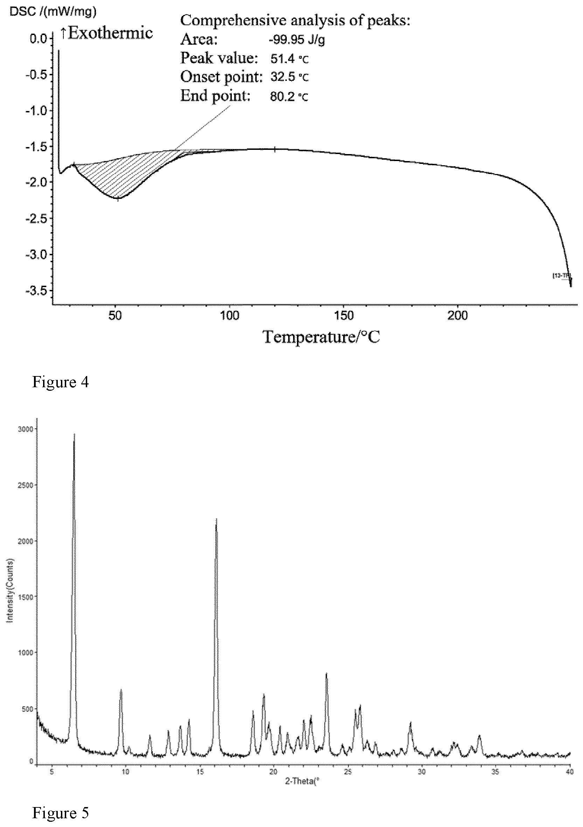

In certain embodiments of the present invention, the DSC thermogram of the hydrochloride crystalline Form A is substantially as shown in FIG. 4.

In certain embodiments of the present invention, an X-ray powder diffraction pattern of the sulfate crystalline Form A has a diffraction peak at 2θ of 6.5 ± 0.2°; or has a diffraction peak at 2θ of 9.7 ± 0.2°; or has a diffraction peak at 2θ of 14.3 ± 0.2°; or has a diffraction peak at 2θ of 16.1 ± 0.2°; or has a diffraction peak at 2θ of 18.6 ± 0.2°; or has a diffraction peak at 2θ of 19.3 ± 0.2°; or has a diffraction peak at 2θ of 19.7 ± 0.2°; or has a diffraction peak at 2θ of 22.0 ± 0.2°; or has a diffraction peak at 2θ of 22.5 ± 0.2°; or has a diffraction peak at 2θ of 23.6 ± 0.2°; or has a diffraction peak at 2θ of 25.5 ± 0.2°; or has a diffraction peak at 2θ of 25.9 ± 0.2°; or has a diffraction peak at 2θ of 29.2 ± 0.2°; preferably, the X-ray powder diffraction pattern comprises any 2-5, or 3-5, or 3-6, or 3-8, or 5-8, or 6-8, or 7-9, or 8-10, or 9-10, or 10-12, or 11-13 of said peaks, more preferably, comprises any 6, 7, 8, 9, 10, 11, 12, or 13 of said peaks.

In certain embodiments of the present invention, the X-ray powder diffraction pattern of the sulfate crystalline Form A comprises at least one or more, preferably two, more preferably three of diffraction peaks at 2θ of 6.5 ± 0.2°, 9.7 ± 0.2°, 16.1 ± 0.2°, and 23.6 ± 0.2°; and optionally further comprises at least one, preferably two or three of diffraction peaks at 2θ of 18.6 ± 0.2°, 19.3 ± 0.2°, 25.5 ± 0.2°, and 25.9 ± 0.2°.

In certain embodiments of the present invention, the X-ray powder diffraction pattern of the sulfate crystalline Form A optionally further comprises one or more of diffraction peaks at 2θ of 11.6 ± 0.2°, 12.9 ± 0.2°, 13.7 ± 0.2°, 20.4 ± 0.2°, and 20.9 ± 0.2°; preferably, at least comprises any 2-3, or 4-5 of said peaks; further preferably, comprises any 2, 3, 4, or 5 of said peaks.

In certain embodiments of the present invention, the X-ray powder diffraction pattern of the sulfate crystalline Form A comprises one or more of diffraction peaks at 2θ of 6.5 ± 0.2°, 9.7 ± 0.2°, 11.6 ± 0.2°, 12.9 ± 0.2°, 13.7 ± 0.2°, 14.3 ± 0.2°, 16.1 ± 0.2°, 18.6 ± 0.2°, 19.3 ± 0.2°, 19.7 ± 0.2°, 20.4 ± 0.2°, 20.9 ± 0.2°, 22.0 ± 0.2°, 22.5 ± 0.2°, 23.6 ± 0.2°, 25.5 ± 0.2°, 25.9 ± 0.2°, and 29.2 ± 0.2°; preferably, comprises any 4, 5, 6, 8, or 10 of said peaks.

In certain embodiments of the present invention, the sulfate crystalline Form A has X-ray characteristic diffraction peaks expressed by 2θ angle and interplanar spacing d-value by using Cu-Kα radiation, as shown in the following table:

**Table 2**

| Serial No. | XRPD data of sulfate crystalline Form A | | | |
|---|---|---|---|---|
| | 2θ (± 0.2°) | d-value | Peak height | Proportion (I%) |
| 1 | 6.493 | 13.6012 | 2798 | 100 |
| 2 | 9.677 | 9.1321 | 590 | 21.1 |
| 3 | 10.208 | 8.6584 | 78 | 2.8 |
| 4 | 11.644 | 7.5933 | 187 | 6.7 |
| 5 | 12.883 | 6.8658 | 224 | 8 |
| 6 | 13.707 | 6.4548 | 262 | 9.4 |
| 7 | 14.258 | 6.2068 | 325 | 11.6 |
| 8 | 16.103 | 5.4994 | 2125 | 75.9 |
| 9 | 18.597 | 4.7672 | 386 | 13.8 |
| 10 | 19.328 | 4.5885 | 531 | 19 |
| 11 | 19.673 | 4.5089 | 282 | 10.1 |
| 12 | 20.422 | 4.3451 | 245 | 8.8 |
| 13 | 20.927 | 4.2414 | 180 | 6.4 |
| 14 | 21.656 | 4.1003 | 113 | 4 |
| 15 | 22.024 | 4.0326 | 284 | 10.2 |
| 16 | 22.488 | 3.9504 | 297 | 10.6 |
| 17 | 23.564 | 3.7723 | 713 | 25.5 |
| 18 | 24.616 | 3.6135 | 91 | 3.3 |
| 19 | 25.511 | 3.4887 | 383 | 13.7 |
| 20 | 25.853 | 3.4434 | 426 | 15.2 |
| 21 | 26.302 | 3.3856 | 137 | 4.9 |
| 22 | 26.835 | 3.3195 | 95 | 3.4 |
| 23 | 28.084 | 3.1746 | 48 | 1.7 |
| 24 | 28.65 | 3.1132 | 49 | 1.8 |
| 25 | 29.237 | 3.0521 | 299 | 10.7 |
| 26 | 30.756 | 2.9046 | 71 | 2.5 |
| 27 | 31.188 | 2.8655 | 48 | 1.7 |
| 28 | 32.123 | 2.7841 | 120 | 4.3 |
| 29 | 32.403 | 2.7607 | 106 | 3.8 |

In certain embodiments of the present invention, the X-ray powder diffraction pattern of the sulfate crystalline Form A is substantially as shown in FIG. 5.

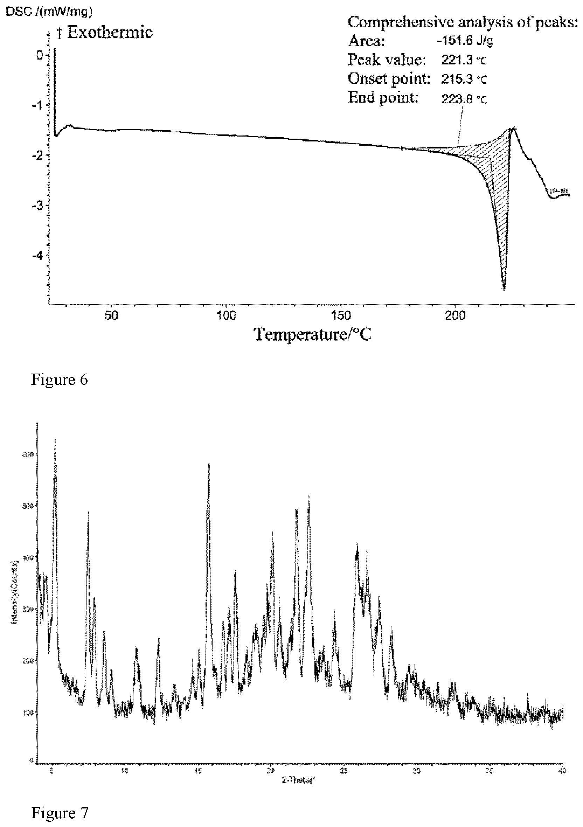

In certain embodiments of the present invention, the DSC thermogram of the sulfate crystalline Form A is substantially as shown in FIG. 6.

In certain embodiments of the present invention, an X-ray powder diffraction pattern of the methanesulfonate crystalline Form A has a diffraction peak at 2θ of 5.2 ± 0.2°; or has a diffraction peak at 2θ of 7.5 ± 0.2°; or has a diffraction peak at 2θ of 7.9 ± 0.2°; or has a diffraction peak at 2θ of 8.6 ± 0.2°; or has a diffraction peak at 2θ of 12.3 ± 0.2°; or has a diffraction peak at 2θ of 15.8 ± 0.2°; or has a diffraction peak at 2θ of 17.1 ± 0.2°; or has a diffraction peak at 2θ of 17.6 ± 0.2°; or has a diffraction peak at 2θ of 19.8 ± 0.2°; or has a diffraction peak at 2θ of 20.1 ± 0.2°; or has a diffraction peak at 2θ of 21.8 ± 0.2°; or has a diffraction peak at 2θ of 22.6 ± 0.2°; or has a diffraction peak at 2θ of 25.9 ± 0.2°; or has a diffraction peak at 2θ of 26.6 ± 0.2°; or has a diffraction peak at 2θ of 27.4 ± 0.2°; preferably, the X-ray powder diffraction pattern comprises any 2-5, or 3-5, or 3-6, or 3-8, or 5-8, or 6-8, or 7-9, or 8-10, or 9-10, or 10-12, or 11-13, or 12-14, or 14-15 of said peaks, more preferably, comprises any 6, 7, 8, 9, 10, 11, 12, 13, 14, or 15 of said peaks.

In certain embodiments of the present invention, the X-ray powder diffraction pattern of the methanesulfonate crystalline Form A comprise at least one or more, preferably two, more preferably, three of diffraction peaks at 2θ of 5.2 ± 0.2°, 7.5 ± 0.2°, 15.8 ± 0.2°, 20.1 ± 0.2°, and 22.6 ± 0.2°; and optionally further comprises at least one, preferably two or three of diffraction peaks at 2θ of 7.9 ± 0.2°, 8.6 ± 0.2°, 12.3 ± 0.2°, 17.1 ± 0.2°, 17.6 ± 0.2°, 19.8 ± 0.2°, 25.9 ± 0.2°, 26.6 ± 0.2°, and 27.4 ± 0.2°.

In certain embodiments of the present invention, the X-ray powder diffraction pattern of the methanesulfonate crystalline Form A optionally further comprises one or more of diffraction peaks at 2θ of 4.6 ± 0.2°, 9.1 ± 0.2°, 10.8 ± 0.2°, 14.6 ± 0.2°, 15.1 ± 0.2°, 16.7 ± 0.2°, 20.6 ± 0.2°, 24.4 ± 0.2°, and 28.2 ± 0.2°; preferably, at least comprises any 2-3, or 4-5 of said peaks; further preferably, comprises any 2, 3, 4, or 5 of said peaks.

In certain embodiments of the present invention, the X-ray powder diffraction pattern of the methanesulfonate crystalline Form A comprises one or more of diffraction peaks at 2θ of 5.2 ± 0.2°, 7.5 ± 0.2°, 7.9 ± 0.2°, 8.6 ± 0.2°, 10.8 ± 0.2°, 12.3 ± 0.2°, 15.8 ± 0.2°, 17.1 ± 0.2°, 17.6 ± 0.2°, 19.8 ± 0.2°, 20.1 ± 0.2°, 20.6 ± 0.2°, 22.6 ± 0.2°, 24.4 ± 0.2°, 25.9 ± 0.2°, 26.6 ± 0.2°, 27.4 ± 0.2°, and 28.2 ± 0.2°; preferably, comprises any 4, 5, 6, 8, or 10 of said peaks.

In certain embodiments of the present invention, the methanesulfonate crystalline Form A has X-ray characteristic diffraction peaks expressed by 2θ angle and interplanar spacing d-value by using Cu-Kα radiation, as shown in the following table:

**Table 3**

| Serial No. | XRPD data of methanesulfonate crystalline Form A | | | |
|---|---|---|---|---|
| | 2θ (± 0.2°) | d-value | Peak height | Proportion (I%) |
| 1 | 4.645 | 19.0095 | 83 | 18.6 |
| 2 | 5.218 | 16.9221 | 412 | 92.2 |
| 3 | 7.507 | 11.7662 | 367 | 82.1 |
| 4 | 7.913 | 11.164 | 201 | 45 |
| 5 | 8.585 | 10.2912 | 141 | 31.5 |
| 6 | 9.072 | 9.7395 | 74 | 16.6 |
| 7 | 10.752 | 8.2217 | 129 | 28.9 |
| 8 | 12.312 | 7.1829 | 136 | 30.4 |
| 9 | 14.623 | 6.0527 | 87 | 19.5 |
| 10 | 15.069 | 5.8745 | 97 | 21.7 |
| 11 | 15.756 | 5.6198 | 447 | 100 |
| 12 | 16.733 | 5.2937 | 115 | 25.7 |
| 13 | 17.137 | 5.17 | 158 | 35.3 |
| 14 | 17.583 | 5.0399 | 223 | 49.9 |
| 15 | 18.798 | 4.7167 | 72 | 16.1 |
| 16 | 19.026 | 4.6607 | 85 | 19 |
| 17 | 19.565 | 4.5335 | 98 | 21.9 |
| 18 | 19.756 | 4.49 | 150 | 33.6 |
| 19 | 20.117 | 4.4103 | 262 | 58.6 |
| 20 | 20.585 | 4.3111 | 127 | 28.4 |
| 21 | 21.35 | 4.1583 | 91 | 20.4 |
| 22 | 21.798 | 4.0738 | 309 | 69.1 |
| 23 | 22.591 | 3.9325 | 336 | 75.2 |
| 24 | 24.352 | 3.652 | 129 | 28.9 |
| 25 | 25.895 | 3.4379 | 249 | 55.7 |
| 26 | 26.565 | 3.3526 | 210 | 47 |
| 27 | 27.414 | 3.2507 | 141 | 31.5 |
| 28 | 28.224 | 3.1593 | 125 | 28 |

In certain embodiments of the present invention, the X-ray powder diffraction pattern of the methanesulfonate crystalline Form A is substantially as shown in FIG. 7.

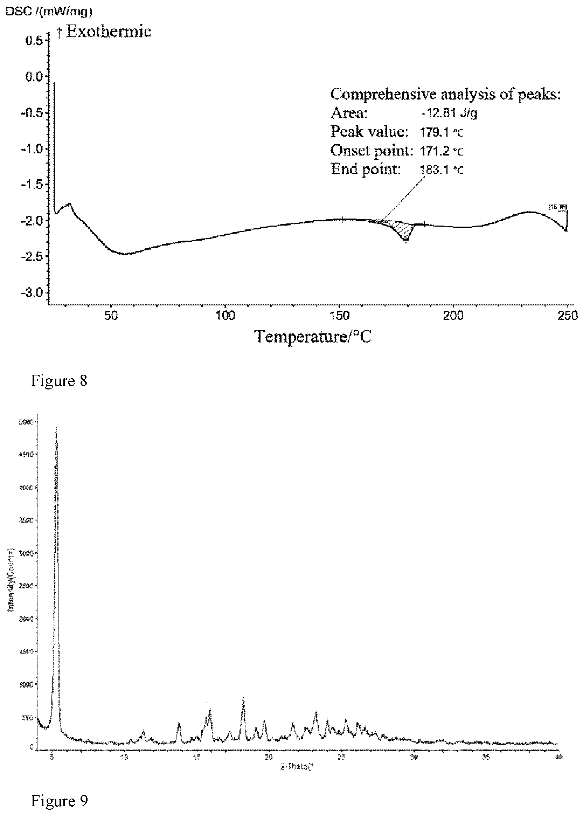

In certain embodiments of the present invention, the DSC thermogram of the methanesulfonate crystalline Form A is substantially as shown in FIG. 8.

In certain embodiments of the present invention, an X-ray powder diffraction pattern of the p-toluenesulfonate crystalline Form A has a diffraction peak at 2θ of 5.3 ± 0.2°; or has a diffraction peak at 2θ of 13.8 ± 0.2°; or has a diffraction peak at 2θ of 15.7 ± 0.2°; or has a diffraction peak at 2θ of 15.9 ± 0.2°; or has a diffraction peak at 2θ of 18.2 ± 0.2°; or has a diffraction peak at 2θ of 19.7 ± 0.2°; or has a diffraction peak at 2θ of 23.2 ± 0.2°; or has a diffraction peak at 2θ of 24.1 ± 0.2°; preferably, the X-ray powder diffraction pattern comprises any 2-5, or 3-5, or 3-6, or 3-8, or 5-8, or 6-8 of said peaks, more preferably, comprises any 6, 7, or 8 of said peaks.

In certain embodiments of the present invention, the X-ray powder diffraction pattern of the p-toluenesulfonate crystalline Form A comprises at least one or more, preferably two, more preferably three of diffraction peaks at 2θ of 5.3 ± 0.2°, 15.9 ± 0.2°, and 18.2 ± 0.2°; and optionally further comprises at least one, preferably two or three of diffraction peaks at 2θ of 13.8 ± 0.2°, 15.7 ± 0.2°, 19.7 ± 0.2°, 23.2 ± 0.2°, and 24.1 ± 0.2°.

In certain embodiments of the present invention, the X-ray powder diffraction pattern of the p-toluenesulfonate crystalline Form A optionally further comprises one or more of diffraction peaks at 2θ of 11.3 ± 0.2°, 19.1 ± 0.2°, 21.6 ± 0.2°, 25.3 ± 0.2°, and 26.1 ± 0.2°; preferably, at least comprises any 2-3, or 4-5 of said peaks; further preferably, comprises any 2, 3, 4, or 5 of said peaks.

In certain embodiments of the present invention, the X-ray powder diffraction pattern of the p-toluenesulfonate crystalline Form A comprises one or more of diffraction peaks at 2θ of 5.3 ± 0.2°, 11.3 ± 0.2°, 13.8 ± 0.2°, 15.7 ± 0.2°, 15.9 ± 0.2°, 18.2 ± 0.2°, 19.1 ± 0.2°, 19.7 ± 0.2°, 21.6 ± 0.2°, 23.2 ± 0.2°, 25.3 ± 0.2°, and 26.1 ± 0.2°; preferably, comprises any 4, 5, 6, 8, or 10 of said peaks.

In certain embodiments of the present invention, the p-toluenesulfonate crystalline Form A has X-ray characteristic diffraction peaks expressed by 2θ angle and interplanar spacing d-value by using Cu-Kα radiation, as shown in the following table:

**Table 4**

| Serial No. | XRPD data of p-toluenesulfonate crystalline Form A | | | |
|---|---|---|---|---|
| | 2θ (± 0.2°) | d-value | Peak height | Proportion (I%) |
| 1 | 5.318 | 16.6042 | 4666 | 100 |
| 2 | 11.053 | 7.998 | 95 | 2 |
| 3 | 11.3 | 7.8236 | 197 | 4.2 |
| 4 | 11.829 | 7.4754 | 74 | 1.6 |
| 5 | 13.774 | 6.4239 | 316 | 6.8 |
| 6 | 15.004 | 5.8998 | 74 | 1.6 |
| 7 | 15.675 | 5.6486 | 348 | 7.5 |
| 8 | 15.939 | 5.5556 | 472 | 10.1 |
| 9 | 17.298 | 5.1221 | 136 | 2.9 |
| 10 | 18.21 | 4.8676 | 628 | 13.5 |
| 11 | 19.084 | 4.6466 | 186 | 4 |
| 12 | 19.691 | 4.5048 | 303 | 6.5 |
| 13 | 21.636 | 4.104 | 241 | 5.2 |
| 14 | 22.509 | 3.9468 | 155 | 3.3 |
| 15 | 23.22 | 3.8276 | 393 | 8.4 |
| 16 | 24.05 | 3.6973 | 287 | 6.2 |
| 17 | 24.434 | 3.6401 | 122 | 2.6 |
| 18 | 24.82 | 3.5843 | 86 | 1.8 |
| 19 | 25.27 | 3.5214 | 233 | 5 |
| 20 | 26.102 | 3.4111 | 204 | 4.4 |
| 21 | 26.644 | 3.3429 | 170 | 3.6 |
| 22 | 27.924 | 3.1925 | 76 | 1.6 |

In certain embodiments of the present invention, the X-ray powder diffraction pattern of the p-toluenesulfonate crystalline Form A is substantially as shown in FIG. 9.

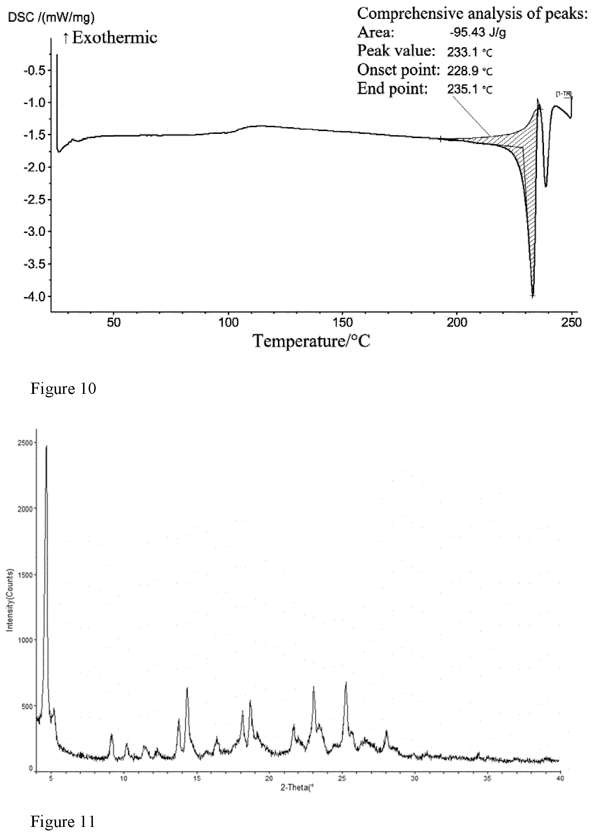

In certain embodiments of the present invention, the DSC thermogram of the p-toluenesulfonate crystalline Form A is substantially as shown in FIG. 10.

In certain embodiments of the present invention, an X-ray powder diffraction pattern of the p-toluenesulfonate crystalline Form B has a diffraction peak at 2θ of 4.7 ± 0.2°; or has a diffraction peak at 2θ of 5.2 ± 0.2°; or has a diffraction peak at 2θ of 13.8 ± 0.2°; or has a diffraction peak at 2θ of 14.3 ± 0.2°; or has a diffraction peak at 2θ of 18.1 ± 0.2°; or has a diffraction peak at 2θ of 18.7 ± 0.2°; or has a diffraction peak at 2θ of 23.1 ± 0.2°; or has a diffraction peak at 2θ of 25.3 ± 0.2°; preferably, the X-ray powder diffraction pattern comprises any 2-5, or 3-5, or 3-6, or 3-8, or 5-8, or 6-8 of said peaks, more preferably, comprises any 6, 7, or 8 of said peaks.

In certain embodiments of the present invention, the X-ray powder diffraction pattern of the p-toluenesulfonate crystalline Form B comprises at least one or more, preferably two, more preferably three of diffraction peaks at 2θ of 4.7 ± 0.2°, 14.3 ± 0.2°, 23.1 ± 0.2°, and 25.3 ± 0.2°; and optionally further comprises at least one, preferably two or three of diffraction peaks at 2θ of 5.2 ± 0.2°, 13.8 ± 0.2°, 18.1 ± 0.2°, and 18.7 ± 0.2°.

In certain embodiments of the present invention, the X-ray powder diffraction pattern of the p-toluenesulfonate crystalline Form B optionally further comprises one or more of diffraction peaks at 2θ of 9.2 ± 0.2°, 16.4 ± 0.2°, 21.7 ± 0.2°, 23.5 ± 0.2°, 25.7 ± 0.2°, and 28.1 ± 0.2°; preferably, at least comprises any 2-3, or 4-5 of said peaks; further preferably, comprises any 2, 3, 4, or 5 of said peaks.

In certain embodiments of the present invention, the X-ray powder diffraction pattern of the p-toluenesulfonate crystalline Form B comprises one or more of diffraction peaks at 2θ of 4.7 ± 0.2°, 5.2 ± 0.2°, 9.2 ± 0.2°, 13.8 ± 0.2°, 14.3 ± 0.2°, 16.4 ± 0.2°, 18.1 ± 0.2°, 18.7 ± 0.2°, 21.7 ± 0.2°, 23.1 ± 0.2°, 23.5 ± 0.2°, 25.3 ± 0.2°, 25.7 ± 0.2°, and 28.1 ± 0.2°; preferably, comprises any 4, 5, 6, 8, or 10 of said peaks.

In certain embodiments of the present invention, the p-toluenesulfonate crystalline Form B has X-ray characteristic diffraction peaks expressed by 2θ angle and interplanar spacing d-value by using Cu-Kα radiation, as shown in the following table:

**Table 5**

| Serial No. | XRPD data of p-toluenesulfonate crystalline Form B | | | |
|---|---|---|---|---|
| | 2θ (± 0.2°) | d-value | Peak height | Proportion (I%) |
| 1 | 4.688 | 18.8335 | 2291 | 100 |
| 2 | 5.197 | 16.9893 | 342 | 14.9 |
| 3 | 9.171 | 9.6351 | 193 | 8.4 |
| 4 | 10.202 | 8.6631 | 119 | 5.2 |
| 5 | 11.416 | 7.7448 | 94 | 4.1 |
| 6 | 12.332 | 7.1714 | 67 | 2.9 |
| 7 | 13.787 | 6.4175 | 254 | 11.1 |
| 8 | 14.34 | 6.1716 | 520 | 22.7 |
| 9 | 16.407 | 5.3984 | 133 | 5.8 |
| 10 | 18.133 | 4.8882 | 274 | 12 |
| 11 | 18.677 | 4.747 | 340 | 14.8 |
| 12 | 19.142 | 4.6327 | 117 | 5.1 |
| 13 | 21.658 | 4.0999 | 204 | 8.9 |
| 14 | 22.007 | 4.0356 | 106 | 4.6 |
| 15 | 23.056 | 3.8544 | 482 | 21 |
| 16 | 23.462 | 3.7885 | 208 | 9.1 |
| 17 | 25.285 | 3.5194 | 514 | 22.4 |
| 18 | 25.692 | 3.4646 | 132 | 5.8 |
| 19 | 26.387 | 3.3749 | 68 | 3 |
| 20 | 26.706 | 3.3353 | 82 | 3.6 |
| 21 | 28.065 | 3.1768 | 166 | 7.2 |
| 22 | 34.383 | 2.6061 | 54 | 2.4 |

In certain embodiments of the present invention, the X-ray powder diffraction pattern of the p-toluenesulfonate crystalline Form B is substantially as shown in FIG. 11.

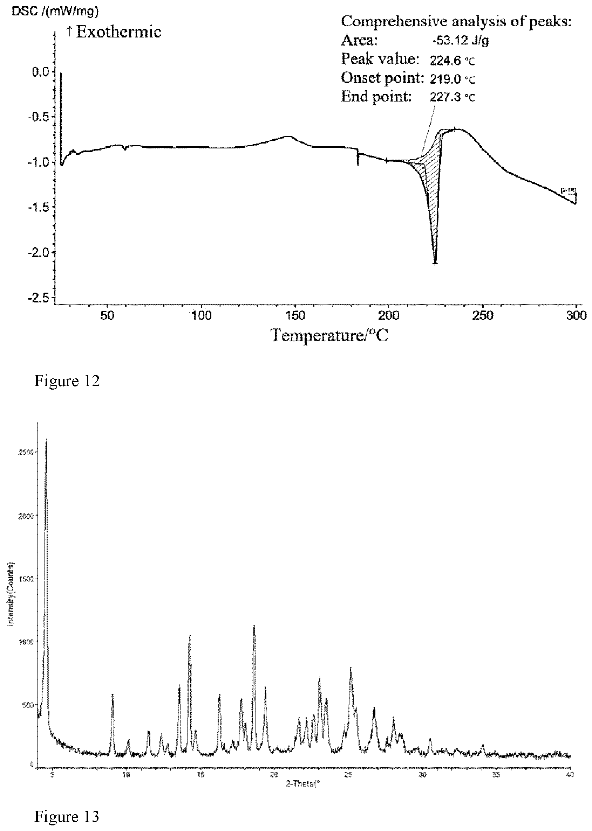

In certain embodiments of the present invention, the DSC thermogram of the p-toluenesulfonate crystalline Form B is substantially as shown in FIG. 12.

In certain embodiments of the present invention, an X-ray powder diffraction pattern of the p-toluenesulfonate crystalline Form C has a diffraction peak at 2θ of 4.6 ± 0.2°; or has a diffraction peak at 2θ of 13.6 ± 0.2°; or has a diffraction peak at 2θ of 14.3 ± 0.2°; or has a diffraction peak at 2θ of 18.6 ± 0.2°; or has a diffraction peak at 2θ of 19.4 ± 0.2°; or has a diffraction peak at 2θ of 23.1 ± 0.2°; or has a diffraction peak at 2θ of 25.2 ± 0.2°; preferably, the X-ray powder diffraction pattern comprises any 2-5, or 3-5, or 3-6, or 3-7, or 5-7, or 6-7 of said peaks, more preferably, comprises any 6 or 7 of said peaks.

In certain embodiments of the present invention, the X-ray powder diffraction pattern of the p-toluenesulfonate crystalline Form C comprises at least one or more, preferably two, more preferably three of diffraction peaks at 2θ of 4.6 ± 0.2°, 14.3 ± 0.2°, 18.6 ± 0.2°, and 25.2 ± 0.2°; and optionally further comprises at least one, preferably two or three of diffraction peaks at 2θ of 9.1 ± 0.2°, 13.6 ± 0.2°, 16.3 ± 0.2°, 19.4 ± 0.2°, and 23.1 ± 0.2°.

In certain embodiments of the present invention, the X-ray powder diffraction pattern of the p-toluenesulfonate crystalline Form C optionally further comprises one or more of diffraction peaks at 2θ of 17.7 ± 0.2°, 21.7 ± 0.2°, 23.5 ± 0.2°, 25.5 ± 0.2°, 26.7 ± 0.2°, and 28.1 ± 0.2°; preferably, at least comprises 2-3, or 4-5 of said peaks; further preferably, comprises any 2, 3, 4, or 5 of said peaks.

In certain embodiments of the present invention, the X-ray powder diffraction pattern of the p-toluenesulfonate crystalline Form C comprises one or more of diffraction peaks at 2θ of 4.6 ± 0.2°, 9.1 ± 0.2°, 13.6 ± 0.2°, 14.3 ± 0.2°, 16.3 ± 0.2°, 17.7 ± 0.2°, 18.6 ± 0.2°, 19.4 ± 0.2°, 21.7 ± 0.2°, 23.1 ± 0.2°, 23.5 ± 0.2°, 25.2 ± 0.2°, 25.5 ± 0.2°, 26.7 ± 0.2°, and 28.1 ± 0.2°; preferably, comprises any 4, 5, 6, 8, or 10 of said peaks.

In certain embodiments of the present invention, the p-toluenesulfonate crystalline Form C has X-ray characteristic diffraction peaks expressed by 2θ angle and interplanar spacing d-value by using Cu-Kα radiation, as shown in the following table:

**Table 6**

| Serial No. | XRPD data of p-toluenesulfonate crystalline Form C | | | |
|---|---|---|---|---|
| | 2θ (± 0.2°) | d-value | Peak height | Proportion (I%) |
| 1 | 4.606 | 19.1695 | 2412 | 100 |
| 2 | 9.07 | 9.742 | 480 | 19.9 |
| 3 | 10.156 | 8.7026 | 125 | 5.2 |
| 4 | 11.504 | 7.686 | 198 | 8.2 |
| 5 | 12.394 | 7.1358 | 171 | 7.1 |
| 6 | 12.817 | 6.9013 | 88 | 3.6 |
| 7 | 13.606 | 6.5029 | 538 | 22.3 |
| 8 | 14.295 | 6.1906 | 939 | 38.9 |
| 9 | 14.683 | 6.028 | 183 | 7.6 |
| 10 | 16.287 | 5.4376 | 465 | 19.3 |
| 11 | 17.14 | 5.1689 | 78 | 3.2 |
| 12 | 17.747 | 4.9937 | 408 | 16.9 |
| 13 | 18.066 | 4.9061 | 210 | 8.7 |
| 14 | 18.617 | 4.7621 | 982 | 40.7 |
| 15 | 19.389 | 4.5743 | 506 | 21 |
| 16 | 21.657 | 4.1 | 240 | 10 |
| 17 | 22.164 | 4.0074 | 221 | 9.2 |
| 18 | 22.632 | 3.9257 | 206 | 8.5 |
| 19 | 23.055 | 3.8544 | 561 | 23.3 |
| 20 | 23.503 | 3.7821 | 421 | 17.5 |
| 21 | 24.759 | 3.5929 | 202 | 8.4 |
| 22 | 25.166 | 3.5357 | 658 | 27.3 |
| 23 | 25.531 | 3.4861 | 336 | 13.9 |
| 24 | 26.747 | 3.3302 | 340 | 14.1 |
| 25 | 28.082 | 3.1749 | 266 | 11 |
| 26 | 28.593 | 3.1193 | 124 | 5.1 |
| 27 | 29.685 | 3.007 | 52 | 2.2 |
| 28 | 30.516 | 2.927 | 127 | 5.3 |
| 29 | 32.316 | 2.7679 | 52 | 2.2 |
| 30 | 34.065 | 2.6297 | 79 | 3.3 |

In certain embodiments of the present invention, the X-ray powder diffraction pattern of the p-toluenesulfonate crystalline Form C is substantially as shown in FIG. 13.

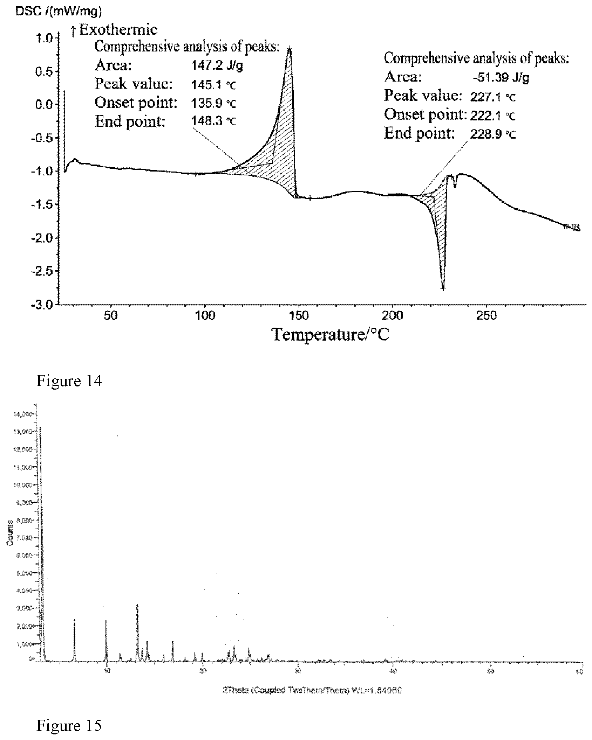

In certain embodiments of the present invention, the DSC thermogram of the p-toluenesulfonate crystalline Form C is substantially as shown in FIG. 14.

In certain embodiments of the present invention, an X-ray powder diffraction pattern of the p-toluenesulfonate dihydrate crystalline Form A has a diffraction peak at 2θ of 3.3 ± 0.2°; or has a diffraction peak at 2θ of 6.6 ± 0.2°; or has a diffraction peak at 2θ of 9.9 ± 0.2°; or has a diffraction peak at 2θ of 13.3 ± 0.2°; or has a diffraction peak at 2θ of 13.7 ± 0.2°; or has a diffraction peak at 2θ of 14.2 ± 0.2°; or has a diffraction peak at 2θ of 16.9 ± 0.2°; or has a diffraction peak at 2θ of 23.3 ± 0.2°; or has a diffraction peak at 2θ of 24.9 ± 0.2°; preferably, the X-ray powder diffraction pattern comprises any 2-5, or 3-5, or 3-6, or 3-8, or 5-8, or 6-8, or 6-9, or 7-9 of said peaks, more preferably, comprises any 6, 7, 8, or 9 of said peaks.

In certain embodiments of the present invention, the X-ray powder diffraction pattern of the p-toluenesulfonate dihydrate crystalline Form A comprises at least one or more, preferably two, more preferably three of diffraction peaks at 2θ of 3.3 ± 0.2°, 6.6 ± 0.2°, 9.9 ± 0.2°, and 13.3 ± 0.2°; and optionally further comprises at least one, preferably two, three, or four of diffraction peaks at 2θ of 13.7 ± 0.2°, 14.2 ± 0.2°, 16.9 ± 0.2°, 23.3 ± 0.2°, and 24.9 ± 0.2°; and for example, at
3.3 ± 0.2° and 6.6 ± 0.2°;
3.3 ± 0.2° and 9.9 ± 0.2°;
9.9 ± 0.2° and 13.3 ± 0.2°;
6.6 ± 0.2° and 9.9 ± 0.2°;
3.3 ± 0.2° and 13.3 ± 0.2°;
6.6 ± 0.2° and 13.3 ± 0.2°;
3.3 ± 0.2°, 6.6 ± 0.2°, and 9.9 ± 0.2°;
3.3 ± 0.2°, 6.6 ± 0.2°, and 13.3 ± 0.2°;
3.3 ± 0.2°, 9.9 ± 0.2°, and 13.3 ± 0.2°;
6.6 ± 0.2°, 9.9 ± 0.2°, and 13.3 ± 0.2°;
3.3 ± 0.2°, 6.6 ± 0.2°, and 13.7 ± 0.2°;
3.3 ± 0.2°, 9.9 ± 0.2°, and 13.7 ± 0.2°;
9.9 ± 0.2°, 13.3 ± 0.2°, and 13.7 ± 0.2°;
6.6 ± 0.2°, 9.9 ± 0.2°, and 13.7 ± 0.2°;
3.3 ± 0.2°, 13.3 ± 0.2°, and 13.7 ± 0.2°;
6.6 ± 0.2°, 13.3 ± 0.2°, and 13.7 ± 0.2°;
3.3 ± 0.2°, 6.6 ± 0.2°, and 14.2 ± 0.2°;
3.3 ± 0.2°, 9.9 ± 0.2°, and 14.2 ± 0.2°;
9.9 ± 0.2°, 13.3 ± 0.2°, and 14.2 ± 0.2°;
6.6 ± 0.2°, 9.9 ± 0.2°, and 14.2 ± 0.2°;
3.3 ± 0.2°, 13.3 ± 0.2°, and 14.2 ± 0.2°;
6.6 ± 0.2°, 13.3 ± 0.2°, and 14.2 ± 0.2°;
3.3 ± 0.2°, 6.6 ± 0.2°, and 16.9 ± 0.2°;
3.3 ± 0.2°, 9.9 ± 0.2°, and 16.9 ± 0.2°;
9.9 ± 0.2°, 13.3 ± 0.2°, and 16.9 ± 0.2°;
6.6 ± 0.2°, 9.9 ± 0.2°, and 16.9 ± 0.2°;
3.3 ± 0.2°, 13.3 ± 0.2°, and 16.9 ± 0.2°;
6.6 ± 0.2°, 13.3 ± 0.2°, and 16.9 ± 0.2°;
3.3 ± 0.2°, 6.6 ± 0.2°, and 23.3 ± 0.2°;
3.3 ± 0.2°, 9.9 ± 0.2°, and 23.3 ± 0.2°;
9.9 ± 0.2°, 13.3 ± 0.2°, and 23.3 ± 0.2°;
6.6 ± 0.2°, 9.9 ± 0.2°, and 23.3 ± 0.2°;
3.3 ± 0.2°, 13.3 ± 0.2°, and 23.3 ± 0.2°;
6.6 ± 0.2°, 13.3 ± 0.2°, and 23.3 ± 0.2°;
3.3 ± 0.2°, 6.6 ± 0.2°, and 24.9 ± 0.2°;
3.3 ± 0.2°, 9.9 ± 0.2°, and 24.9 ± 0.2°;
9.9 ± 0.2°, 13.3 ± 0.2°, and 24.9 ± 0.2°;
6.6 ± 0.2°, 9.9 ± 0.2°, and 24.9 ± 0.2°;
3.3 ± 0.2°, 13.3 ± 0.2°, and 24.9 ± 0.2°;
6.6 ± 0.2°, 13.3 ± 0.2°, and 24.9 ± 0.2°;
3.3 ± 0.2°, 6.6 ± 0.2°, and 9.9 ± 0.2°;
3.3 ± 0.2°, 6.6 ± 0.2°, and 13.3 ± 0.2°;
3.3 ± 0.2°, 9.9 ± 0.2°, and 13.3 ± 0.2°;
6.6 ± 0.2°, 9.9 ± 0.2°, and 13.3 ± 0.2°;
3.3 ± 0.2°, 6.6 ± 0.2°, 9.9 ± 0.2°, and 13.3 ± 0.2°;
3.3 ± 0.2°, 6.6 ± 0.2°, 9.9 ± 0.2°, and 13.7 ± 0.2°;
3.3 ± 0.2°, 6.6 ± 0.2°, 13.3 ± 0.2°, and 13.7 ± 0.2°;
3.3 ± 0.2°, 9.9 ± 0.2°, 13.3 ± 0.2°, and 13.7 ± 0.2°;
6.6 ± 0.2°, 9.9 ± 0.2°, 13.3 ± 0.2°, and 13.7 ± 0.2°;
3.3 ± 0.2°, 6.6 ± 0.2°, 9.9 ± 0.2°, and 14.2 ± 0.2°;
3.3 ± 0.2°, 6.6 ± 0.2°, 13.3 ± 0.2°, and 14.2 ± 0.2°;
3.3 ± 0.2°, 9.9 ± 0.2°, 13.3 ± 0.2°, and 14.2 ± 0.2°;
6.6 ± 0.2°, 9.9 ± 0.2°, 13.3 ± 0.2°, and 14.2 ± 0.2°;
3.3 ± 0.2°, 6.6 ± 0.2°, 9.9 ± 0.2°, and 16.9 ± 0.2°;
3.3 ± 0.2°, 6.6 ± 0.2°, 13.3 ± 0.2°, and 16.9 ± 0.2°;
3.3 ± 0.2°, 9.9 ± 0.2°, 13.3 ± 0.2°, and 16.9 ± 0.2°;
6.6 ± 0.2°, 9.9 ± 0.2°, 13.3 ± 0.2°, and 16.9 ± 0.2°;
3.3 ± 0.2°, 6.6 ± 0.2°, 9.9 ± 0.2°, and 23.3 ± 0.2°;
3.3 ± 0.2°, 6.6 ± 0.2°, 13.3 ± 0.2°, and 23.3 ± 0.2°;
3.3 ± 0.2°, 9.9 ± 0.2°, 13.3 ± 0.2°, and 23.3 ± 0.2°;
6.6 ± 0.2°, 9.9 ± 0.2°, 13.3 ± 0.2°, and 23.3 ± 0.2°;
3.3 ± 0.2°, 6.6 ± 0.2°, 9.9 ± 0.2°, and 24.9 ± 0.2°;
3.3 ± 0.2°, 6.6 ± 0.2°, 13.3 ± 0.2°, and 24.9 ± 0.2°;
3.3 ± 0.2°, 9.9 ± 0.2°, 13.3 ± 0.2°, and 24.9 ± 0.2°;
6.6 ± 0.2°, 9.9 ± 0.2°, 13.3 ± 0.2°, and 24.9 ± 0.2°;
3.3 ± 0.2°, 6.6 ± 0.2°, 9.9 ± 0.2°, 13.3 ± 0.2°, and 13.7 ± 0.2°;
3.3 ± 0.2°, 6.6 ± 0.2°, 9.9 ± 0.2°, 13.3 ± 0.2°, and 14.2 ± 0.2°;
3.3 ± 0.2°, 6.6 ± 0.2°, 9.9 ± 0.2°, 13.3 ± 0.2°, and 16.9 ± 0.2°;
3.3 ± 0.2°, 6.6 ± 0.2°, 9.9 ± 0.2°, 13.3 ± 0.2°, and 23.3 ± 0.2°;
3.3 ± 0.2°, 6.6 ± 0.2°, 9.9 ± 0.2°, 13.3 ± 0.2°, and 24.9 ± 0.2°;
3.3 ± 0.2°, 6.6 ± 0.2°, 9.9 ± 0.2°, 13.7 ± 0.2°, and 14.2 ± 0.2°;
3.3 ± 0.2°, 6.6 ± 0.2°, 13.3 ± 0.2°, 13.7 ± 0.2°, and 24.9 ± 0.2°;
3.3 ± 0.2°, 9.9 ± 0.2°, 13.3 ± 0.2°, 16.9 ± 0.2°, and 23.3 ± 0.2°;
6.6 ± 0.2°, 9.9 ± 0.2°, 13.3 ± 0.2°, 14.2 ± 0.2°, and 23.3 ± 0.2°;
3.3 ± 0.2°, 6.6 ± 0.2°, 9.9 ± 0.2°, 13.3 ± 0.2°, 13.7 ± 0.2°, and 14.2 ± 0.2°;
3.3 ± 0.2°, 6.6 ± 0.2°, 9.9 ± 0.2°, 13.3 ± 0.2°, 23.3 ± 0.2°, and 24.9 ± 0.2°;
3.3 ± 0.2°, 6.6 ± 0.2°, 9.9 ± 0.2°, 13.3 ± 0.2°, 13.7 ± 0.2°, and 24.9 ± 0.2°;
3.3 ± 0.2°, 6.6 ± 0.2°, 9.9 ± 0.2°, 13.3 ± 0.2°, 14.2 ± 0.2°, and 16.9 ± 0.2°;
3.3 ± 0.2°, 6.6 ± 0.2°, 9.9 ± 0.2°, 13.3 ± 0.2°, 14.2 ± 0.2°, and 23.3 ± 0.2°;
3.3 ± 0.2°, 6.6 ± 0.2°, 9.9 ± 0.2°, 13.3 ± 0.2°, 16.9 ± 0.2°, and 24.9 ± 0.2°;
3.3 ± 0.2°, 6.6 ± 0.2°, 9.9 ± 0.2°, 13.7 ± 0.2°, 14.2 ± 0.2°, and 16.9 ± 0.2°;
3.3 ± 0.2°, 6.6 ± 0.2°, 13.3 ± 0.2°, 16.9 ± 0.2°, 23.3 ± 0.2°, and 24.9 ± 0.2°;
3.3 ± 0.2°, 9.9 ± 0.2°, 13.3 ± 0.2°, 13.7 ± 0.2°, 23.3 ± 0.2°, and 24.9 ± 0.2°;
6.6 ± 0.2°, 9.9 ± 0.2°, 13.3 ± 0.2°, 14.2 ± 0.2°, 16.9 ± 0.2°, and 23.3 ± 0.2°;
3.3 ± 0.2°, 6.6 ± 0.2°, 9.9 ± 0.2°, 13.3 ± 0.2°, 14.2 ± 0.2°, 16.9 ± 0.2°, 23.3 ± 0.2°, and 24.9 ± 0.2°;
3.3 ± 0.2°, 6.6 ± 0.2°, 9.9 ± 0.2°, 13.3 ± 0.2°, 13.7 ± 0.2°, 16.9 ± 0.2°, 23.3 ± 0.2°, and 24.9 ± 0.2°;
3.3 ± 0.2°, 6.6 ± 0.2°, 9.9 ± 0.2°, 13.3 ± 0.2°, 13.7 ± 0.2°, 14.2 ± 0.2°, 23.3 ± 0.2°, and 24.9 ± 0.2°;
3.3 ± 0.2°, 6.6 ± 0.2°, 9.9 ± 0.2°, 13.3 ± 0.2°, 13.7 ± 0.2°, 14.2 ± 0.2°, 16.9 ± 0.2°, and 24.9 ± 0.2°;
3.3 ± 0.2°, 6.6 ± 0.2°, 9.9 ± 0.2°, 13.3 ± 0.2°, 13.7 ± 0.2°, 14.2 ± 0.2°, 16.9 ± 0.2°, and 23.3 ± 0.2°;
3.3 ± 0.2°, 6.6 ± 0.2°, 9.9 ± 0.2°, 13.7 ± 0.2°, 14.2 ± 0.2°, 16.9 ± 0.2°, 23.3 ± 0.2°, and 24.9 ± 0.2°;
3.3 ± 0.2°, 6.6 ± 0.2°, 13.3 ± 0.2°, 13.7 ± 0.2°, 14.2 ± 0.2°, 16.9 ± 0.2°, 23.3 ± 0.2°, and 24.9 ± 0.2°;
3.3 ± 0.2°, 9.9 ± 0.2°, 13.3 ± 0.2°, 13.7 ± 0.2°, 14.2 ± 0.2°, 16.9 ± 0.2°, 23.3 ± 0.2°, and 24.9 ± 0.2°; and
6.6 ± 0.2°, 9.9 ± 0.2°, 13.3 ± 0.2°, 13.7 ± 0.2°, 14.2 ± 0.2°, 16.9 ± 0.2°, 23.3 ± 0.2°, and 24.9 ± 0.2°.

In certain embodiments of the present invention, the X-ray powder diffraction pattern of the p-toluenesulfonate dihydrate crystalline Form A optionally further comprises one or more of diffraction peaks at 2θ of 11.4 ± 0.2°, 14.4 ± 0.2°, 19.2 ± 0.2°, 20.0 ± 0.2°, 22.7 ± 0.2°, 22.9 ± 0.2°, and 26.9 ± 0.2°; preferably, at least comprises any 2-3, or 4-5 of said peaks; further preferably, comprises any 2, 3, 4, or 5 of said peaks; and for example, at
11.4 ± 0.2° and 14.4 ± 0.2°;
11.4 ± 0.2° and 26.9 ± 0.2°;
19.2 ± 0.2° and 20.0 ± 0.2°;
14.4 ± 0.2° and 19.2 ± 0.2°;
14.4 ± 0.2° and 22.7 ± 0.2°;
22.9 ± 0.2° and 26.9 ± 0.2°;
11.4 ± 0.2°, 14.4 ± 0.2°, and 19.2 ± 0.2°;
22.7 ± 0.2°, 22.9 ± 0.2°, and 26.9 ± 0.2°;
19.2 ± 0.2°, 20.0 ± 0.2°, and 22.7 ± 0.2°;
14.4 ± 0.2°, 19.2 ± 0.2°, and 20.0 ± 0.2°;
20.0 ± 0.2°, 22.7 ± 0.2°, and 22.9 ± 0.2°;
22.7 ± 0.2°, 22.9 ± 0.2°, and 26.9 ± 0.2°;
14.4 ± 0.2°, 20.0 ± 0.2°, and 22.7 ± 0.2°;
11.4 ± 0.2°, 14.4 ± 0.2°, 19.2 ± 0.2°, and 20.0 ± 0.2°;
20.0 ± 0.2°, 22.7 ± 0.2°, 22.9 ± 0.2°, and 26.9 ± 0.2°;
11.4 ± 0.2°, 22.7 ± 0.2°, 22.9 ± 0.2°, and 26.9 ± 0.2°;
11.4 ± 0.2°, 14.4 ± 0.2°, 22.9 ± 0.2°, and 26.9 ± 0.2°;
11.4 ± 0.2°, 14.4 ± 0.2°, 19.2 ± 0.2°, and 26.9 ± 0.2°;
14.4 ± 0.2°, 19.2 ± 0.2°, 20.0 ± 0.2°, and 22.7 ± 0.2°;
19.2 ± 0.2°, 20.0 ± 0.2°, 22.7 ± 0.2°, and 22.9 ± 0.2°;
20.0 ± 0.2°, 22.7 ± 0.2°, 22.9 ± 0.2°, and 26.9 ± 0.2°;
11.4 ± 0.2°, 14.4 ± 0.2°, 19.2 ± 0.2°, 20.0 ± 0.2°, and 22.7 ± 0.2°;
11.4 ± 0.2°, 14.4 ± 0.2°, 19.2 ± 0.2°, 20.0 ± 0.2°, and 26.9 ± 0.2°;
11.4 ± 0.2°, 14.4 ± 0.2°, 19.2 ± 0.2°, 22.9 ± 0.2°, and 26.9 ± 0.2°;
11.4 ± 0.2°, 14.4 ± 0.2°, 22.7 ± 0.2°, 22.9 ± 0.2°, and 26.9 ± 0.2°;
11.4 ± 0.2°, 20.0 ± 0.2°, 22.7 ± 0.2°, 22.9 ± 0.2°, and 26.9 ± 0.2°;
19.2 ± 0.2°, 20.0 ± 0.2°, 22.7 ± 0.2°, 22.9 ± 0.2°, and 26.9 ± 0.2°;
14.4 ± 0.2°, 19.2 ± 0.2°, 20.0 ± 0.2°, 22.7 ± 0.2°, and 22.9 ± 0.2°;
11.4 ± 0.2°, 19.2 ± 0.2°, 20.0 ± 0.2°, 22.7 ± 0.2°, and 26.9 ± 0.2°;
11.4 ± 0.2°, 19.2 ± 0.2°, 20.0 ± 0.2°, 22.7 ± 0.2°, and 22.9 ± 0.2°;
11.4 ± 0.2°, 14.4 ± 0.2°, 20.0 ± 0.2°, 22.7 ± 0.2°, and 26.9 ± 0.2°;
14.4 ± 0.2°, 19.2 ± 0.2°, 22.7 ± 0.2°, 22.9 ± 0.2°, and 26.9 ± 0.2°; and
14.4 ± 0.2°, 19.2 ± 0.2°, 20.0 ± 0.2°, 22.7 ± 0.2°, and 26.9 ± 0.2°.

In certain embodiments of the present invention, the X-ray powder diffraction pattern of the p-toluenesulfonate dihydrate crystalline Form A comprises one or more of diffraction peaks at 2θ of 3.3 ± 0.2°, 6.6 ± 0.2°, 9.9 ± 0.2°, 11.4 ± 0.2°, 13.3 ± 0.2°, 13.7 ± 0.2°, 14.2 ± 0.2°, 14.4 ± 0.2°, 16.9 ± 0.2°, 19.2 ± 0.2°, 20.0 ± 0.2°, 22.7 ± 0.2°, 22.9 ± 0.2°, 23.3 ± 0.2°, 24.9 ± 0.2°, and 26.9 ± 0.2°; preferably, comprises any 4, 5, 6, 8, or 10 of said peaks; for example, the X-ray powder diffraction pattern of the p-toluenesulfonate dihydrate crystalline Form A has diffraction peaks at the following 2θ positions:
3.3 ± 0.2°, 6.6 ± 0.2°, 9.9 ± 0.2°, and 11.4 ± 0.2°;
3.3 ± 0.2°, 6.6 ± 0.2°, 13.3 ± 0.2°, and 14.4 ± 0.2°;
3.3 ± 0.2°, 9.9 ± 0.2°, 13.3 ± 0.2°, and 19.2 ± 0.2°;
6.6 ± 0.2°, 9.9 ± 0.2°, 13.3 ± 0.2°, and 20.0 ± 0.2°;
3.3 ± 0.2°, 6.6 ± 0.2°, 13.7 ± 0.2°, and 22.7 ± 0.2°;
3.3 ± 0.2°, 9.9 ± 0.2°, 13.7 ± 0.2°, and 22.9 ± 0.2°;
9.9 ± 0.2°, 13.3 ± 0.2°, 13.7 ± 0.2°, and 26.9 ± 0.2°;
6.6 ± 0.2°, 9.9 ± 0.2°, 11.4 ± 0.2°, and 13.7 ± 0.2°;
3.3 ± 0.2°, 13.3 ± 0.2°, 13.7 ± 0.2°, and 14.4 ± 0.2°;
6.6 ± 0.2°, 13.3 ± 0.2°, 13.7 ± 0.2°, and 19.2 ± 0.2°;
3.3 ± 0.2°, 6.6 ± 0.2°, 14.2 ± 0.2°, and 20.0 ± 0.2°;
3.3 ± 0.2°, 9.9 ± 0.2°, 14.2 ± 0.2°, and 22.7 ± 0.2°;
9.9 ± 0.2°, 13.3 ± 0.2°, 14.2 ± 0.2°, and 22.9 ± 0.2°;
6.6 ± 0.2°, 9.9 ± 0.2°, 14.2 ± 0.2°, and 26.9 ± 0.2°;
3.3 ± 0.2°, 11.4 ± 0.2°, 13.3 ± 0.2°, and 14.2 ± 0.2°;
6.6 ± 0.2°, 13.3 ± 0.2°, 14.2 ± 0.2°, and 14.4 ± 0.2°;
3.3 ± 0.2°, 6.6 ± 0.2°, 16.9 ± 0.2°, and 19.2 ± 0.2°;
3.3 ± 0.2°, 9.9 ± 0.2°, 16.9 ± 0.2°, and 20.0 ± 0.2°;
9.9 ± 0.2°, 13.3 ± 0.2°, 16.9 ± 0.2°, and 22.7 ± 0.2°;
6.6 ± 0.2°, 9.9 ± 0.2°, 16.9 ± 0.2°, and 22.9 ± 0.2°;
3.3 ± 0.2°, 13.3 ± 0.2°, 16.9 ± 0.2°, and 26.9 ± 0.2°;
3.3 ± 0.2°, 6.6 ± 0.2°, 9.9 ± 0.2°, 11.4 ± 0.2°, and 13.3 ± 0.2°;
3.3 ± 0.2°, 6.6 ± 0.2°, 9.9 ± 0.2°, 13.7 ± 0.2°, and 14.4 ± 0.2°;
3.3 ± 0.2°, 6.6 ± 0.2°, 13.3 ± 0.2°, 13.7 ± 0.2°, and 19.2 ± 0.2°;
3.3 ± 0.2°, 9.9 ± 0.2°, 13.3 ± 0.2°, 13.7 ± 0.2°, and 20.0 ± 0.2°;
6.6 ± 0.2°, 9.9 ± 0.2°, 13.3 ± 0.2°, 13.7 ± 0.2°, and 22.7 ± 0.2°;
3.3 ± 0.2°, 6.6 ± 0.2°, 9.9 ± 0.2°, 14.2 ± 0.2°, and 22.9 ± 0.2°;
3.3 ± 0.2°, 6.6 ± 0.2°, 13.3 ± 0.2°, 14.2 ± 0.2°, and 26.9 ± 0.2°;
3.3 ± 0.2°, 9.9 ± 0.2°, 11.4 ± 0.2°, 13.3 ± 0.2°, and 14.2 ± 0.2°;
6.6 ± 0.2°, 9.9 ± 0.2°, 13.3 ± 0.2°, 14.2 ± 0.2°, and 14.4 ± 0.2°;
3.3 ± 0.2°, 6.6 ± 0.2°, 9.9 ± 0.2°, 16.9 ± 0.2°, and 19.2 ± 0.2°;
3.3 ± 0.2°, 6.6 ± 0.2°, 13.3 ± 0.2°, 16.9 ± 0.2°, and 20.0 ± 0.2°;
3.3 ± 0.2°, 9.9 ± 0.2°, 13.3 ± 0.2°, 16.9 ± 0.2°, and 22.7 ± 0.2°;
6.6 ± 0.2°, 9.9 ± 0.2°, 13.3 ± 0.2°, 16.9 ± 0.2°, and 22.9 ± 0.2°;
3.3 ± 0.2°, 6.6 ± 0.2°, 9.9 ± 0.2°, 23.3 ± 0.2°, and 26.9 ± 0.2°;
3.3 ± 0.2°, 6.6 ± 0.2°, 11.4 ± 0.2°, 13.3 ± 0.2°, and 23.3 ± 0.2°;
3.3 ± 0.2°, 9.9 ± 0.2°, 13.3 ± 0.2°, 14.4 ± 0.2°, and 23.3 ± 0.2°;
6.6 ± 0.2°, 9.9 ± 0.2°, 13.3 ± 0.2°, 19.2 ± 0.2°, and 23.3 ± 0.2°;
3.3 ± 0.2°, 6.6 ± 0.2°, 9.9 ± 0.2°, 20.0 ± 0.2°, and 24.9 ± 0.2°;
3.3 ± 0.2°, 6.6 ± 0.2°, 13.3 ± 0.2°, 22.7 ± 0.2°, and 24.9 ± 0.2°;
3.3 ± 0.2°, 9.9 ± 0.2°, 13.3 ± 0.2°, 22.9 ± 0.2°, and 24.9 ± 0.2°;
6.6 ± 0.2°, 9.9 ± 0.2°, 13.3 ± 0.2°, 26.9 ± 0.2°, and 24.9 ± 0.2°;
3.3 ± 0.2°, 6.6 ± 0.2°, 9.9 ± 0.2°, 11.4 ± 0.2°, 13.3 ± 0.2°, and 13.7 ± 0.2°;
3.3 ± 0.2°, 6.6 ± 0.2°, 9.9 ± 0.2°, 13.3 ± 0.2°, 14.2 ± 0.2°, and 14.4 ± 0.2°;
3.3 ± 0.2°, 6.6 ± 0.2°, 9.9 ± 0.2°, 13.3 ± 0.2°, 16.9 ± 0.2°, and 19.2 ± 0.2°;
3.3 ± 0.2°, 6.6 ± 0.2°, 9.9 ± 0.2°, 13.3 ± 0.2°, 20.0 ± 0.2°, and 23.3 ± 0.2°;
3.3 ± 0.2°, 6.6 ± 0.2°, 9.9 ± 0.2°, 13.3 ± 0.2°, 22.7 ± 0.2°, and 24.9 ± 0.2°;
3.3 ± 0.2°, 6.6 ± 0.2°, 9.9 ± 0.2°, 13.7 ± 0.2°, 14.2 ± 0.2°, and 22.9 ± 0.2°;
3.3 ± 0.2°, 6.6 ± 0.2°, 13.3 ± 0.2°, 13.7 ± 0.2°, 24.9 ± 0.2°, and 26.9 ± 0.2°;
3.3 ± 0.2°, 9.9 ± 0.2°, 13.3 ± 0.2°, 14.4 ± 0.2°, 16.9 ± 0.2°, and 23.3 ± 0.2°;
6.6 ± 0.2°, 9.9 ± 0.2°, 13.3 ± 0.2°, 14.2 ± 0.2°, 19.2 ± 0.2°, and 23.3 ± 0.2°;
3.3 ± 0.2°, 6.6 ± 0.2°, 9.9 ± 0.2°, 11.4 ± 0.2°, 13.3 ± 0.2°, 13.7 ± 0.2°, 14.2 ± 0.2°, and 14.4 ± 0.2°;
3.3 ± 0.2°, 6.6 ± 0.2°, 9.9 ± 0.2°, 13.3 ± 0.2°, 14.4 ± 0.2°, 19.2 ± 0.2°, 23.3 ± 0.2°, and 24.9 ± 0.2°;
3.3 ± 0.2°, 6.6 ± 0.2°, 9.9 ± 0.2°, 13.3 ± 0.2°, 13.7 ± 0.2°, 19.2 ± 0.2°, 20.0 ± 0.2°, and 24.9 ± 0.2°;
3.3 ± 0.2°, 6.6 ± 0.2°, 9.9 ± 0.2°, 13.3 ± 0.2°, 14.2 ± 0.2°, 16.9 ± 0.2°, 20.0 ± 0.2°, and 22.7 ± 0.2°;
3.3 ± 0.2°, 6.6 ± 0.2°, 9.9 ± 0.2°, 13.3 ± 0.2°, 14.2 ± 0.2°, 22.7 ± 0.2°, 22.9 ± 0.2°, and 23.3 ± 0.2°;
3.3 ± 0.2°, 6.6 ± 0.2°, 9.9 ± 0.2°, 13.3 ± 0.2°, 16.9 ± 0.2°, 22.9 ± 0.2°, 24.9 ± 0.2°, and 26.9 ± 0.2°;
3.3 ± 0.2°, 6.6 ± 0.2°, 9.9 ± 0.2°, 11.4 ± 0.2°, 13.7 ± 0.2°, 14.2 ± 0.2°, 16.9 ± 0.2°, and 19.2 ± 0.2°;
3.3 ± 0.2°, 6.6 ± 0.2°, 13.3 ± 0.2°, 14.4 ± 0.2°, 16.9 ± 0.2°, 20.0 ± 0.2°, 23.3 ± 0.2°, and 24.9 ± 0.2°;
3.3 ± 0.2°, 9.9 ± 0.2°, 13.3 ± 0.2°, 13.7 ± 0.2°, 19.2 ± 0.2°, 22.7 ± 0.2°, 23.3 ± 0.2°, and 24.9 ± 0.2°;
6.6 ± 0.2°, 9.9 ± 0.2°, 13.3 ± 0.2°, 14.2 ± 0.2°, 16.9 ± 0.2°, 20.0 ± 0.2°, 22.9 ± 0.2°, and 23.3 ± 0.2°;
3.3 ± 0.2°, 6.6 ± 0.2°, 9.9 ± 0.2°, 11.4 ± 0.2°, 13.3 ± 0.2°, 14.2 ± 0.2°, 14.4 ± 0.2°, 16.9 ± 0.2°, 23.3 ± 0.2°, and 24.9 ± 0.2°;
3.3 ± 0.2°, 6.6 ± 0.2°, 9.9 ± 0.2°, 13.3 ± 0.2°, 13.7 ± 0.2°, 14.4 ± 0.2°, 16.9 ± 0.2°, 19.2 ± 0.2°, 23.3 ± 0.2°, and 24.9 ± 0.2°;
3.3 ± 0.2°, 6.6 ± 0.2°, 9.9 ± 0.2°, 13.3 ± 0.2°, 13.7 ± 0.2°, 14.2 ± 0.2°, 19.2 ± 0.2°, 20.0 ± 0.2°, 23.3 ± 0.2°, and 24.9 ± 0.2°;
3.3 ± 0.2°, 6.6 ± 0.2°, 9.9 ± 0.2°, 13.3 ± 0.2°, 13.7 ± 0.2°, 14.2 ± 0.2°, 16.9 ± 0.2°, 20.0 ± 0.2°, 22.7 ± 0.2°, and 24.9 ± 0.2°;
3.3 ± 0.2°, 6.6 ± 0.2°, 9.9 ± 0.2°, 13.3 ± 0.2°, 13.7 ± 0.2°, 14.2 ± 0.2°, 16.9 ± 0.2°, 22.7 ± 0.2°, 22.9 ± 0.2°, and 23.3 ± 0.2°;
3.3 ± 0.2°, 6.6 ± 0.2°, 9.9 ± 0.2°, 13.7 ± 0.2°, 14.2 ± 0.2°, 16.9 ± 0.2°, 22.9 ± 0.2°, 23.3 ± 0.2°, 24.9 ± 0.2°, and 26.9 ± 0.2°;
3.3 ± 0.2°, 6.6 ± 0.2°, 13.3 ± 0.2°, 13.7 ± 0.2°, 14.2 ± 0.2°, 14.4 ± 0.2°, 16.9 ± 0.2°, 20.0 ± 0.2°, 23.3 ± 0.2°, and 24.9 ± 0.2°;
3.3 ± 0.2°, 9.9 ± 0.2°, 13.3 ± 0.2°, 13.7 ± 0.2°, 14.2 ± 0.2°, 16.9 ± 0.2°, 19.2 ± 0.2°, 22.7 ± 0.2°, 23.3 ± 0.2°, and 24.9 ± 0.2°; and
6.6 ± 0.2°, 9.9 ± 0.2°, 13.3 ± 0.2°, 13.7 ± 0.2°, 14.2 ± 0.2°, 16.9 ± 0.2°, 20.0 ± 0.2°, 23.3 ± 0.2°, 24.9 ± 0.2°, and 26.9 ± 0.2°.

In certain embodiments of the present invention, the p-toluenesulfonate dihydrate crystalline Form A has X-ray characteristic diffraction peaks expressed by2θ angle and interplanar spacing d-value by using Cu-Kα radiation, as shown in the following table:

**Table 7**

| Serial No. | XRPD data of p-toluenesulfonate dihydrate crystalline Form A | | | |
|---|---|---|---|---|
| | 2θ (± 0.2°) | d-value | Peak height | Proportion (I%) |
| 1 | 3.326 | 26.5452 | 12860 | 100.0 |
| 2 | 6.632 | 13.3164 | 2331 | 18.1 |
| 3 | 9.942 | 8.8893 | 2282 | 17.7 |
| 4 | 11.379 | 7.7702 | 470 | 3.7 |
| 5 | 12.210 | 7.2432 | 46 | 0.4 |
| 6 | 12.500 | 7.0755 | 192 | 1.5 |
| 7 | 13.271 | 6.6661 | 3161 | 24.6 |
| 8 | 13.721 | 6.4487 | 720 | 5.6 |
| 9 | 14.247 | 6.2116 | 1118 | 8.7 |
| 10 | 14.384 | 6.1527 | 420 | 3.3 |
| 11 | 15.036 | 5.8873 | 41 | 0.3 |
| 12 | 15.361 | 5.7635 | 63 | 0.5 |
| 13 | 15.970 | 5.5452 | 363 | 2.8 |
| 14 | 16.926 | 5.2340 | 1101 | 8.6 |
| 15 | 17.644 | 5.0227 | 48 | 0.4 |
| 16 | 18.193 | 4.8724 | 279 | 2.2 |
| 17 | 18.472 | 4.7993 | 38 | 0.3 |
| 18 | 19.210 | 4.6166 | 540 | 4.2 |
| 19 | 19.951 | 4.4468 | 440 | 3.4 |
| 20 | 20.632 | 4.3014 | 40 | 0.3 |
| 21 | 21.610 | 4.1089 | 48 | 0.4 |
| 22 | 21.858 | 4.0630 | 42 | 0.3 |
| 23 | 22.115 | 4.0163 | 121 | 0.9 |
| 24 | 22.314 | 3.9809 | 74 | 0.6 |
| 25 | 22.549 | 3.9400 | 199 | 1.5 |
| 26 | 22.716 | 3.9113 | 495 | 3.8 |
| 27 | 22.858 | 3.8874 | 579 | 4.5 |
| 28 | 23.327 | 3.8103 | 806 | 6.3 |
| 29 | 23.486 | 3.7849 | 338 | 2.6 |
| 30 | 23.943 | 3.7137 | 37 | 0.3 |
| 31 | 24.087 | 3.6917 | 58 | 0.5 |
| 32 | 24.554 | 3.6226 | 161 | 1.3 |
| 33 | 24.864 | 3.5781 | 714 | 5.5 |
| 34 | 25.017 | 3.5566 | 363 | 2.8 |
| 35 | 25.321 | 3.5145 | 95 | 0.7 |
| 36 | 25.778 | 3.4532 | 159 | 1.2 |
| 37 | 26.218 | 3.3963 | 189 | 1.5 |
| 38 | 26.913 | 3.3102 | 389 | 3.0 |
| 39 | 27.193 | 3.2767 | 147 | 1.1 |
| 40 | 27.784 | 3.2084 | 106 | 0.8 |
| 41 | 28.263 | 3.1550 | 43 | 0.3 |
| 42 | 28.485 | 3.1310 | 24 | 0.2 |
| 43 | 29.194 | 3.0566 | 39 | 0.3 |
| 44 | 29.438 | 3.0317 | 31 | 0.2 |
| 45 | 29.836 | 2.9922 | 23 | 0.2 |
| 46 | 32.319 | 2.7678 | 110 | 0.9 |
| 47 | 32.873 | 2.7224 | 91 | 0.7 |
| 48 | 33.582 | 2.6665 | 83 | 0.6 |
| 49 | 34.475 | 2.5995 | 47 | 0.4 |
| 50 | 36.811 | 2.4397 | 23 | 0.2 |
| 51 | 37.031 | 2.4257 | 130 | 1.0 |
| 52 | 39.271 | 2.2923 | 94 | 0.7 |
| 53 | 44.328 | 2.0418 | 47 | 0.4 |
| 54 | 47.777 | 1.9022 | 28 | 0.2 |
| 55 | 51.322 | 1.7788 | 31 | 0.2 |

In certain embodiments of the present invention, the X-ray powder diffraction pattern of the p-toluenesulfonate dihydrate crystalline Form A is substantially as shown in FIG. 15.

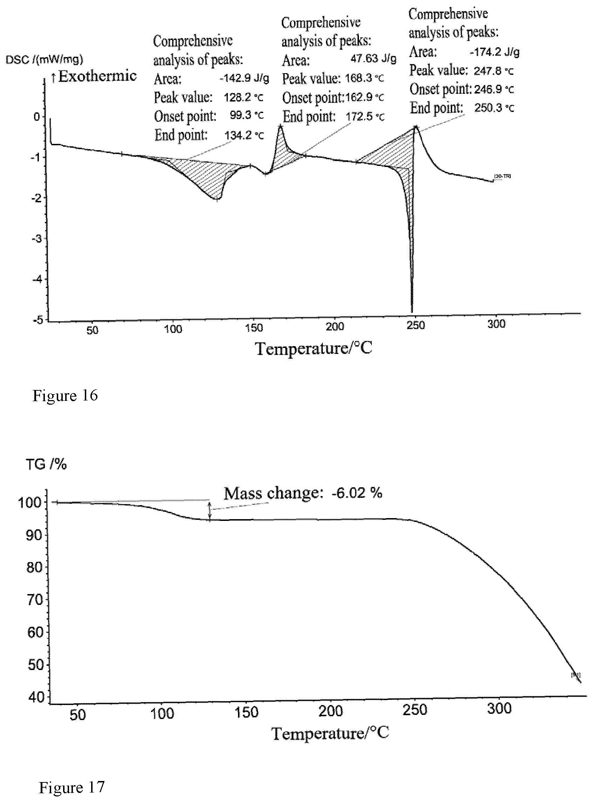

In certain embodiments of the present invention, the DSC thermogram of the p-toluenesulfonate dihydrate crystalline Form A is substantially as shown in FIG. 16.

In certain embodiments of the present invention, the TGA thermogram of the p-toluenesulfonate dihydrate crystalline Form A is substantially as shown in FIG. 17.

In certain embodiments of the present invention, an X-ray powder diffraction pattern of the benzenesulfonate crystalline Form A has a diffraction peak at 2θ of 5.3 ± 0.2°; or has a diffraction peak at 2θ of 9.1 ± 0.2°; or has a diffraction peak at 2θ of 13.9 ± 0.2°; or has a diffraction peak at 2θ of 15.7 ± 0.2°; or has a diffraction peak at 2θ of 18.9 ± 0.2°; or has a diffraction peak at 2θ of 23.3 ± 0.2°; or has a diffraction peak at 2θ of 23.9 ± 0.2°; or has a diffraction peak at 2θ of 26.1 ± 0.2°; preferably, the X-ray powder diffraction pattern comprises any 2-5, or 3-5, or 3-6, or 3-8, or 5-8, or 6-8 of said peaks, more preferably, comprises any 6, 7, or 8 of said peaks.

In certain embodiments of the present invention, the X-ray powder diffraction pattern of the benzenesulfonate crystalline Form A comprises at least one or more, preferably two, more preferably three of diffraction peaks at 2θ of 5.3 ± 0.2°, 9.1 ± 0.2°, 13.9 ± 0.2°, 15.7 ± 0.2°, and 26.1 ± 0.2°; and optionally further comprises at least one, preferably two or three of diffraction peaks at 2θ of 18.9 ± 0.2°, 23.3 ± 0.2°, and 23.9 ± 0.2°.

In certain embodiments of the present invention, the X-ray powder diffraction pattern of the benzenesulfonate crystalline Form A optionally further comprises one or more of diffraction peaks at 2θ of 10.5 ± 0.2°, 11.3 ± 0.2°, 18.2 ± 0.2°, 22.8 ± 0.2°, 25.0 ± 0.2°, 28.2 ± 0.2°, and 32.2 ± 0.2°; preferably, at least comprises any 2-3, or 4-5 of said peaks; further preferably, comprises any 2, 3, 4, or 5 of said peaks.

In certain embodiments of the present invention, the X-ray powder diffraction pattern of the benzenesulfonate crystalline Form A comprises one or more of diffraction peaks at 2θ of 5.3 ± 0.2°, 9.1 ± 0.2°, 10.5 ± 0.2°, 11.3 ± 0.2°, 13.9 ± 0.2°, 15.7 ± 0.2°, 18.2 ± 0.2°, 18.9 ± 0.2°, 22.8 ± 0.2°, 23.3 ± 0.2°, 23.9 ± 0.2°, 25.0 ± 0.2°, 26.1 ± 0.2°, 28.2 ± 0.2°, and 32.2 ± 0.2°; preferably, comprises any 4, 5, 6, 8, or 10 of said peaks.

In certain embodiments of the present invention, the benzenesulfonate crystalline Form A has X-ray characteristic diffraction peaks expressed by 2θ angle and interplanar spacing d-value by using Cu-Kα radiation, as shown in the following table:

**Table 8**

| Serial No. | XRPD data of benzenesulfonate crystalline Form A | | | |
|---|---|---|---|---|
| | 2θ (± 0.2°) | d-value | Peak height | Proportion (I%) |
| 1 | 5.278 | 16.731 | 7043 | 100 |
| 2 | 9.068 | 9.7445 | 539 | 7.7 |
| 3 | 10.467 | 8.4443 | 299 | 4.2 |
| 4 | 11.34 | 7.7963 | 163 | 2.3 |
| 5 | 13.873 | 6.3779 | 553 | 7.9 |
| 6 | 15.698 | 5.6405 | 629 | 8.9 |
| 7 | 17.372 | 5.1005 | 112 | 1.6 |
| 8 | 17.889 | 4.9543 | 132 | 1.9 |
| 9 | 18.172 | 4.8777 | 181 | 2.6 |
| 10 | 18.901 | 4.6913 | 444 | 6.3 |
| 11 | 19.309 | 4.593 | 91 | 1.3 |
| 12 | 20.402 | 4.3494 | 128 | 1.8 |
| 13 | 21.437 | 4.1417 | 115 | 1.6 |
| 14 | 22.753 | 3.905 | 343 | 4.9 |
| 15 | 23.34 | 3.8081 | 417 | 5.9 |
| 16 | 23.868 | 3.7251 | 371 | 5.3 |
| 17 | 24.982 | 3.5614 | 227 | 3.2 |
| 18 | 26.137 | 3.4065 | 539 | 7.7 |
| 19 | 27.274 | 3.2671 | 127 | 1.8 |
| 20 | 27.66 | 3.2224 | 118 | 1.7 |
| 21 | 28.245 | 3.157 | 189 | 2.7 |
| 22 | 29.306 | 3.045 | 74 | 1.1 |
| 23 | 32.159 | 2.7811 | 145 | 2.1 |
| 24 | 33.696 | 2.6577 | 86 | 1.2 |

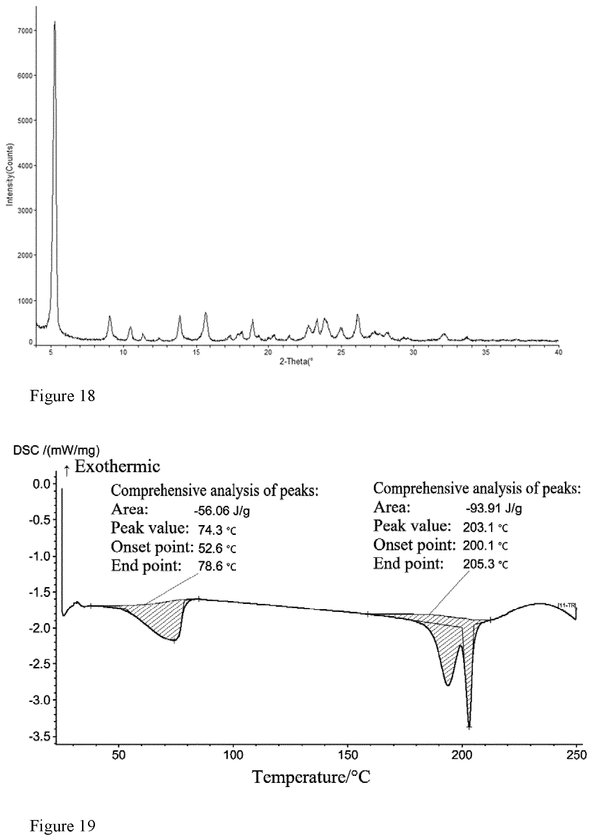

In certain embodiments of the present invention, the X-ray powder diffraction pattern of the benzenesulfonate crystalline Form A is substantially as shown in FIG. 18.

In certain embodiments of the present invention, the DSC thermogram of the benzenesulfonate crystalline Form A is substantially as shown in FIG. 19.

In certain embodiments of the present invention, the 2θ positions of the top ten most intense diffraction peaks in the X-ray powder diffraction patterns of the hydrochloride crystalline Form A, the sulfate crystalline Form A, the methanesulfonate crystalline Form A, the p-toluenesulfonate crystalline Form A, the p-toluenesulfonate crystalline Form B, the p-toluenesulfonate crystalline Form C, the p-toluenesulfonate dihydrate crystalline Form A, and the benzenesulfonate crystalline Form A have a deviation of ±0.2° to +0.5°, preferably ±0.2° to ±0.3°, most preferably ±0.2° from the corresponding peak positions in the X-ray powder diffraction patterns.

The present invention further provides a method for preparing the acid addition salt of the compound or a stereoisomer thereof as described above, comprising the following steps:
1) weighing an appropriate amount of a free base and dissolving it in a benign solvent;
2) weighing an appropriate amount of a counter-ion acid and dissolving it in an organic solvent, wherein the amount of the counter-ion acid is preferably 1.2 equivalents;
3) combining the above two solutions, and stirring for precipitation, or adding a poor solvent dropwise with stirring for precipitation; and
4) obtaining the acid addition salt by rapid centrifugation or static drying,
wherein
the benign solvent is selected from one or more of methanol, acetone, ethyl acetate, acetonitrile, ethanol, 88% acetone, tetrahydrofuran, 2-methyltetrahydrofuran, dichloromethane, 1,4-dioxane, benzene, toluene, isopropanol, n-butanol, isobutanol, N,N-dimethylformamide, N,N-dimethylacetamide, n-propanol, tert-butanol, 2-butanone, 3-pentanone, or N-methylpyrrolidone; preferably one or more of N-methylpyrrolidone, methanol, dichloromethane, or anhydrous ethanol;
the organic solvent is selected from methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-butanone, 3-pentanone, heptane, methyl tert-butyl ether, isopropyl ether, 1,4-dioxane, tert-butyl alcohol, or N,N-dimethylformamide; preferably methanol, ethanol, or acetonitrile; the above good solvent and organic solvent are necessarily mutually soluble when used;
the poor solvent is selected from one or more of heptane, cyclohexane, n-hexane, n-pentane, water, ethyl acetate, methyl tert-butyl ether, toluene, or isopropyl ether; preferably one or more of water, heptane, methyl tert-butyl ether, or isopropyl ether; and
the counter-ion acid is selected from hydrochloric acid, sulfuric acid, nitric acid, hydrobromic acid, hydrofluoric acid, hydroiodic acid, phosphoric acid, the organic acid is selected from 2,5-dihydroxybenzoic acid, 1-hydroxy-2-naphthoic acid, acetic acid, dichloroacetic acid, trichloroacetic acid, acetohydroxamic acid, adipic acid, benzenesulfonic acid, 4-chlorobenzenesulfonic acid, benzoic acid, 4-acetylaminobenzoic acid, 4-aminobenzoic acid, decanoic acid, hexanoic acid, octanoic acid, cinnamic acid, citric acid, cyclohexylsulfamic acid, camphorsulfonic acid, aspartic acid, camphanic acid, gluconic acid, glucuronic acid, glutamic acid, isoascorbic acid, lactic acid, malic acid, mandelic acid, pyroglutamic acid, tartaric acid, dodecylsulfuric acid, dibenzoyltartaric acid, ethane-1,2-disulfonic acid, ethanesulfonic acid, formic acid, fumaric acid, galactonic acid, gentisuric acid, glutaric acid, 2-ketoglutaric acid, glycolic acid, hippuric acid, hydroxyethylsulfonic acid, lactobionic acid, ascorbic acid, aspartic acid, lauric acid, camphanic acid, maleic acid, malonic acid, methanesulfonic acid, 1,5-naphthalenedisulfonic acid, naphthalene-2-sulfonic acid, nicotinic acid, oleic acid, orotic acid, oxalic acid, palmitic acid, pamoic acid, propanoic acid, salicylic acid, 4-aminosalicylic acid, sebacic acid, stearic acid, succinic acid, thiocyanic acid, undecylenoic acid, trifluoroacetic acid, benzenesulfonic acid, p-methylbenzenesulfonic acid, or L-malic acid; preferably fumarate, p-toluenesulfonate, or succinate; most preferably p-toluenesulfonate.

The present invention further provides a method for preparing the crystalline form of the acid addition salt of the compound or a stereoisomer thereof as described above, wherein the method is Method **I, II,** or III;

Method I comprises the following steps:
1) suspending the compound in a poor solvent;
2) adding a counter-ion acid, wherein the amount of the counter-ion acid is preferably 1.2 equivalents; the counter-ion acid may be dissolved in an organic solvent;
3) stirring to dissolve, followed by continued stirring for precipitation or dropwise addition of a poor solvent with stirring to induce precipitation; and
4) isolating to obtain the anhydrous crystalline form;

Method II comprises the following step:
converting the anhydrous crystalline form obtained from Method I via crystal transformation;
Method III comprises the following step:
suspending the anhydrous crystalline form obtained from Method I in water, and isolating to obtain the hydrate crystalline form,
wherein
the poor solvent is selected from one or more of acetone, ethyl acetate, isopropyl acetate, acetonitrile, ethanol, 88% acetone, tetrahydrofuran, 2-methyltetrahydrofuran, dichloromethane, 1,4-dioxane, benzene, toluene, isopropanol, n-butanol, isobutanol, N,N-dimethylformamide, N,N-dimethylacetamide, n-propanol, tert-butanol, 2-butanone or 3-pentanone, methyl tert-butyl ether, or water; preferably one or more of acetone, ethanol, tetrahydrofuran, acetonitrile, or toluene.

The organic solvent is selected from methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-butanone, 3-pentanone, heptane, methyl tert-butyl ether, isopropyl ether, 1,4-dioxane, tert-butyl alcohol, or N,N-dimethylformamide; preferably methanol, ethanol, or acetonitrile;
the counter-ion acid is selected from hydrochloric acid, sulfuric acid, nitric acid, hydrobromic acid, hydrofluoric acid, hydroiodic acid, phosphoric acid, the organic acid is selected from 2,5-dihydroxybenzoic acid, 1-hydroxy-2-naphthoic acid, acetic acid, dichloroacetic acid, trichloroacetic acid, acetohydroxamic acid, adipic acid, benzenesulfonic acid, 4-chlorobenzenesulfonic acid, benzoic acid, 4-acetylaminobenzoic acid, 4-aminobenzoic acid, decanoic acid, hexanoic acid, octanoic acid, cinnamic acid, citric acid, cyclohexylsulfamic acid, camphorsulfonic acid, aspartic acid, camphanic acid, gluconic acid, glucuronic acid, glutamic acid, isoascorbic acid, lactic acid, malic acid, mandelic acid, pyroglutamic acid, tartaric acid, dodecylsulfuric acid, dibenzoyltartaric acid, ethane-1,2-disulfonic acid, ethanesulfonic acid, formic acid, fumaric acid, galactonic acid, gentisuric acid, glutaric acid, 2-ketoglutaric acid, glycolic acid, hippuric acid, hydroxyethylsulfonic acid, lactobionic acid, ascorbic acid, aspartic acid, lauric acid, camphanic acid, maleic acid, malonic acid, methanesulfonic acid, 1,5-naphthalenedisulfonic acid, naphthalene-2-sulfonic acid, nicotinic acid, oleic acid, orotic acid, oxalic acid, palmitic acid, pamoic acid, propanoic acid, salicylic acid, 4-aminosalicylic acid, sebacic acid, stearic acid, succinic acid, thiocyanic acid, undecylenoic acid, trifluoroacetic acid, benzenesulfonic acid, p-methylbenzenesulfonic acid, or L-malic acid; preferably fumarate, p-toluenesulfonate, or succinate; most preferably p-toluenesulfonate.

In certain embodiments of the present invention, the compound is suspended with ethanol and then mixed with a solution of hydrochloric acid in methanol, and the mixture is separated to obtain hydrochloride crystalline Form A of the compound.

In certain embodiments of the present invention, the compound is suspended with ethanol and then mixed with a solution of sulfuric acid in methanol, and the mixture is separated to obtain sulfate crystalline Form A of the compound.

In certain embodiments of the present invention, the compound is suspended with ethanol and then mixed with a solution of methanesulfonic acid in methanol, and the mixture is separated to obtain methanesulfonate crystalline Form A of the compound.

In certain embodiments of the present invention, the compound is suspended with ethanol and then mixed with a solution of p-toluenesulfonic acid in methanol, and the mixture is separated to obtain p-toluenesulfonate crystalline Form A of the compound.

In certain embodiments of the present invention, the p-toluenesulfonate crystalline Form A of the compound is transformed in tetrahydrofuran or 2-methyltetrahydrofuran to obtain p-toluenesulfonate crystalline Form B of the compound.

In certain embodiments of the present invention, the p-toluenesulfonate crystalline Form A of the compound is transformed in 1,4-dioxane to obtain p-toluenesulfonate crystalline Form C of the compound.

In certain embodiments of the present invention, the p-toluenesulfonate crystalline Form A of the compound is suspended with water, followed by separation to obtain p-toluenesulfonate dihydrate crystalline Form A of the compound.

In certain embodiments of the present invention, the compound is suspended with ethanol and then mixed with a solution of benzenesulfonic acid in methanol, and the mixture is separated to obtain benzenesulfonate crystalline Form A of the compound.

The present invention further provides a pharmaceutical composition comprising a therapeutically effective amount of the acid addition salt of the compound or a stereoisomer thereof as described above and/or the crystalline form of the acid addition salt of the compound or a stereoisomer thereof as described above, and one or more pharmaceutically acceptable carriers, diluents or excipients.

The present invention further provides the use of the acid addition salt of the compound or a stereoisomer thereof, the crystalline form of the acid addition salt of the compound or a stereoisomer thereof as described above, or the pharmaceutical composition as described above in the manufacture of a PARP inhibitor medicament, wherein the PARP is preferably PARP1.

The present invention further provides the acid addition salt of the compound or a stereoisomer thereof, the crystalline form of the acid addition salt of the compound or a stereoisomer thereof as described above, or the pharmaceutical composition as described above, for use as a PARP inhibitor medicament, wherein the PARP is preferably PARP1.

The present invention further provides the use of the acid addition salt of the compound or a stereoisomer thereof as described above, the crystalline form of the acid addition salt of the compound or a stereoisomer thereof as described above, or the pharmaceutical composition as described above in the manufacture of a medicament for treating cancer, ischemic diseases, or neurodegenerative diseases; preferably wherein the cancer is selected from breast cancer, ovarian cancer, pancreatic cancer, prostate cancer, hematological cancer, gastric cancer, colorectal cancer, gastrointestinal cancer, and lung cancer.

The present invention further provides the acid addition salt of the compound or a stereoisomer thereof as described above, the crystalline form of the acid addition salt of the compound or a stereoisomer thereof as described above, or the pharmaceutical composition as described above, for use in treating cancer, ischemic diseases, or neurodegenerative diseases; preferably wherein the cancer is selected from breast cancer, ovarian cancer, pancreatic cancer, prostate cancer, hematological cancer, gastric cancer, colorectal cancer, gastrointestinal cancer, and lung cancer.

The present invention further relates to a method for treating, preventing and/or treating cancer, ischemic diseases, or neurodegenerative diseases, comprising administering to a patient a therapeutically effective dose of the acid addition salt of the compound or a stereoisomer thereof as described above, the crystalline form of the acid addition salt of the compound or a stereoisomer thereof as described above, or the pharmaceutical composition as described above; preferably wherein the cancer is selected from breast cancer, ovarian cancer, pancreatic cancer, prostate cancer, hematological cancer, gastric cancer, colorectal cancer, gastrointestinal cancer, and lung cancer.

### Detailed Description of the Invention

Unless stated to the contrary, all the technical and scientific terms used herein usually have the same meanings as commonly understood by those of ordinary skill in the art. Specifically, the terms used in the description and claims have the following meanings.

The term "alkyl" refers to a linear or branched saturated aliphatic hydrocarbon group, and the alkyl may be optionally substituted with one or more substituents. In specific embodiments, the alkyl refers to a linear saturated hydrocarbon group having 1 to 20 (C₁₋₂₀), 1 to 15 (C₁₋₁₅), 1 to 12 (C₁₋₁₂), 1 to 10 (C₁₋₁₀), 1 to 8 (C₁₋₈), 1 to 6 (C₁₋₆), or 1 to 3 (C₁₋₃) carbon atoms, or a branched saturated hydrocarbon group having 3 to 20 (C₃₋₂₀), 3 to 15 (C₃₋₁₅), 3 to 12 (C₃₋₁₂), 3 to 10 (C₃₋₁₀), 3 to 8 (C₃₋₈), or 3 to 6 (C₃₋₆) carbon atoms. As used herein, linear C₁₋₆ alkyl and branched C₃₋₆ alkyl groups are also referred to as "lower alkyl groups". For example, C₁₋₆ alkyl refers to a linear saturated monovalent hydrocarbon group having 1 to 6 carbon atoms or a branched saturated monovalent hydrocarbon group having 3 to 6 carbon atoms. In one embodiment, the C₁₋₆ alkyl contains 1 to 6 (e.g., 1, 2, 3, 4, 5, or 6) carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. In one embodiment, the alkyl is an optionally substituted alkyl group as described elsewhere herein.

The term "alkynyl" refers to a linear or branched unsaturated aliphatic hydrocarbon group containing at least one carbon-carbon triple bond, wherein the carbon-carbon triple bond can be located at any position within the alkynyl, and the alkynyl can be optionally substituted with one or more substituents. In specific embodiments, the alkynyl is a linear unsaturated hydrocarbon group having 2 to 20 (C₂₋₂₀), 2 to 15 (C₂₋₁₅), 2 to 12 (C₂₋₁₂), 2 to 10 (C₂₋₁₀), 2 to 8 (C₂₋₈), 2 to 6 (C₂₋₆), or 2 to 4 (C₂₋₄) carbon atoms, or a branched unsaturated hydrocarbon group having 3 to 20 (C₃₋₂₀), 3 to 15 (C₃₋₁₅), 3 to 12 (C₃₋₁₂), 3 to 10 (C₃₋₁₀), 3 to 8 (C₃₋₈), or 3 to 6 (C₃₋₆) carbon atoms. Unless otherwise specified, the term "alkynyl" as used herein includes both linear and branched alkynyl groups. For example, C₂₋₆ alkynyl refers to a linear unsaturated hydrocarbon group having 2 to 6 carbon atoms or a branched unsaturated hydrocarbon group having 3 to 6 carbon atoms. In one embodiment, the C₂₋₆ alkynyl contains 2 to 6 (e.g., 2, 3, 4, 5, or 6) carbon atoms. Non-limiting examples of alkynyl include: 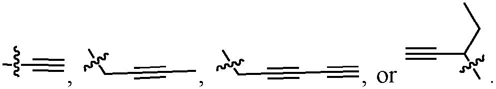 In one embodiment, the alkynyl is an optionally substituted alkynyl group as described elsewhere herein.

The term "cycloalkyl" refers to saturated or partially unsaturated aliphatic hydrocarbon monocyclic or polycyclic (bi- and higher) cyclic groups, which may be optionally substituted with one or more substituents. In specific embodiments, the cycloalkyl ring contains 3 to 20 (C₃₋₂₀), 3 to 12 (C₃₋₁₂), 3 to 8 (C₃₋₈), or 3 to 6 (C₃₋₆) carbon atoms; In one embodiment, the cycloalkyl ring comprises 6 to 14 (C₆₋₁₄) or 7 to 10 (C₇₋₁₀) carbon atoms, which may contain one or more double bonds but have no completely conjugated π-electron system. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; and in one embodiment, the polycyclic cycloalkyl includes spirocycloalkyl, fused cycloalkyl, and bridged cycloalkyl. In one embodiment, the cycloalkyl is an optionally substituted cycloalkyl group described elsewhere herein or cycloalkyl optionally fused with heterocyclyl, aryl, or heteroaryl, and non-limiting examples include indanyl, tetrahydronaphthyl, benzocycloheptanyl, etc.

The term "heterocyclyl" refers to a saturated or partially unsaturated monocyclic or polycyclic hydrocarbon group, in which one or more ring atoms are heteroatoms selected from nitrogen, oxygen, boron, phosphorus, or sulfur, wherein the nitrogen, phosphorus, or sulfur atom can be optionally oxidized, the nitrogen atom can be optionally quaternized, the ring carbon atoms can be optionally replaced by oxygen, with the ring fragments -O-O- and -O-S- being excluded, and the remaining ring atoms are carbon, which may contain one or more double bonds but have no completely conjugated π-electron system. In specific embodiments, the heterocyclyl comprises 3 to 20, 3 to 12, 3 to 8, or 3 to 6 ring atoms, of which 1 to 4 are heteroatoms; in one embodiment, the heterocyclyl comprises 3 to 6, 4 to 6, 3 to 8, 3 to 10, 6 to 10, or 7 to 11 ring atoms; in one embodiment, the heterocyclyl comprises 3 to 8 (e.g., 3, 4, 5, 6, 7, or 8) ring atoms. Non-limiting examples of monocyclic heterocyclyl include tetrahydropyrrolyl, azetidinyl, oxetanyl, oxacyclohexyl, tetrahydrofuryl, tetrahydropyranyl, tetrahydrothienyl, dihydroimidazolyl, dihydrofuryl, dihydropyrazolyl, dihydropyrrolyl, piperidinyl, piperazinyl, morpholinyl, thiomorpholinyl, homopiperazinyl, pyranyl, etc. Polycyclic heterocyclyl includes spiro, fused and bridged heterocyclyl. In one embodiment, the heterocyclyl is an optionally substituted heterocyclyl group described elsewhere herein, or a heterocyclyl group further fused to other cycloalkyl, heterocyclyl, aryl and heteroaryl groups via any two or more atoms on the ring.

The term "alkoxy" refers to -O-(alkyl) and -O-(unsubstituted cycloalkyl), wherein the alkyl or cycloalkyl is as defined previously. Non-limiting examples of alkoxy include: methoxy, ethoxy, propoxy, butoxy, cyclopropoxy, cyclobutoxy, cyclopentyloxy, or cyclohexyloxy. In one embodiment, the alkoxy is an optionally substituted alkoxy group as described elsewhere herein.

The term "haloalkyl" refers to alkyl substituted with one or more halogen, wherein the alkyl is as defined previously. Non-limiting examples of haloalkyl include: trifluoromethyl, -CH₂CF₃,

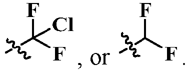

The term "hydrogen" includes protons (¹H), deuterium (²H), tritium (³H) and/or mixtures thereof. In certain embodiments, one or more hydrogen-occupied positions in the compound can be enriched with deuterium and/or tritium. Such isotope-enriched analogues can be prepared by suitable isotope-labeled starting raw materials obtained from commercial sources or by known literature procedures.

Different terms such as "X is selected from A, B, or C", "X is selected from A, B and C", "X is A, B or C", and "X is A, B and C" all express the same meaning, i.e., X can be any one or more of A, B, and C.

"Optional" or "optionally" means that the event or circumstance subsequently described may but need not to occur, and the description includes the occasion where the event or circumstance occurs or does not occur. For example, "heterocyclic group optionally substituted with alkyl" means the alkyl may but need not be present, and the description includes the case where the heterocyclic group is substituted with alkyl and the case where the heterocyclic group is not substituted with alkyl.

In various parts of the present invention, linking substituents are described. When it is clear that a linking group is required for the structure, the markush variables listed for that group should be understood as referring to the linking group. For example, if a linking group is required for the structure and the markush group definition for that variable lists "alkyl" or "aryl," it should be understood that the "alkyl" or "aryl" respectively represents the attached alkylene group or arylene group.

In one embodiment, "substituted" means that any one or more hydrogen atoms on a specific atom are replaced by substituents, as long as the valence state of the specific atom is normal and the substituted compound is stable. When the substituent is oxo (i.e., = O), it means that two hydrogen atoms are substituted. The term "optionally substituted" means that it may or may not be substituted, and unless otherwise specified, the types and numbers of substituents may be arbitrary on the basis of being chemically realizable. It goes without saying, the substituents may be only in their possible chemical positions, a person skilled in the art can determine the possible or impossible substitutions (by experiment or theory) without paying too much effort. For example, the amino group having a free hydrogen or a hydroxyl group may be unstable when combined the carbon atoms having an unsaturated (e.g., olefinic) bond.

Unless stated to the contrary, the indefinite articles "a" and "an" and the definite article "the" in the description and claims include plural and singular forms.

"Pharmaceutical composition" denotes a mixture containing one or more compounds described herein or physiologically/pharmaceutically acceptable salts or prodrug thereof and other chemical components, as well as other components, such as physiologically/pharmaceutically acceptable carriers and excipients. The purpose of pharmaceutical compositions is to facilitate administration to living organisms and facilitate the absorption of active ingredients to exert biological activity.

"Pharmaceutically acceptable salts" refer to salts of the compounds of the present invention, which are safe and effective when used in mammals, and have appropriate biological activity.

"Stereoisomer" includes all enantiomerically/diastereoisomerically/ stereoisomerically pure and enantiomerically/diastereoisomerically/ stereoisomerically enriched compounds of the present invention.

"Stereoisomerically pure" refers to a composition comprising one stereoisomer of a certain compound and being substantially free of the other stereoisomer of the compound. For example, a stereoisomerically pure composition of a compound with a chiral center will be substantially free of the relative enantiomer of the compound. A stereoisomerically pure composition of a compound with two chiral centers will be substantially free of other diastereomers of the compound. A typical stereoisomerically pure compound includes one stereoisomer of the compound with a mass content of more than about 80% and the other stereoisomer of the compound with a mass content of less than about 20%, one stereoisomer of the compound with a mass content of more than about 90% and the other stereoisomer of the compound with a mass content of less than about 10%, one stereoisomer of the compound with a mass content of more than about 95% and the other stereoisomer of the compound with a mass content of less than about 5%, one stereoisomer of the compound with a mass content of more than about 97% and the other stereoisomer of the compound with a mass content of less than about 3%, or one stereoisomer of the compound with a mass content of more than about 99% and the other stereoisomer of the compound with a mass content of less than about 1%.

"Stereoisomerically enriched" refers to a composition comprising a stereoisomer of a certain compound with a mass content of more than about 55%, a mass content of more than about 60%, a mass content of more than about 70%, or a mass content of more than about 80%.

"Enantiomerically pure" refers to a stereoisomerically pure composition of a compound with a chiral center. Similarly, the term "enantiomerically enriched" refers to a stereoisomerically enriched composition of a compound with a chiral center.

"Optically active" and "enantiomerically active" refer to a molecular combination having not less than about 50%, not less than about 70%, not less than about 80%, not less than about 90%, not less than about 91%, not less than about 92%, not less than about 93%, not less than about 94%, not less than about 95%, not less than about 96%, not less than about 97%, not less than about 98%, not less than about 99%, not less than about 99.5%, or not less than about 99.8% enantiomeric excess or diastereomeric excess. In a specific embodiment, the compound comprises about 95% or more of the desired enantiomer or diastereomer and about 5% or less of the less preferred enantiomer or diastereomer on the basis of the total weight of the racemate.

When describing optically active compounds, the prefixes R and S are used to indicate the absolute configuration of the molecule relative to the chiral center thereof. (+) and (-) are used to indicate the optical rotation of the compound, that is, the direction of the plane of polarized light rotated by the optically active compound. The prefix (-) indicates that the compound is levorotatory, that is, the compound rotates the plane of polarized light to the left or counterclockwise. The prefix (+) indicates that the compound is dextrorotatory, that is, the compound rotates the plane of polarized light to the right or clockwise. However, the symbols (+) and (-) of optical rotation are irrelevant to the absolute configurations R and S of molecules.

### Brief Description of the Drawings

FIG. 1 shows the inhibition of PAR by Example 1 in MDA-MB-436 model within 24 h after single administration;
FIG. 2 shows the inhibition of PAR by Example 1 in MDA-MB-436 model within 24-72 h after single administration;
FIG. 3 is an XRPD pattern of hydrochloride crystalline Form A;
FIG. 4 is a DSC thermogram of the hydrochloride crystalline Form A;
FIG. 5 is an XRPD pattern of sulfate crystalline Form A;
FIG. 6 is a DSC thermogram of the sulfate crystalline Form A;
FIG. 7 is an XRPD pattern of methanesulfonate crystalline Form A;
FIG. 8 is a DSC thermogram of the methanesulfonate crystalline Form A;
FIG. 9 is an XRPD pattern of p-toluenesulfonate crystalline Form A;
FIG. 10 is a DSC thermogram of the p-toluenesulfonate crystalline Form A;
FIG. 11 is an XRPD pattern of p-toluenesulfonate crystalline Form B;
FIG. 12 is a DSC thermogram of the p-toluenesulfonate crystalline Form B;
FIG. 13 is an XRPD pattern of p-toluenesulfonate crystalline Form C;
FIG. 14 is a DSC thermogram of the p-toluenesulfonate crystalline Form C;
FIG. 15 is an XRPD pattern of p-toluenesulfonate dihydrate crystalline Form A;
FIG. 16 is a DSC thermogram of the p-toluenesulfonate dihydrate crystalline Form A;
FIG. 17 is a TGA thermogram of the p-toluenesulfonate dihydrate crystalline Form A;
FIG. 18 is an XRPD pattern of benzenesulfonate crystalline Form A;
FIG. 19 is a DSC thermogram of the benzenesulfonate crystalline Form A; and
FIG. 20 is a single crystal structure diagram of the p-toluenesulfonate dihydrate crystalline Form A (free of hydrogen atoms).

### Detailed Description of Embodiments

The present invention will be further described below in conjunction with examples, but these examples are not meant to limit the scope of the present invention.

### Examples

The structure of the compound of the present invention is determined by nuclear magnetic resonance (NMR) or/and liquid chromatography-mass spectrometry (LC-MS). NMR chemical shift (δ) is given in parts per million (ppm). NMR is determined with Bruker AVANCE-400 nuclear magnetic resonance instrument; the solvents for determination are deuterated dimethyl sulfoxide (DMSO-*d₆*), deuterated methanol (CD₃OD) and deuterated chloroform (CDCl₃); and the internal standard is tetramethylsilane (TMS).

Agilent 1200 Infinity Series mass spectrometer is used for measurement by liquid chromatography-mass chromatography (LC-MS). HPLC uses Agilent 1200DAD high-pressure liquid chromatograph (Sunfire C18 150 × 4.6 mm column) and Waters 2695-2996 high-pressure liquid chromatograph (Gimini C₁₈ 150 × 4.6 mm column).

Yantai Huanghai HSGF254 or Qingdao GF254 silica gel plate is used as a thin layer chromatography silica plate, and TLC is of the specification of 0.15 mm-0.20 mm, and the specification when separating and purifying a product by thin layer chromatography is 0.4 mm - 0.5 mm. For the column chromatography, Yantai Huanghai silica gel of 200-300 mesh silica gel is generally used as a carrier.

The starting materials in the examples of the present invention are known and can be purchased on the market, or can be synthesized using or according to methods known in the art.

Unless otherwise specified, all reactions of the present invention are carried out under continuous magnetic stirring in a dry nitrogen or argon atmosphere, the solvent is a dry solvent, and the reaction temperature unit is degrees Celsius.

### Example 1

### 1'-((7-Ethyl-6-carbonyl-5,6-dihydro-1,5-diazanaphth-3-yl)methyl)-N-methyl-1',2',3',6'-tetrahydro-[3,4'-bipyridinyl]-6-carboxamide

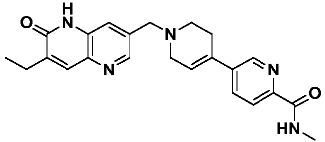

### Step 1: Preparation of ethyl 6-formyl-5-nitronicotinate

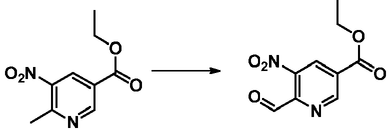

Selenium dioxide (3.96 g, 35.68 mmol) was added to a solution of ethyl 6-methyl-5-nitronicotinate (5.0 g, 23.8 mmol) in 1,4-dioxane (25 mL), and the mixture was heated to 110°C and stirred for 20 hours. The reaction was cooled to room temperature and then filtered under reduced pressure through diatomite, and after filtration, the filtrate was concentrated under reduced pressure to remove the organic solvent, followed by separation by column chromatography to obtain ethyl 6-formyl-5-nitronicotinate as a brown oily compound (4.8 g, 90%).

MS m/z (ES⁺): 224.1 [M]⁺.

### Step 2: Preparation of ethyl (E)-6-(2-(ethoxycarbonyl)but-1-en-1-yl)-5-nitronicotinate

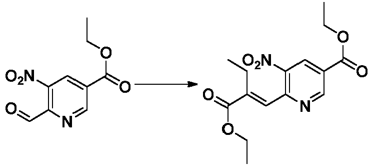

In an ice bath, triethyl 2-phosphocarboxybutyrate (12.8 g, 50.6 mmol) was slowly dropwise added to a solution of NaH (60 wt%, 2.0 g, 50.6 mmol) in tetrahydrofuran (30 mL), the mixture was then stirred for 0.5 hours in the ice bath, then heated to room temperature, stirred for 0.5 hours, further heated to 40°C, and stirred for 10 minutes. The reaction was cooled to -78°C, a solution of ethyl 6-formyl-5-nitronicotinate (4.8 g, 21 mmol) in tetrahydrofuran (20 mL) was slowly dropwise added, and the mixture was then stirred at -78°C for 1 hour. The reaction was quenched with a saturated aqueous ammonium chloride solution and extracted three times with ethyl acetate, and the organic phases were combined and washed with a saturated aqueous sodium chloride solution. The organic phase was separated and dried over anhydrous sodium sulfate. After filtration, the filtrate was concentrated under reduced pressure to remove the organic solvent and separated by column chromatography to obtain the title compound ethyl (E)-6-(2-(ethoxycarbonyl)but-1-en-1-yl)-5-nitronicotinate (5.1 g, 75%).

MS m/z (ES⁺): 322.2 [M]⁺.

### Step 3: Preparation of ethyl 7-ethyl-6-carbonyl-5,6,7,8-tetrahydro-1,5-diazanaphthalene-3-carboxylate

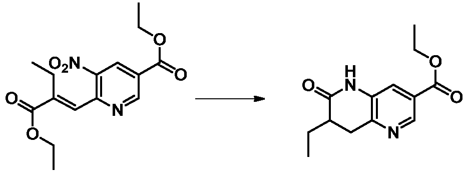

Palladium on carbon (1.86 g, 1.76 mmol) was added to a solution of ethyl (E)-6-(2-(ethoxycarbonyl)but-1-en-1-yl)-5-nitronicotinate (3.76 g, 11.6 mmol) in ethanol (50 mL). After five minutes of oxygen removal by nitrogen, the reaction was placed in a hydrogen atmosphere, stirred overnight at room temperature, and filtered under reduced pressure through diatomite. After filtration, the filtrate was concentrated under reduced pressure to obtain crude ethyl 7-ethyl-6-carbonyl-5,6,7,8-tetrahydro-1,5-diazanaphthalene-3-carboxylate (2.8 g), which was directly used in the next step.

MS m/z (ESI): 249.2 [M+H]⁺.

### Step 4: Preparation of ethyl 7-ethyl-6-carbonyl-5,6-dihydro-1,5-diazanaphthalene-3-carboxylate

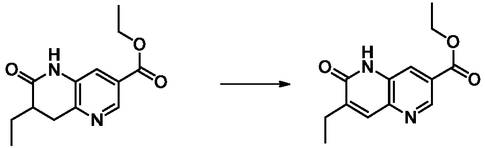

A solution of ethyl 7-ethyl-6-carbonyl-5,6,7,8-tetrahydro-1,5-diazanaphthalene-3-carboxylate (2.8 g, 11.3 mmol) in 1,4-dioxane (50 mL), DDQ (2.85 g, 12.5 mmol) was added, and the mixture was heated to 110°C and stirred for 4 hours. The reaction was cooled to room temperature and then filtered under reduced pressure through diatomite, and the filtrate was concentrated under reduced pressure and then separated by column chromatography to obtain the compound ethyl 7-ethyl-6-carbonyl-5,6-dihydro-1,5-diazanaphthalene-3-carboxylate (2.1 g, 76%).

MS m/z (ESI): 247.2 [M+H]⁺.

### Step 5: Preparation of 3-ethyl-7-(hydroxymethyl)-1,5-diazanaphthalen-2(1H)-one

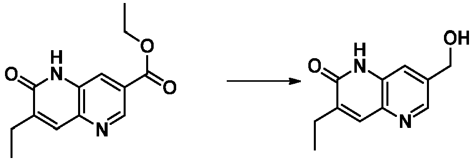

In an ice bath, a solution of 7-ethyl-6-carbonyl-5,6-dihydro-1,5-diazanaphthalene-3-carboxylate (2.1 g, 8.5 mmol) in THF (30 mL), a solution of lithium aluminum hydride in THF (2 M, 8.5 mL, 17.0 mmol) was slowly added, and the mixture was then further stirred for 2 hours in the ice bath. The reaction was quenched by sodium sulfate decahydrate and filtered under reduced pressure through diatomite, and the filtrate was concentrated under reduced pressure and then separated by column chromatography to obtain the compound 3-ethyl-7-(hydroxymethyl)-1,5-diazanaphthalen-2(1H)-one (1.5 g, 86%).

MS m/z (ESI): 205.2 [M+H]⁺.

### Step 6: Preparation of 7-(chloromethyl)-3-ethyl-1,5-diazanaphthalen-2(1H)-one

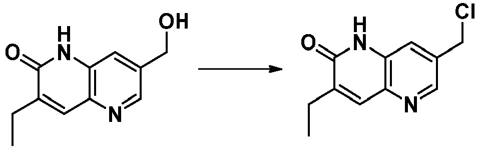

In an ice bath, thionyl chloride (3.2 mL, 44.1 mmol) was added to a solution of 3-ethyl-7-(hydroxymethyl)-1,5-diazanaphthalen-2(1H)-one (1.5 g, 7.4 mmol) in dichloromethane (30 mL), followed by DMF (0.06 mL, 0.77 mmol), and the mixture was then stirred for 6 hours at room temperature. After concentration under reduced pressure to remove the organic solvent, crude 7-(chloromethyl)-3-ethyl-1,5-diazanaphthalen-2(1H)-one (1.61 g) was obtained, which was directly used in the next step.

MS m/z (ESI): 223.1 [M+H]⁺.

### Step 7: Preparation of 1'-(tert-butyl) 6-methyl 3',6'-dihydro-[3,4'-bipyridinyl]-1',6(2'H)-dicarboxylate

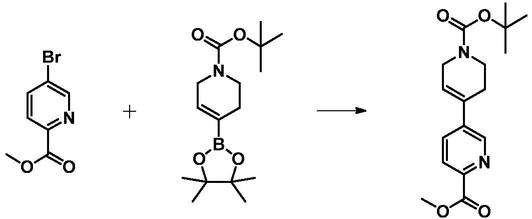

At room temperature, methyl 5-bromomethylpicolinate (1.0 g, 4.6 mmol), tert-butyl 4-(4,4,5,5-tetramethyl-1,3,2-dioxaboran-2-yl)-3,6-dihydropyridine-1(2H)-carboxylate (1.6 g, 5.1 mmol), 1,1'-bis(diphenylphosphino)ferrocene dichloropalladium (146 mg, 0.2 mmol), and potassium carbonate (1.6 g, 11.6 mmol) were dissolved in N,N-dimethylformamide (10 mL). After displacement of oxygen with nitrogen for 1 minute, the mixture was heated to 140°C and reacted for 30 minutes under microwave, and water was added to the reaction system, and the reaction system was extracted with ethyl acetate. The organic phase was separated and washed with a saturated sodium chloride aqueous solution, and the filtrate was dried over anhydrous sodium sulfate, filtered, then concentrated under reduced pressure to remove the organic solvent, and separated by column chromatography to obtain 1'-(tert-butyl) 6-methyl 3',6'-dihydro-[3,4'-bipyridinyl]-1',6(2'H)-dicarboxylate as a brown oily compound (620 mg, 42%).

MS m/z (ES+): 319.1 [M+H]⁺.

### Step 8: Preparation of tert-butyl 6-(methylcarbamyl)-3',6'-dihydro-[3,4'-bipyridinyl]-1'(2'H)-carboxylate

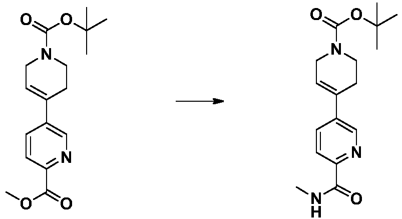

At room temperature, a methylamine alcohol solution (30 wt%, 2.0 g, 19.5 mmol) was added to a solution of 1'-(tert-butyl) 6-methyl 3',6'-dihydro-[3,4'-bipyridinyl]-1',6(2'H)-dicarboxylate (620 mg, 1.9 mmol) in methanol (8 mL), and the solution was then stirred for 4 hours at room temperature. The reaction liquid was concentrated under reduced pressure, a saturated aqueous ammonium chloride solution was added, and the mixture was extracted three times with DCM. The organic phases were combined, and the filtrate was dried over anhydrous sodium sulfate, filtered, and then concentrated under reduced pressure to remove the organic solvent to obtain crude tert-butyl 6-(methylcarbamyl)-3',6'-dihydro-[3,4'-bipyridinyl]-1'(2'H)-carboxylate (600 mg), which was directly used in the next step without further purification.

MS m/z (ESI): 318.2 [M+H]⁺.

### Step 9: Preparation of N-methyl-1',2',3',6'-tetrahydro-[3,4'-bipyridinyl]-6-carboxamide

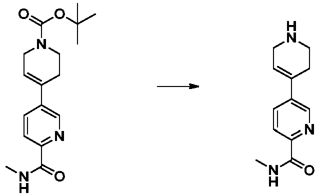

In an ice bath, trifluoroacetic acid (1 mL) was added to a solution of tert-butyl 6-(methylcarbamyl)-3',6'-dihydro-[3,4'-bipyridinyl]-1'(2'H)-carboxylate (200 mg, 0.6 mmol) in dichloromethane (5 mL). The reaction was stirred for 4 hours at room temperature and concentrated under reduced pressure to remove the organic solvent to obtain crude N-methyl-1',2',3',6'-tetrahydro-[3,4'-bipyridinyl]-6-carboxamide (155 mg), which was directly used in the next step without further purification.

MS m/z (ESI): 218.2 [M+H]⁺.

### Step 10: Preparation of 1'-((7-ethyl-6-carbonyl-5,6-dihydro-1,5-diazanaphth-3-yl)methyl)-N-methyl-1',2',3',6'-tetrahydro-[3,4'-bipyridinyl]-6-carboxamide

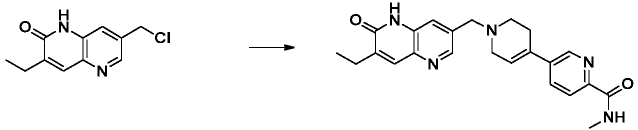

DIPEA (58 mg, 0.45 mmol) and potassium iodide (3 mg, 0.02 mmol) were added to a solution of 7-(chloromethyl)-3-ethyl-1,5-diazanaphthalen-2(1H)-one (20 mg, 0.09 mmol) and N-methyl-1',2',3',6'-tetrahydro-[3,4'-bipyridinyl]-6-carboxamide (52 mg, 0.24 mmol) in acetonitrile (3 mL), and the mixture was heated to 80°C and stirred for 2 hours. The reaction was cooled to room temperature and then filtered under reduced pressure, and the filtrate was concentrated under reduced pressure and then separated by column chromatography to obtain the compound 1'-((7-ethyl-6-carbonyl-5,6-dihydro-1,5-diazanaphth-3-yl)methyl)-N-methyl-1',2',3',6'-tetrahydro-[3,4'-bipyridinyl]-6-carboxamide (6.2 mg, 17%).
¹H NMR (400 MHz, DMSO-*d₆*) *δ* 11.76-11.91 (m, 1H), 8.68-8.73 (m, 2H), 8.40-8.44 (m, 1H), 8.02-7.95 (m, 2H), 7.76 (s, 1H), 7.65 (s, 1H), 6.41-6.44 (m, 1H), 3.69-3.76 (m, 2H), 3.19-3.13 (m, 2H), 2.77-2.85 (m, 3H), 2.66-2.74 (m, 2H), 2.51-2.59 (m, 4H), 1.18 (t, *J =* 7.4 Hz, 3H);
MS m/z (ESI): 404.2 [M+H]⁺.

The following examples were prepared with reference to Example 1.

| Examples | Structure | MS m/z (ESI): [M+H]⁺. | Examples | Structure | MS m/z (ESI): [M+H]⁺. |
|---|---|---|---|---|---|
| 2 | 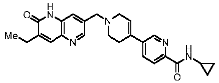 | 430.2 | 13 | 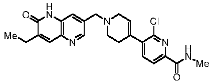 | 438.2 |
| 3 | 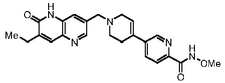 | 420.2 | 14 | 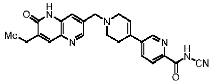 | 415.2 |
| 4 | 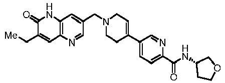 | 460.2 | 15 | 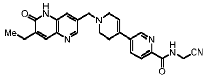 | 429.2 |
| 5 | 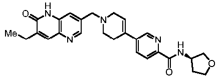 | 460.2 | 16 | 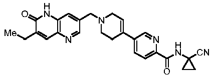 | 455.2 |
| 6 | 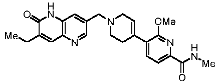 | 434.2 | 17 | 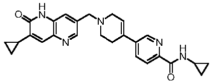 | 442.2 |
| 7 | 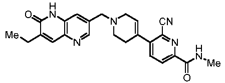 | 429.2 | 18 | 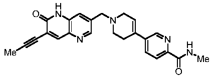 | 414.2 |
| 8 | 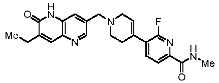 | 422.2 | 19 | 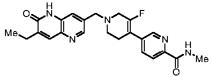 | 422.2 |
| 9 | 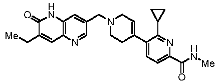 | 444.2 | 20 | 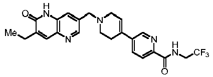 | 472.2 |
| 10 | 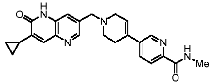 | 416.2 | 21 | 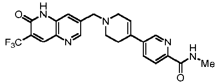 | 444.2 |
| 11 | 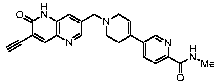 | 400.2 | 22 | 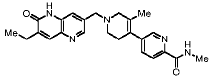 | 418.2 |
| 12 | 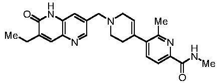 | 418.2 | 23 | 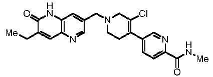 | 438.2 |

| Examples | ¹H NMR |
|---|---|
| 2 | ¹H NMR (400 MHz, DMSO-*d₆*) *δ* 11.77 (s, 1H), 8.60 (d, *J =* 4.2 Hz, 2H), 8.30-8.43 (m, 1H), 7.85-8.01 (m, 2H), 7.69 (s, 1H), 7.58 (s, 1H), 6.34 (s, 1H), 3.65 (s, 2H), 3.09 (d, *J =* 3.5 Hz, 2H), 2.83 (q, *J =* 6.4, 5.2 Hz, 1H), 2.63 (t, *J =* 5.5 Hz, 2H), 2.48 (t*, J =* 7.2 Hz, 4H), 1.13 (q, *J =* 9.3, 7.4 Hz, 3H), 0.54-0.72 (m, 4H); |
| 3 | ¹H NMR (400 MHz, DMSO-*d₆*) *δ* 11.92 (s, 1H), 11.78 (s, 1H), 8.61 (d, *J =* 2.2 Hz, 1H), 8.35 (d, *J =* 1.9 Hz, 1H), 7.94 (dd, *J =* 8.4, 2.3 Hz, 1H), 7.87 (d, *J =* 8.3 Hz, 1H), 7.69 (s, 1H), 7.58 (s, 1H), 6.36 (s, 1H), 3.64 (d, *J =* 13.4 Hz, 5H), 3.09 (d, *J =* 3.4 Hz, 2H), 2.64 (t, *J =* 5.6 Hz, 2H), 2.48 (d, *J =* 7.5 Hz, 4H), 1.12 (t, *J =* 7.4 Hz, 3H); |
| 7 | ¹H NMR (400 MHz, DMSO-*d₆*) *δ* 11.78 (s, 1H), 8.74 (q, *J =* 4.8 Hz, 1H), 8.36 (d, *J =* 1.8 Hz, 1H), 8.04-8.21 (m, 2H), 7.69 (s, 1H), 7.59 (d, *J* = 1.9 Hz, 1H), 6.17 (d, *J* = 4.1 Hz, 1H), 3.68 (s, 2H), 3.12 (d, *J =* 3.3 Hz, 2H), 2.76 (d, *J =* 4.8 Hz, 3H), 2.66 (t, *J =* 5.5 Hz, 2H), 2.48 (d, *J =* 7.0 Hz, 4H), 1.12 (t, *J =* 7.4 Hz, 3H); |
| 8 | ¹H NMR (400 MHz, DMSO-*d₆*) *δ* 11.78 (s, 1H), 8.57 (d, *J =* 5.0 Hz, 1H), 8.35 (d, *J* = 1.8 Hz, 1H), 8.02 (dd, *J =* 9.9, 7.7 Hz, 1H), 7.81-7.90 (m, 1H), 7.69 (s, 1H), 7.58 (s, 1H), 6.19 (s, 1H), 3.65 (s, 2H), 3.10 (s, 2H), 2.72 (d, *J =* 4.8 Hz, 3H), 2.62 (t, *J =* 5.6 Hz, 2H), 2.48 (dd, *J =* 10.3, 4.7 Hz, 4H), 1.12 (t, *J =* 7.4 Hz, 3H); |
| 10 | ¹H NMR (400 MHz, DMSO-*d₆*) *δ* 11.83-11.91 (m, 1H), 8.67-8.73 (m, 2H), 8.38-8.41 (m, 1H), 7.95-8.02 (m, 2H), 7.60-7.64 (m, 1H), 7.42 (s, 1H), 6.39-6.45 (m, 1H), 3.71 (s, 2H), 3.12-3.19 (m, 2H), 2.78-2.86 (m, 3H), 2.68-2.74 (m, 2H), 2.53-2.59 (m, 2H), 2.10-2.19 (m, 1H), 0.94-1.00 (m, 2H), 0.80-0.85 (m, 2H); |
| 13 | ¹H NMR (400 MHz, DMSO-*d₆*) *δ* 11.80 (s, 1H), 8.82 (d, *J =* 2.8 Hz, 1H), 8.70 (s,1H), 7.98 (d, *J =* 1.8 Hz, 1H), 7.90 (d, *J =* 1.8 Hz, 1H), 7.75 (s, 1H), 7.60 (s, 1H), 6.80 (s, 1H), 3.76 (s, 2H), 3.114-3.24 (m, 2H), 2.85 (d, *J =* 4.8 Hz, 3H), 2.65-2.70 (m, 2H), 2.43-2.61 (m, 4H), 1.20 (t, *J=* 7.2 Hz, 3H); |
| 15 | ¹H NMR (400 MHz, DMSO-*d₆*) *δ* 11.85 (s, 1H), 9.41-9.48 (m, 1H), 8.75 (s, 1H), 8.40 (s, 1H), 8.01-8.10 (m, 2H), 7.74 (s, 1H), 7.67 (s, 1H), 5.97 (s, 1H), 4.28-4.35 (m, 2H), 3.74 (s, 2H), 3.12-3.21 (m, 2H), 2.70-2.78 (m, 2H), 2.49-2.60 (m, 4H), 1.21 (t, *J* = 7.4 Hz, 3H); |
| 16 | ¹H NMR (400 MHz, DMSO-*d₆*) *δ* 11.84 (s, 1H), 9.67 (s, 1H), 8.71 (s, 1H), 8.41 (s, 1H), 8.00-8.04 (m, 2H), 7.76 (s, 1H), 7.65 (d, *J =* 5.6 Hz, 1H), 6.46 (s, 1H), 3.73 (s, 2H), 3.16 (d, *J =* 7.2 Hz, 2H), 2.70-2.73 (m, 2H), 2.53-2.57 (m, 4H), 1.51-1.54 (m, 2H), 1.38-1.40 (m, 2H), 1.22 (t, J= 7.2 Hz, 3H); |

### Biological test and evaluation

The present invention will be further described and explained below in conjunction with test examples, but these examples are not meant to limit the scope of the present invention.

### Test Example 1. Determination of inhibitory activity of the compound of the present invention on PARP1 enzyme

1. Experimental objective: The objective of this test example is to measure the inhibitory activity of the compound on PARP1 enzyme.
2. Experimental instruments:
Centrifuge (Eppendorf 5810R), pipettor (Eppendorf or Rainin), and
microplate reader (BioTek Synergy H1 or PerkinElmer Envision).

3. Experimental reagents:
PARP1 Chemiluminescent Assay Kit purchased from BPS bioscience, with catalog number 80569; and
20×PBST purchased from Thermo, with catalog number 28352, and PBS purchased from Gibco, with catalog number 10010023.

4. Experimental method:
In this experiment, the inhibitory activity of the compound on PARP1 enzyme was detected by a chemiluminescence method. This experiment was carried out in a 384-well plate. Firstly, the 384-well plate was coated with histone: a 5×histone solution was diluted 5-fold with PBS and added to the 384-well ELISA plate, with 25 µL per well, followed by incubation overnight at 4 degrees Celsius. The coated ELISA plate was washed with 1×PBST buffer, then blocked with a blocking solution (blocking buffer 3 in the kit) for 30 to 120 minutes, with 100 µL per well, and washed 3 to 6 times with 1×PBST buffer. A mixed solution of biotinylated substrate for PARP reaction, activated DNA, 10× PARP buffer, and water was added, with 12.5 µL per well. An experimental buffer (10% DMSO aqueous solution containing 1.25 mM DTT) was used to prepare compound solutions with different concentrations. The final concentration of detection was started with 100 nM, with 3-fold dilution to generate 8 concentrations, which were added to reaction wells of the 384-well plate, with 2.5 µL per well. 2.5 µL of a 10% DMSO aqueous solution containing 1.25 mM DTT was added to positive control group and blank wells, with 2.5 µL per well. 10 µL of a PARP1 enzyme solution prepared with 1 ×PARP buffer was further added to start the reaction, followed by centrifugation at 1000 rpm for 1 minute by the centrifuge. The reaction was carried out at room temperature for 60 minutes. After the reaction was complete, the reaction liquid was poured out, and after washing with 1×PBST buffer, a Streptavidin-HRP solution diluted 50-fold with Blocking buffer 3 was added, with 25 µL per well, followed by incubation at room temperature for 30 minutes. After the reaction liquid was poured out, the plate was washed 3 to 6 times with 1×PBST buffer. A luminescent reaction liquid of ECL substrate A and ELISA ECL substrate B mixed at 1 : 1 was added for a reaction, with 50 µL per well. Chemiluminescence reading was carried out immediately using BioTek Synergy H1 or Envision instrument.
5. Experimental data processing method:
By reading with BioTek Synergy H1 or Envision instrument, chemiluminescence readouts were recorded, the inhibition rate was calculated, and the concentration and inhibition rate were subjected to non-linear regression curve fitting by using Graphpad Prism software to obtain an IC₅₀ value. The inhibitory activities of the compounds of some examples of the present invention on PARP1 enzyme were as shown in the following table:

**Table 3**

| Examples | PARP1 IC₅₀ (nM) |
|---|---|
| Example 1 | 0.93 |
| Example 2 | 0.98 |
| Example 3 | 1.40 |
| Example 4 | 1.00 |
| Example 5 | 1.00 |
| Example 6 | 1.60 |
| Example 7 | 1.00 |
| Example 8 | 0.80 |
| Example 10 | 0.80 |
| Example 13 | 1.30 |
| Example 14 | 2.80 |
| Example 15 | 0.90 |
| Example 16 | 0.80 |
| Example 19 | 0.90 |
| Example 20 | 1.40 |
| Example 21 | 2.70 |

6. Experimental conclusion: The compounds as shown in the present invention showed excellent biological activity in the PARP1 enzyme inhibition experiment.

### Test Example 2. Determination of inhibitory activity of the compound of the present invention on PARP2 enzyme

1. Experimental objective: The objective of this test example is to measure the inhibitory activity of the compound on PARP2 enzyme.
2. Experimental instruments:
   Centrifuge (Eppendorf 5810R), pipettor (Eppendorf or Rainin), and
   microplate reader (BioTek Synergy H1 or PerkinElmer Envision).
3. Experimental reagents:
   PARP2 Chemiluminescent Assay Kit purchased from BPS bioscience, catalog number 80552; and
   20×PBST purchased from Thermo, catalog number 28352, and PBS purchased from Gibco, catalog number 10010023.
4. Experimental method: In this experiment, the inhibitory activity of the compound on PARP2 enzyme was detected by chemiluminescence method. This experiment was carried out in a 96-well plate. Firstly, the 96-well plate was coated with histone: a 5×histone solution was diluted 5-fold with PBS and added to the 96-well ELISA plate, with 50 µL per well, followed by incubation overnight at 4 degrees Celsius. The coated ELISA plate was washed with 1×PBST buffer, then blocked with a blocking solution (blocking buffer 3 in the kit) for 30 to 120 minutes, with 200 µL per well, and washed 3 to 6 times with 1×PBST buffer. A mixed solution of biotinylated substrate for PARP reaction, activated DNA, 10× PARP buffer, and water was added, with 25 µL per well. An experimental buffer (10% DMSO aqueous solution containing 1.25 mM DTT) was used to prepare compound solutions with different concentrations. The final concentration of detection was started with 10 µM, with 3-fold dilution to generate 8 concentrations, which were added to reaction wells of the 96-well plate, with 5 µL per well. 5 µL of a 10% DMSO aqueous solution containing 1.25 mM DTT was added to positive control group and blank wells, with 5 µL per well. 20 µL of a PARP2 enzyme solution prepared with 1 ×PARP buffer was further added to start the reaction, followed by centrifugation at 1000 rpm for 1 minute by the centrifuge. The reaction was carried out at room temperature for 60 minutes. After the reaction was complete, the reaction liquid was poured out, and after washing with 1×PBST buffer, a Streptavidin-HRP solution diluted 50-fold with Blocking buffer 3 was added, with 50 µL per well, followed by incubation at room temperature for 30 minutes. After the reaction liquid was poured out, the plate was washed 3 to 6 times with 1×PBST buffer. A luminescent reaction liquid of ECL substrate A and ELISA ECL substrate B mixed at 1 : 1 was added for reaction, with 100 µL per well. Chemiluminescence reading was carried out immediately using BioTek Synergy H1 or Envision instrument.
5. Experimental data processing method: By reading with BioTek Synergy H1 or Envision instrument, chemiluminescence readouts were recorded, the inhibition rate was calculated, and the concentration and inhibition rate were subjected to non-linear regression curve fitting by using Graphpad Prism software to obtain an IC₅₀ value.
6. Experimental conclusion: The compound as shown in the present invention showed higher selectivity for PARP2 in the experiment of the inhibition activity on PARP2 enzyme.

### Test Example 3. Determination of the inhibitory effect of the compound of the present invention on the proliferation activity of BRCA2 Knockout DLD-1 cells

1. Experimental objective: The objective of this test example is to measure the inhibitory effect of the compound on the proliferation activity of BRCA2 Knockout DLD-1 cells.
2. Experimental instruments:
Centrifuge (Eppendorf 5810R), pipettor (Eppendorf or Rainin), and
microplate reader (BioTek Synergy H1 or PerkinElmer Envision).

3. Experimental reagents:
BRCA2 Knockout DLD-1 cells purchased from Creative Biogene;
Cell Titer-Glo purchased from Promega, with catalog number G7573;
RPMI 1640 purchased from Gibco, with catalog number 22400089;
FBS purchased from Gibco, with catalog number 10091148; PBS purchased from Gibco, with catalog number 10010023;
pancreatic enzyme purchased from Gibco, with catalog number 25200056; and cell culture plates purchased from Corning, with catalog number 3610.

4. Experimental method: When using RPMI1640 medium containing 10% FBS to culture BRCA2 Knockout DLD-1 cells to an appropriate cell density, the cells were collected and adjusted with complete medium to an appropriate cell concentration, the cell suspension was spread in a 96-well plate, with 90 µL per well, and the plate was placed in a 37°C, 5% CO₂ incubator for overnight adherence. Compound solutions with different concentrations were prepared with DMSO and the culture medium, and a vehicle control was prepared. The compound solutions were added to the 96-well plate, with 10 µL per well, and the plate was placed in a 37°C, 5% CO₂ incubator for continued culturing for about 144 hours. CellTiter-Glo solution was added and uniformly mixed by shaking. After incubation for 10-30 minutes in the dark, reading was carried out with Synergy H1 or Envision microplate reader.
5. Experimental data processing method: The inhibition rate was calculated from the luminescence signal value, and the concentration and inhibition rate were subjected to non-linear regression curve fitting by using Graphpad Prism software to obtain an IC₅₀ value. The inhibitory effects of the compounds of some examples of the present invention on the proliferation activity of BRCA2 Knockout DLD-1 cells were as shown in the following table:

**Table 4**

| Examples | IC₅₀ (nM) |
|---|---|
| Example 1 | 1.2 |
| Example 2 | 1.9 |
| Example 3 | 3.2 |
| Example 7 | 2.3 |
| Example 8 | 1.6 |
| Example 10 | 3.5 |
| Example 13 | 1.0 |
| Example 15 | 2.4 |
| Example 16 | 2.7 |
| Example 19 | 1.5 |
| Example 20 | 1.4 |
| Example 21 | 5.1 |

6. Experimental conclusion: The compounds as shown in the present invention showed excellent biological activity in the experiment of the inhibition on the proliferation activity of BRCA2 Knockout DLD-1 cells.

### Test Example 4. Bidirectional permeability test of compounds through Caco-2 cell model

1. Experimental objective:
The objective of this experiment is to test the bidirectional permeability of compounds through a Caco-2 cell model.
2. Experimental instruments and materials:
Liquid chromatography-mass spectrometer, centrifuge, vortexer, pipette, 24-well test plate, acetonitrile solution with an internal standard added, Caco-2 cells (ATCC), Hank's balance solution (HBSS), and dimethyl sulfoxide (DMSO).
3. Experimental steps:
1) Culturing of Caco-2 monolayer cells: Caco-2 cells in good condition were selected and plated, the medium was changed every 2 to 3 days, and the cells were cultured for 21 to 28 days to form a dense cell monolayer for permeability testing.
2) Assessment of permeability of test compounds:
   a. 100 µL of transport buffer (HBSS containing 10 µM of a test compound, 0.5% of BSA, and 0.5% of DMSO) was added to the administration end of A to B.
   b. 300 µL of transport buffer (HBSS containing of 0.5% BSA) was added to the receiving end of A to B.
   c. 300 µL of transport buffer (HBSS containing 10 µM of the test compound, 0.5% of BSA, and 0.5% of DMSO) was added to the administration end of B to A.
   d. 100 µL of transport buffer (HBSS containing 0.5% of BSA) was added to the receiving end of B to A.
   e. The system was incubated for 2 h.
   d. A sample was taken, treated, and detected with mass spectrometry.

4. Chromatography conditions:
Instruments: Liquid chromatography;
Chromatographic column: Waters XSelect HSS T3 C18 (2.1 * 50 mm, 2.5 um);
Mobile phase: Phase A: an aqueous solution containing 0.1% of formic acid; and Phase B: an acetonitrile solution containing 0.1% of formic acid.

5. Mass spectrometry conditions:
Instruments: Model API4000 liquid chromatography-mass spectrometer;
The ion source is electrospray ionization source (ESI);
The detection method is positive ion detection;
The scanning method is selected reaction monitoring (MRM).

6. Experimental results: The bidirectional permeabilities of the compounds of the examples of the present invention through the Caco-2 cell model are as shown in the following table:

**Table 5**

| Compound No. | Pₐₚₚ (10⁻⁶ cm·s⁻¹) | | Pₐₚₚ(B-A)/Pₐₚₚ(A-B) |
|---|---|---|---|
| | A-B | B-A | |
| 1 | 12.77 | 19.23 | 1.5 |
| 2 | 12.20 | 19.00 | 1.6 |
| 7 | 5.19 | 15.69 | 3.0 |
| 8 | 14.76 | 15.62 | 1.1 |
| 10 | 9.80 | 15.35 | 1.6 |

7. Experimental conclusion: As can be seen from the experimental structure in the above table, the compounds in the examples of the present invention had higher permeability.

### Test Example 5. Pharmacokinetic assay in Balb/C mice

1. Experimental objective: To use Balb/C mice as test animals to study the pharmacokinetic behaviors of the following compounds of the examples in the plasma of mice after oral administration at a dose of 1 mg/kg.
2. Experimental scheme
2.1 Test drugs: the examples of the present invention, made in house.
2.2 Test animals: 6 Balb/C mice per example, male, from Shanghai Jiesijie Experimental Animal Co., Ltd., animal production license number (SCXK (Shanghai) 2013-0006 N0.311620400001794).
2.3 Drug preparation: 5 g of hydroxyethyl cellulose (HEC, CMC-Na, viscosity: 800-1200 Cps) was weighed and dissolved in 1000 mL of purified water, and 10 g of Tween 80 was added. These materials were uniformly mixed to form a clear solution.
2.05 mg was weighed, dissolved in the solution, uniformly shaken, crushed by a cell crusher for 1 min, and ultrasonicated for 15 minutes to obtain a suspension solution with a concentration of 0.1 mg/mL.
2.4 Administration: Balb/C mice, male, were administered p.o. at a dose of 1 mg/kg in a dosage volume of 10 mL/kg after they were fasted overnight.
2.5 Sample collection: After administration of the mice, 0.04 mL of blood was collected from the orbit at 0, 0.5, 1, 2, 4, 6, 8, and 24 h, and placed in EDTA-K₂ test tubes, which were centrifuged at 6000 rpm at 4°C for 6 min to separate plasma, and the plasma was stored at -80°C. The mice were fed at 4 h after administration.
2.6 Sample treatment:
1) 160 µL of acetonitrile was added to 40 µL of the plasma sample for pelleting, and the mixture was mixed and then centrifuged at 3500 × g for 5 to 20 minutes.
2) 100 µL of the treated supernatant solution was taken and subjected to LC/MS/MS to analyze the concentrations of the test compound.

2.7 Liquid phase analysis
• Liquid phase conditions: Shimadzu LC-20AD pump
• Mass spectrometry conditions: AB Sciex API 4000 mass spectrometer
• Chromatographic column: Phenomenex Gemiu 5 um C18 50 × 4.6 mm
• Mobile phase: liquid A: 0.1% aqueous formic acid solution, liquid B: acetonitrile
• Flow rate: 0.8 mL/min
• Elution time: 0-4.0 min, the eluent is as follows:

| Time/min | Liquid A | Liquid B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

3. Experimental results and analysis: The main pharmacokinetic parameters were calculated using WinNonlin 8.2. The results of the pharmacokinetic experiment in mice are as shown in the following table.

**Table 6**

| **Example No.** | Pharmacokinetic experiment (1 mg/kg) | | | |
|---|---|---|---|---|
| | Time to peak | Area under the curve | Plasma concentration | Half-life |
| | tₘₐₓ (h) | AUC₀₋ₜ (ng/mL*h) | Cₘₐₓ (ng/mL) | t_{1/2} (h) |
| 1 | 1.0 | 119232 | 9933 | 6.9 |
| 2 | 0.5 | 60550 | 8267 | 3.7 |
| 7 | 0.5 | 43203 | 6100 | 2.7 |
| 8 | 0.5 | 61917 | 6547 | 4.6 |
| 10 | 1.0 | 221085 | 8977 | 23.0 |

| | | | | |
|---|---|---|---|---|
| Note: 0.5% CMC-Na (1% Tween 80) | | | | |

4. Experimental conclusion: As can be seen from the results of the pharmacokinetic experiment in mice in the table, the compounds of the examples of the present invention exhibited good absorption and metabolic properties and performed well in both the exposure (AUC) and maximum plasma concentration Cₘₐₓ.

### Test Example 6. PK/PD study of compound in subcutaneous graft tumor model of nude mice with human breast cancer cell strain MDA-MB-436

1. Experimental objective: To evaluate the distribution of the compound in plasma and tumor in a subcutaneous graft tumor model of nude mice with human breast cancer cell strain MDA-MB-436 after a single oral dosage and the inhibitory effect thereof on PAR in tumor tissue.
2. Experimental instruments and reagents
   2.1 Instruments
      Refrigerator (BCD-268TN, Haier) and electric pipette-aid (Easypet 3, Eppendorf);
      Biological safety cabinet (BSC-1300II A2, Shanghai Boxun Industrial Co., Ltd. Medical Equipment Factory);
      Ultra-clean workbench (CJ-2F, Suzhou Fengshi Experimental Animal Equipment Co., Ltd.);
      Thermostat water bath (HWS-12, Shanghai Bluepard Instruments Co., Ltd.) and CO₂ incubator (Thermo-311, Thermo);
      Centrifuge (Centrifuge 5720R, Eppendorf);
      Full-automatic cell counter (Countess II, Life Technologies);
      Caliper (CD-6" AX, Mitutoyo Japan) and cell culture flask (T25/T75/T225, Corning);
      Electronic balance (CPA2202S, Sartorius) and electronic balance (BSA2202S-CW, Sartorius);
      Ultrasonic cleaner (115F0032, Shanghai Kudos) and water purifier (Pacific TII, Thermo);
      Magnetic stirrer (08-2G, Chijiu) and centrifuge (Centrifuge 5418R, Eppendorf);
      Small-scale vertical protein electrophoresis and transfer system (PowerPac Universal Power Supply, Bio-Rad);
      Microplate reader (H1MFD, Biotek) and molecular imaging system (ChemiDoc^{™}MP, Bio-Rad);
      Semi-dry membrane transfer instrument (690BR027087, Bio-Rad) and pipettor (METTLER TOLEDO/Eppendorf);
      Tissue grinder (TISS-48, Shanghai Jingxin Shiyan Shebei Kejibu); and
      Dry thermostat (MK200-2, Hangzhou Allsheng Instruments Co., Ltd.).
   2.2 Reagents
      DMEM medium (31600-034, Gibco) and fetal bovine serum (FBS) (10099-141C, Gibco);
      Insulin-transferrin-selenium (ITS-G) (41400-045, Gibco);
      Phosphate Buffer solution (PBS) (10010-023, Gibco) and Tween 80 (30189828, Sinopharm Chemical Reagent Co., Ltd.);
      Sodium carboxymethylcellulose (30036365, Sinopharm Chemical Reagent Co., Ltd.) and Matrigel Matrix (356234, Corning);
      Trans-Blot Turbo Transfer Pack (1704157, Bio-Rad);
      4-15% Criterion^{™} TGX^{™} Gel (5671085, Bio-Rad);
      GAPDH (D4C6R) Mouse (97166S, CST);
      IRDye^{®} 800CW Goat anti-Mouse IgG (H + L) (P/N 926-32210, LI-COR);
      IRDye^{®} 680RD Goat anti-Rabbit IgG (H + L) (P/N 926-68071, LI-COR);
      Poly/Mono-ADP Ribose (E6F6A)Rabbit mAb (83732S, CST);
      Pierce BCA Protein Assay Kit (23227, Thermo Fisher);
      QuickBlock Western Blocking Solution (P0252-500ml, Beyotime);
      NuPAGE^{®} Sample Reducing Agent (10×) (NP0009, Thermo Fisher);
      NuPAGE^{™} LDS Sample Buffer (4×) (NP0007, Thermo Fisher); and
      Pierce 20× TBS with Tween-20 (28360, Thermo Fisher).
3. Experimental method
   3.1 Animals
      BALB/c nude mice, 6-8 weeks old, ♀, purchased from the Laboratory Animal Business Department of Shanghai Institute of Planned Parenthood Research.
   3.2 Cell culture and cell suspension preparation
      a. A strain of MDA-MB-436 cells were taken from a cell bank, the cells were thawed with DMEM medium (DMEM + 10% FBS + 1% ITS-G), and the thawed cells were placed in a cell culture flask (with the cell type, date, the name of the culture operator, etc., being marked on the wall of the flask), which were placed in a CO₂ incubator for culturing (wherein the temperature of the incubator was 37°C, and the CO₂ concentration was 5%).
      b. The cells were passaged every three days, and after passage, the cells were placed in a CO₂ incubator to continue the culture. This process was repeated until the cell number meets the needs of in vivo efficacy.
      c. The cultured cells were collected, counted using a full-automatic cell counter, resuspended with PBS according to the counting results, mixed with Matrigel Matrix at 1 : 1 to a final concentration of 5 × 10⁷/mL, and then placed in an ice box for use.
   3.3 Cell inoculation
      a. Nude mouse was marked with a disposable universal ear tag for mice and rats before inoculation.
      b. At the time of inoculation, the cell suspension was mixed well, 0.1-1 mL of cell suspension was taken with a 1 mL syringe, and after removing air bubbles, the syringe was placed on an ice bag until use.
      c. The nude mouse was secured with the left hand. The position of the right back near the right shoulder of the nude mouse (inoculation site) was disinfected with a 75% alcohol cotton ball, and inoculation was carried out after 30 seconds.
      d. The nude mice were inoculated successively (inoculation with 0.1 mL of cell suspension per mouse).
   3.4 PK/PD experiment
      a. Grouping: On the basis of the tumor growth, when the tumor grew to a volume of 300-500 mm³, tumor-bearing mice were selected according to the experimental design and randomly grouped (3 mice per time point), and the PK/PD experiment was started.
      b. Fasting: All the tumor-bearing mice were fasted overnight with free access to water (fasting > 8 hours) before administration.
      c. Administration: Except for the blank control group, according to the experimental design time, a single oral administration was given, with the administration volume being 10 mL/kg.
      d. Sample collection: According to the design time, the experimental mice were euthanized by CO₂ asphyxiation and sampled, and 1 plasma sample and 3 tumor tissue samples were collected from each animal.

According to the design time, the mice were euthanized by CO₂ asphyxiation and sampled.

Plasma collection: After euthanasia of the mice, blood was collected from the heart, and the collected blood was added to a centrifuge tube containing EDTA-K₂, which was manually inverted 3 or 4 times, then placed on ice, and centrifuged at 4°C at 8000 rpm for 5 minutes. 100 µL of the centrifuged plasma was taken and transferred to a new labeled centrifuge tube, and 1 part was taken, quickly freezed with dry ice, placed in a refrigerator at -80°C ± 10°C for storage for PK detection.

Tumor tissue collection: After blood collection, the tumor tissue was stripped, and the stripped tumor tissue was divided into 3 parts (about 0.1 g per part), put into a labeled 2 mL centrifuge tube, and then transferred to a refrigerator at -80°C ± 10°C for storage for PK or PD detection. The remaining tumor-bearing mice were used to collect blank plasma and blank tumor tissue.

### 3.5 PK detection

a. Sample treatment:
1) 20% methanol water was added to the tumor tissue sample in an amount of 4 folds by weight, which was homogenized for 400 s at 40 Hz, 20 uL of the homogenate was taken, 100 uL of acetonitrile was added for pelleting, and after uniform mixing, the mixture was centrifuged at 3500 × g for 5-20 minutes after mixing.
2) 100 uL of the treated supernatant solution was taken and subjected to LC/MS/MS to analyze the concentrations of the test compound.

b. Liquid phase analysis
• Liquid phase conditions: Shimadzu LC-20AD pump
• Mass spectrometry conditions: AB Sciex API 4000 mass spectrometer
• Chromatographic column: Phenomenex Gemiu 5 um C18 50 × 4.6 mm
• Mobile phase: liquid A: 0.1% aqueous formic acid solution, liquid B: acetonitrile
• Flow rate: 0.8 mL/min
• Elution time: 0-4.0 min, the eluent is as follows:

| Time/min | Liquid A | Liquid B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

### 3.6 PD detection

a. Lysis of tumor tissue sample
   1 mL of a tumor lysis solution was added to the tumor tissue sample in each tube, steel balls were placed therein, and the tube was placed in a tissue grinder for tissue homogenization. After lysis on ice for 20 minutes followed by centrifugation for 5 minutes at 4°C at 10000 g in a freezing centrifuge, the protein supernatant was collected.
b. Preparation of protein sample
   Protein quantification was carried out by using a BCA protein quantitative kit. On the basis of a concentration, protein supernatant samples, 10× Sample Reducing Agent, 4× LDS Sample Buffer, and the lysis solution were prepared into protein loading solutions with the same concentration. The protein loading solution was put into a pre-heated dry thermostat and incubated at 100°C for 10 minutes for protein denaturation.
c. Western blot experiment of protein samples.
   1) Electrophoresis: 4-15% of Criterion^{™} TGX^{™} Gel protein gel was taken and added. Each protein sample was 15 µL. The protein sample was put into an electrophoresis tank, in which an electrophoresis solution was added, and underwent protein gel electrophoresis, running at 150 V for 60 minutes.
   2) Membrane transfer: Trans-Blot Turbo Transfer Pack Kit was used. Multilayer filter paper, a PVDF membrane, a protein gel, and thick filter paper were well placed in order and put into a membrane transfer instrument, and the program MIXED MW (2.5A-25V-7min) was selected for membrane transfer.
   3) Blocking and incubating antibodies: The PVDF membrane was taken out from the membrane transfer instrument, put into QuickBlock Western blocking solution, placed on a shaking table, and shaken at room temperature for 1 hour or more for protein blocking. The PVDF membrane was added to a PAR (1:500) or GAPDH (1:5000) primary antibody dilution solution diluted with the QuickBlock Western blocking solution and incubated overnight at 4 degrees Celsius. The primary antibody dilution was removed. The PVDF membrane was washed 6 times with 1×TBST. Goat anti-rabbit (1:3000) and murine fluorescence secondary antibody solution (1:5000) diluted with QuickBlock Western blocking solution were added, and after incubation for 1 hour at room temperature in the dark, the antibody dilution was removed, and the PVDF membrane was washed 6 times with 1×TBST.
   4) Imaging: The cleaned PVDF membrane was put into Biorad ChemiDoc^{™}MP imager for imaging. PAR and fluorescence imaging was performed. The Gapdh internal reference was imaged for fluorescence from IRDye 800 CW channel.

### 4. Experimental results

**Table 7**

| **Examples** | **Dose (mg/kg)** | **Time (h)** | **Average plasma concentration (ng/mL)** | **Average tumor tissue concentration (ng/g)** |
|---|---|---|---|---|
| AZD5305 | 0.3 | 1 | 1463 | 41 |
| | | 2 | 1373 | 100 |
| | | 4 | 1119 | 131 |
| | | 6 | 713 | 117 |
| | | 8 | 584 | 95 |
| | | 16 | 252 | 94 |
| | | 24 | 102 | 61 |
| | | 32 | 41 | 59 |
| | | 48 | 10 | 44 |
| | | 72 | 6 | 31 |

| **Examples** | **Dose (mg/kg)** | **Time (h)** | **Average plasma concentration (ng/mL)** | **Average tumor tissue concentration (ng/g)** |
|---|---|---|---|---|
| | | 1 | 2497 | 41 |
| | | 2 | 2120 | 148 |
| | | 4 | 2327 | 198 |
| | | 6 | 1767 | 232 |
| Example 1 | 0.3 | 8 | 1633 | 214 |
| | | 16 | 591 | 119 |
| | | 24 | 362 | 91 |
| | | 32 | 319 | 67 |
| | | 48 | 105 | 61 |
| | | 72 | 37 | 36 |

5. Experimental conclusion: In the MDA-MB-436 (breast cancer, BRCA1 mutation) model, the tumor and plasma concentrations of Example 1 were higher than those of AZD5305 within 24 h after single administration, and the degree of inhibition of PAR in tumor was comparable. The inhibition of PAR in tumor by Example 1 upon the single administration could last for 72 h, which was superior to AZD5305.

### Test Example 7. In vivo pharmacodynamics study of compound in subcutaneous graft tumor model of nude mice with human colorectal cancer cell strain DLD-1 BRCA2^{-/-}

1. Experimental objective: To evaluate the in vivo pharmacodynamics of the compound in a subcutaneous graft tumor model of nude mice with human colorectal cancer cell strain DLD-1 BRCA2^{-/-}.
2. Experimental instruments and reagents
   2.1 Instruments
      Refrigerator (BCD-268TN, Haier) and electric pipette-aid (Easypet 3, Eppendorf);
      Biological safety cabinet (BSC-1300II A2, Shanghai Boxun Industrial Co., Ltd. Medical Equipment Factory);
      Ultra-clean workbench (CJ-2F, Suzhou Fengshi Experimental Animal Equipment Co., Ltd.);
      Thermostat water bath (HWS-12, Shanghai Bluepard Instruments Co., Ltd.) and CO₂ incubator (Thermo-311, Thermo);
      centrifuge (Centrifuge 5720R, Eppendorf) and magnetic stirrer (08-2G, Chijiu);
      Full-automatic cell counter (Countess II, Life Technologies);
      Caliper (CD-6"AX, Mitutoyo Japan) and cell culture flask (T25/T75/T225, Corning);
      Electronic balance (CPA2202S, Sartorius) and electronic balance (BSA2202S-CW, Sartorius);
      Ultrasonic cleaner (115F0032, Shanghai Kudos) and water purifier (Pacific TII, Thermo).
   2.2 Reagents
      RPMI-1640 medium (22400-089, Gibco) and fetal bovine serum (FBS) (10099-141C, Gibco);
      Phosphate Buffer solution (PBS) (10010-023, Gibco) and Tween 80 (30189828, Sinopharm Chemical Reagent Co., Ltd.); and
      Sodium carboxymethylcellulose (30036365, Sinopharm Chemical Reagent Co., Ltd.).
3. Experimental operations and data processing
   3.1 Animals: BALB/c nude mice, 6-8 weeks old, ♀, purchased from the Laboratory Animal Business Department of Shanghai Institute of Planned Parenthood Research.
   3.2 Cell culture and cell suspension preparation
      a. A strain of DLD-1 BRCA2^{-/-} cells were taken from a cell bank, the cells were thawed with RPMI-1640 medium (RPMI-1640 + 10% FBS), and the thawed cells were placed in a cell culture flask (with the cell type, date, the name of the culture operator, etc., being marked on the wall of the flask), which were placed in a CO₂ incubator for culturing (wherein the temperature of the incubator was 37°C, and the CO₂ concentration was 5%).
      b. The cells were passaged every three days, and after passage, the cells were placed in a CO₂ incubator to continue the culture. This process was repeated until the cell number meets the needs of in vivo efficacy.
      c. The cultured cells were collected, counted using a full-automatic cell counter, resuspended with PBS according to the counting results, and prepared into a cell suspension (at a density of 5 × 10⁷/mL), which was then placed in an ice box for use.
   3.3 Cell inoculation
      a. Nude mouse was marked with a disposable universal ear tag for mice and rats before inoculation.
      b. At the time of inoculation, the cell suspension was mixed well, 0.1-1 mL of cell suspension was taken with a 1 mL syringe, and after removing air bubbles, the syringe was placed on an ice bag until use.
      c. The nude mouse was secured with the left hand. The position of the right back near the right shoulder of the nude mouse (inoculation site) was disinfected with a 75% alcohol cotton ball, and inoculation was carried out after 30 seconds.
      d. The nude mice were inoculated successively (inoculation with 0.1 mL of cell suspension per mouse).
   3.4 Tumor volume measurement, grouping and administration in tumor-bearing mice
      a. Tumor was measured on days 10-18 after inoculation depending on the tumor growth, and the tumor size was calculated. Tumor volume calculation: tumor volume (mm3) = length (mm) x width (mm) x width (mm)/2
      b. According to the weight of tumor-bearing mice and the size of the tumor, the mice were grouped by random grouping.
      c. According to the grouping results, the test drug was started to be administered (administration method: oral administration; administration volume: 10 mL/kg; administration frequency: once/day; administration cycle: 28 days; vehicle: 0.5% CMC-Na/1% Tween 80).
      d. Tumors were measured and weighed twice a week after starting administration of test drugs.
      e. Animals were euthanized at the end of the experiment.
      f. The data was processed with software such as Excel. Calculation of tumor growth inhibition (TGI) (%) of compound: when the tumor did not regress, TGI (%) = [(1 - (average tumor volume at the end of administration in a treatment group - average tumor volume at the beginning of administration in the treatment group))/(average tumor volume at the end of administration in the solvent control group - average tumor volume at the beginning of administration in the solvent control group)] × 100%. When the tumor regressed, TGI(%) = [1 - (average tumor volume at the end of administration in a treatment group - average tumor volume at the beginning of administration in the treatment group)/average tumor volume at the beginning of administration in the treatment group] x 100%.
   1.4 Experimental results and experimental conclusion:
      Compounds 1, 2, and 7 of the examples of the present invention exhibited excellent tumor inhibition effects in this model experiment, with the tumor inhibition rate TGI (%) > 90% and the tumor inhibition rate TGI (%) of preferred compounds > 150% in the tumor-bearing animals, without significant reduction of the animal weight.

### III. Study on the salt and crystalline form of the compound of Example 1

### 1.1 Experimental instruments

### 1.1.1 Some parameters of physical and chemical testing instruments

| | | |
|---|---|---|
| XRPD | Instrument model | BRUKER D8 ADVANCE |
| | Diffracted ray | Cu/K-α1 (40 kV, 25 mA) |
| | Scan rate | 0.02°/S (2θ value) |
| | Scan range | 4-40° or 3-60° (2θ value) |
| DSC | Instrument model | NETZSCH DSC 214 polyma |
| | Purge gas | Nitrogen |
| | Purge rate | 40 mL/min |
| | Heating rate | 10°C/min |
| | Temperature range | 25-250/300°C |
| | Tray type | Aluminum tray |
| TGA | Instrument model | NETZSCH TG 209 Tarsus |
| | Purge gas | Nitrogen |
| | Purge rate | 40 mL/min |
| | Heating rate | 10°C/min |
| | Temperature range | Room temperature to 300/350°C |
| | Tray type | Al₂O₃ |

### 1.2 Instrument and liquid phase analysis conditions

### 1.2.1 Instruments and equipment

| Name of instrument | Model |
|---|---|
| Analytical balance | Sartorius BSA224S-CW |
| Water purifier | Milli-Q Plus, Millipore |
| High performance liquid chromatograph | Agilent1260 |
| Pump | Agilent G1311B |
| Sample injector | G1329B |
| Column oven | G1316A |
| Detector | G1315D |

### 1.2.2 Chromatographic conditions

Chromatographic column: Waters Xbridge C18 (4.6 mm * 150 mm, 3.5 µm)
Flow rate: 1.0 mL/min Column temperature: 40°C Detection wavelength: 260 nm
Injection volume: 5 µL Running time: 15 min Diluent: methanol
Mobile phase: A: water (0.05% trifluoroacetic acid); B: acetonitrile (0.05% trifluoroacetic acid)

| T (min) | A (%) | B (%) |
|---|---|---|
| 0.00 | 95.0 | 5.0 |
| 8.00 | 70.0 | 30.0 |
| 12.00 | 10.0 | 90.0 |
| 12.01 | 95.0 | 5.0 |
| 15.00 | 95.0 | 5.0 |

### 1.3 Preparation of different crystalline forms of the compound of Example 1

### 1.3.1 Preparation of hydrochloride crystalline Form A

20 mg of the compound of Example 1 was weighed, added to 0.2 mL of ethanol, and stirred at 50°C for suspension, and 0.06 mL of a solution of 1M hydrochloric acid in methanol was added to the system and the system was dissolved until clear, then precipitation occurred. The system was reacted for 1 h, then cooled to room temperature, stirred for 2 h, and centrifuged to obtain a solid, which was then dried to finally obtain the hydrochloride crystalline Form A. After detection and analysis, the crystalline form had an XRPD pattern as shown in FIG. 3 and a DSC thermogram as shown in FIG. 4.

### 1.3.2 Preparation of sulfate crystalline Form A

20 mg of the compound of Example 1 was weighed, added to 0.2 mL of ethanol, and stirred at 50°C for suspension, and 0.06 mL of a solution of 1M sulfuric acid in methanol was added to the system and the system was dissolved until clear, then precipitation occurred. The system was reacted for 1 h, then cooled to room temperature, stirred for 2 h, and centrifuged to obtain a solid, which was then dried to finally obtain the sulfate crystalline Form A. After detection and analysis, the crystalline form had an XRPD pattern as shown in FIG. 5 and a DSC thermogram as shown in FIG. 6.

### 1.3.3 Preparation of methanesulfonate crystalline Form A

20 mg of the compound of Example 1 was weighed, added to 0.2 mL of ethanol, and stirred at 50°C for suspension, and 0.06 mL of a solution of 1M methanesulfonic acid in methanol was added to the system and the system was dissolved until clear. After cooling to room temperature, a solid precipitated out. After stirring at room temperature for 2 h, the system was centrifuged to obtain a solid, which was then dried to finally obtain the methanesulfonate crystalline Form A. After detection and analysis, the crystalline form had an XRPD pattern as shown in FIG. 7 and a DSC thermogram as shown in FIG. 8.

### 1.3.4 Preparation of p-toluenesulfonate crystalline Form A

20 mg of the compound of Example 1 was weighed, added to 0.2 mL of ethanol, and stirred at 50°C for suspension, and 0.06 mL of a solution of 1M p-toluenesulfonic acid in methanol was added to the system and the system was dissolved until clear, then precipitation occurred. The system was reacted for 1 h, then cooled to room temperature, stirred for 2 h, and centrifuged to obtain a solid, which was then dried to finally obtain the p-toluenesulfonate crystalline Form A. After detection and analysis, the crystalline form had an XRPD pattern as shown in FIG. 9 and a DSC thermogram as shown in FIG. 10.

### 1.3.5 Preparation of p-toluenesulfonate crystalline Form B

20 mg of p-toluenesulfonate crystalline Form A was weighed, added to 0.2 mL of tetrahydrofuran, and the mixture was slurried at 50°C for suspension for 7 days, and centrifuged to obtain a solid, which was then dried to finally obtain the p-toluenesulfonate crystalline Form B. After detection and analysis, the crystalline form had an XRPD pattern as shown in FIG. 11 and a DSC thermogram as shown in FIG. 12.

### 1.3.6 Preparation of p-toluenesulfonate crystalline Form C

20 mg of p-toluenesulfonate crystalline Form A was weighed, added to 0.2 mL of 1,4-dioxane, and the mixture was slurried at 50°C for suspension for 7 days, and centrifuged to obtain a solid, which was then dried to finally obtain the p-toluenesulfonate crystalline Form C. After detection and analysis, the crystalline form had an XRPD pattern as shown in FIG. 13 and a DSC thermogram as shown in FIG. 14.

### 1.3.7 Preparation of p-toluenesulfonate dihydrate crystalline Form A

20 mg of p-toluenesulfonate crystalline Form A was weighed, added to 0.2 mL of water, and the mixture was slurried at room temperature for suspension for 12 h, and centrifuged to obtain a solid, which was then dried to finally obtain the p-toluenesulfonate hydrate crystalline Form A. After detection and analysis, the crystalline form had an XRPD pattern as shown in FIG. 15, a DSC thermogram as shown in FIG. 16, and a TGA thermogram as shown in FIG. 17.

The composition of p-toluenesulfonate hydrate crystalline Form A was determined by the following method.

### 1) Quantification of water content in p-toluenesulfonate hydrate crystalline Form A

The water content of the p-toluenesulfonate hydrate crystalline Form A was mainly determined by the weight loss by TGA in combination with the water content result determined by Karl Fischer moisture analyzer:
It can be concluded from FIG. 17 that the weight loss by TGA was about 6.02%.

The water content was measured three times in total by Karl Fischer moisture analyzer, and the results were as follows:

| | | | | | | | |
|---|---|---|---|---|---|---|---|
| First | 5.918% | Second | 5.938% | Third | 5.938% | Average value | 5.945% |

The results of the three water content measurements were comparable, and the average water content was 5.945%, which was in good agreement with the weight loss by TGA.

### 2) Liquid phase analysis of free base content

Taking the compound of Example 1 as a control, the free base content of the p-toluenesulfonate hydrate crystalline Form A of the compound was determined by HPLC external standard method. The results were as follows:

**Table 8**

| | First measurement value | Second measurement value |
|---|---|---|
| Theoretical value of free base content (%) | 70.35% | 70.59% |
| Actual value of free base content (%) | 65.96% | |

According to the above water content determination results and free base content determination data, the ratio of free base : p-toluenesulfonic acid : water in the p-toluenesulfonate hydrate crystalline Form A was finally determined to be 1 : 1 : 2.

In summary, the crystalline Form A of the p-toluenesulfonate hydrate was the p-toluenesulfonate dihydrate crystalline Form A.

### 1.3.8 Preparation of benzenesulfonate crystalline Form A

20 mg of the compound of Example 1 was weighed, added to 0.2 mL of ethanol, the mixture was stirred at 50°C for suspension, 0.06 mL of a solution of 1M benzenesulfonic acid in methanol was added to the system and the system was dissolved until clear. After cooling to room temperature, a solid precipitated out. After stirring at room temperature for 2 h, the system was centrifuged to obtain a solid, which was then dried to finally obtain the benzenesulfonate crystalline Form A. After detection and analysis, the crystalline form had an XRPD pattern as shown in FIG. 18 and a DSC thermogram as shown in FIG. 19.

### 1.4 Study on polymorphism of salt form of Example 1

1.4.1 Study on polymorphism of hydrochloride: About 20 mg of the hydrochloride crystalline Form A was weighed into a small glass flask, 200 µL of an organic solvent was added, and the mixture was slurried at 50°C for 7 days. The results were as follows:

**Table 9**

| Solvent | Phenomenon | Slurrying result |
|---|---|---|
| IPA | Suspension | Crystalline Form A |
| THF | Suspension | Crystalline Form A |
| DCM | Suspension | Crystalline Form A |
| EA | Suspension | Crystalline Form A |
| Acetone | Suspension | Crystalline Form A |

Results and discussion: The hydrochloride crystalline Form A did not undergo crystalline transformation in the above solvent and was stable in the above solvent.

### 1.4.2 Study on polymorphism of sulfate

About 20 mg of the sulfate crystalline Form A was weighed into a small glass flask, 200 µL of an organic solvent was added, and the mixture was slurried at 50°C for 7 days. The results were as follows:

**Table 10**

| Solvent | Phenomenon | Slurrying result |
|---|---|---|
| MeOH | Suspension | Crystalline Form A |
| EtOH | Suspension | Crystalline Form A |
| ACN | Suspension | Crystalline Form A |
| THF | Suspension | Crystalline Form A |
| 2-Me-THF | Suspension | Crystalline Form A |
| EA | Suspension | Crystalline Form A |
| Acetone | Suspension | Crystalline Form A |
| 1,4-Dioxane | Suspension | Crystalline Form A |
| Toluene | Suspension | Crystalline Form A |

Results and discussion: The sulfate crystalline Form A was stable in the above solvent, which was beneficial to the subsequent drug development.

### 1.4.3 Study on polymorphism of methanesulfonate

About 20 mg of the methanesulfonate crystalline Form A was weighed into a small glass flask, 200 µL of an organic solvent was added, and the mixture was slurried at 50°C for 7 days. The results were as follows:

**Table 11**

| Solvent | Phenomenon | Slurrying result |
|---|---|---|
| EA | Suspension | Crystalline Form A |
| Acetone | Suspension | Crystalline Form A |

Results and discussion: The methanesulfonate crystalline Form A was stable, and the preparation process had strong operability and could give a stable single crystalline form.

### 1.4.4 Study on polymorphism of p-toluenesulfonate

About 20 mg of the polymorph A of the p-toluenesulfonate was weighed into a small glass flask, 200 µL of an organic solvent was added, and the mixture was slurried at 50°C for 7 days. The results were as follows:

**Table 12**

| Solvent | Phenomenon | Slurrying result |
|---|---|---|
| MeOH | Suspension | Crystalline Form A |
| EtOH | Suspension | Crystalline Form A |
| ACN | Suspension | Crystalline Form A |
| THF | Suspension | Crystalline Form B |
| 2-Me-THF | Suspension | Crystalline Form B |
| EA | Suspension | Crystalline Form A |
| Acetone | Suspension | Crystalline Form A |
| 1,4-Dioxane | Suspension | Crystalline Form C |
| Toluene | Suspension | Crystalline Form A |
| H₂O | Suspension | Hydrate Crystalline Form A |

Results and discussion: Polymorph B, polymorph C, and hydrate crystalline Form A of the p-toluenesulfonate were screened out by the above experiment.

### 1.4.5 Study on polymorphism of p-toluenesulfonate hydrate

1.4.5.1 Slurry conversion test; About 10 mg of the p-toluenesulfonate dihydrate crystalline Form A was weighed into a small glass flask, 200 µL of an organic solvent was added, and the mixture was slurried at 40°C for 7 days. The results were as follows:

**Table 13**

| Solvent | Phenomenon | Slurrying result |
|---|---|---|
| methanol | Suspension | p-Toluenesulfonate crystalline Form A |
| Ethanol | Suspension | p-Toluenesulfonate crystalline Form A |
| Isopropanol | Suspension | Still as hydrate crystalline Form A |
| Acetonitrile | Suspension | p-Toluenesulfonate crystalline Form A |
| Acetone | Suspension | p-Toluenesulfonate crystalline Form A |
| 2-Butanone | Suspension | Still as hydrate crystalline Form A |
| Tetrahydrofuran | Suspension | Still as hydrate crystalline Form A |
| 2-Methyltetrahydrofuran | Suspension | Still as hydrate crystalline Form A |
| Dichloromethane | Suspension | p-Toluenesulfonate crystalline Form A |
| Trichloromethane | Suspension | p-Toluenesulfonate crystalline Form A |
| Toluene | Suspension | Still as hydrate crystalline Form A |
| Methyl tert-butyl ether | Suspension | Still as hydrate crystalline Form A |
| Ethyl acetate | Suspension | Still as hydrate crystalline Form A |
| Ethyl formate | Suspension | p-Toluenesulfonate crystalline Form A |
| 1,4-Dioxane | Suspension | Still as hydrate crystalline Form A |

1.4.5.2 Results and discussion: It was demonstrated that the p-toluenesulfonate dihydrate crystalline Form A was relatively stable, which was beneficial to the subsequent drug development.

### 1.5 Hygroscopicity determination

1.5.1 Experimental objective: To investigate the hygroscopicity of crystalline forms of different salts of the compound under different relative humidity conditions.

1.5.2 Experimental scheme: The crystalline forms of the salt of the compound were placed in saturated water vapor at different relative humidities to reach a dynamic equilibrium between the crystalline forms of the salt of the compound and the water vapor, and the weight gain percentage after moisture absorption after equilibrium was calculated.

### 1.5.3 Experimental results:

1) The sulfate crystalline Form A had a weight gain of 1.785% after moisture absorption under RH 80% condition, indicating weak hygroscopicity. After 1 cycle of moisture absorption and desorption under 0-95% relative humidity condition, there was no change in the XRPD pattern of the sulfate crystalline Form A, that is, no crystalline form transformation occurred.
2) The p-toluenesulfonate crystalline Form A had a weight gain of 9.00% after moisture absorption under RH 80% condition, indicating weak hygroscopicity. After 1 cycle of moisture absorption and desorption under 0-95% relative humidity condition, there was no change in the XRPD pattern of the p-toluenesulfonate crystalline Form A, that is, no crystalline form transformation occurred.
3) The p-toluenesulfonate dihydrate crystalline Form A had a weight gain of 1.906% after moisture absorption under RH 80% condition, indicating weak hygroscopicity. After 1 cycle of moisture absorption and desorption under 0-95% relative humidity condition, there was no change in the XRPD pattern of the p-toluenesulfonate dihydrate crystalline Form A, that is, no crystalline form transformation occurred.

1.5.4 Experimental conclusion: The above crystalline forms did not undergo crystalline form transformation under different relative humidity conditions.

### 1.6. Solid stability experiment

1.6.1 Experimental objective: To investigate the physical and chemical stability of crystalline forms of different salts of the compound under the conditions of a high temperature of 60°C, a high humidity of RH = 92.5%, and a high temperature and high humidity of 50°C and 75% RH.

1.6.2 Experimental scheme: About 1 mg of crystalline forms of different salts were taken and investigated at a high temperature of 60°C, a high humidity of RH = 92.5%, and a high temperature and high humidity of 50°C and 75% RH for 7 and 14 days. The changes of related substances were calculated for the crystalline forms of the salts by a chromatographic peak area normalization method.

1.6.3 Experimental results:

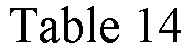

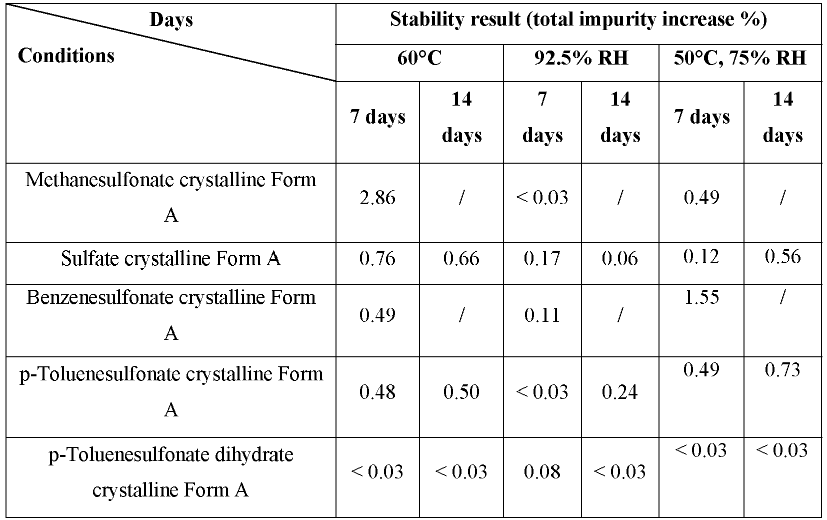

1.6.4 Experimental conclusion: Compared with the stability results, the p-toluenesulfonate dihydrate crystalline Form A, after salt formation, could be significantly improved for its solid stability and was relatively stable under all the high temperature, high humidity, and high temperature and high humidity conditions, and no impurity increase was found, thereby meeting the requirements of the subsequent drug development. The benzenesulfonate crystalline Form A was relatively stable under high temperature and high humidity conditions and no significant impurity increase was found, indicating that the benzenesulfonate crystalline Form A was insensitive to both the changes of temperature and humidity, thereby satisfying the subsequent drug storage. The p-toluenesulfonate crystalline Form A and the sulfate crystalline Form A could both remain stable after being placed for a period of time under various conditions and no significant impurity increase was found, indicating that the p-toluenesulfonate crystalline Form A and the sulfate crystalline Form A could effectively improve the operational flexibility of the subsequent development of drug preparations. The methanesulfonate crystalline Form A was relatively stable under the high humidity and high temperature and high humidity conditions and no significant impurity increase was found, indicating that the methanesulfonate crystalline Form A was extremely insensitive to humidity, and no significant impurity increase was also found when the temperature was raised to 50°C at 75% humidity, which reduced the harshness of the conditions for the subsequent drug storage.

### 1.7. Solubility experiment in different vehicles

1.7.1 Experimental objective: To compare the solubilities of the p-toluenesulfonate dihydrate crystalline Form A and the sulfate crystalline Form A in vehicles such as water, artificial simulated gastric fluid (FaSSGF), fasted artificial simulated intestinal fluid (FaSSIF), and non-fasted artificial simulated intestinal fluid (FeSSIF).

1.7.2 Experimental scheme: About 1 mg of the crystalline form of the salt was suspended in different media for 2 hours, and the solubility of the compound was determined at 37°C by an HPLC external standard method.

1.7.3 Experimental results:

**Table 15**

| Vehicle/solubility (mg/mL) | p-Toluenesulfonate dihydrate crystalline Form A | Sulfate crystalline Form A |
|---|---|---|
| Water | 0.308 | 0.696 |
| FaSSGF (simulated gastric fluid, fasted) | > 0.437 | > 0.466 |
| FaSSIF (simulated intestinal fluid, fasted) | 0.147 | 0.133 |
| FeSSIF (simulated intestinal fluid, fed) | > 0.340 | > 0.347 |

1.7.4 Experimental conclusion: The solubilities of the crystalline forms of the two salts as described above were substantially the same in the gastrointestinal simulated liquids and water medium, and there was no significant difference.

### 1.8. PK study of crystalline forms of different salts in rats

1.8.1 Experimental objective: To use SD rats as test animals to study the pharmacokinetic behaviors of the sulfate crystalline Form A and the p-toluenesulfonate dihydrate crystalline Form A in rats (plasma) after single oral administration and compare the changes in exposure; and the bioavailabilities of the sulfate crystalline Form A and the p-toluenesulfonate dihydrate crystalline Form A after oral administration.

1.8.2 Experimental scheme: The sulfate crystalline Form A and the p-toluenesulfonate dihydrate crystalline Form A were both uniformly suspended with an aqueous solution containing 0.5% HPMC K4M and then administered to rats by gavage, with three rats in parallel, and the dosage was 5 mg/kg for sulfate crystalline Form A (as a clear solution), and 5 mg/kg and 20 mg/kg for p-toluenesulfonate dihydrate crystalline Form A (both as suspensions).

1.8.3 Experimental results:

**Table 16**

| Parameters | p-Toluenesulfonate dihydrate crystalline Form A - 5 mpk | p-Toluenesulfonate dihydrate crystalline Form A - 20 mpk | Sulfate crystalline Form A - 5 mpk |
|---|---|---|---|
| tₘₐₓ (h) | 1.0 | 4.0 | 2.0 |
| Cₘₐₓ (ng/mL) | 17433 | 53400 | 14467 |
| AUC₀₋ₜ (ng/mL*h) | 145036 | 558440 | 118078 |
| AUC_{0-∞} (ng/mL*h) | 146622 | 587652 | 119241 |
| t_{1/2} (h) | 3.8 | 5.7 | 3.7 |
| MRT_{0-∞} (h) | 5.4 | 7.5 | 5.3 |
| F% | 92 | 93 | 75 |

1.8.4 Experimental conclusion: As can be seen from the above data, the PK oral bioavailabilities of the sulfate crystalline Form A and the p-toluenesulfonate dihydrate crystalline Form A were both relatively high and the exposure of the p-toluenesulfonate dihydrate crystalline form had a linear relationship with the dosage (5 mpk and 20 mpk).

### 1.9 Single crystal experiment of p-toluenesulfonate dihydrate crystalline form

1.9.1 Experimental objective: To confirm the structure of the toluenesulfonate dihydrate crystalline form.

1.9.2 Experimental scheme: At room temperature, 50 mg of p-toluenesulfonate was weighed and added to 20 ml of methanol, 10 ml of methyl tert-butyl ether, and 5 ml of n-heptane, mixed and stirred for 0.5 hours, and the mixture was filtered through a 0.22 filter membrane. The filtrate was diluted by adding 2 ml of methanol, the diluted solution was placed in a beaker, the beaker was sealed with tin foil paper, pierced with small holes, and placed in a vibration-free ventilated place, so that the solvent naturally and slowly volatilized to dryness to obtain a granular crystal.

1.9.3 Data characterization and results

1.9.3.1 Instrument parameters
Single crystal determination instruments and parameters: a Rigaku Saturn70 CCD diffractometer Mo-Kα radiation.
Wavelength: γ = 0.71073 Å Temperature: 113 K
Scan range (theta range for data collection) 2.3-32.3°
Related data of single crystal structure: Empirical formula: C₇H₇O₃S·C₂₃H₂₆N₅O₂·2(H₂O)
Formula weight: 611.70
Crystal system and space group: Triclinic, P₋₁
Unit cell dimensions:
   *a* = 7.0829 (3) Å alpha = 95.646 (3)°
      *b* = 8.2802 (4) Å beta = 91.317 (3)°
      *c* = 26.7236 (9) Å gamma = 109.519 (4)°
   Cell volume: 1467.43 (11) Å³
   Calculated density: Z *=* 2, *D_{c}* = 1.384 Mg m⁻³
   Absorption coefficient: *µ*(Mo*Kα*) = 0.167 mm⁻¹, F(000) = 648
   R(int) = 0.056 (Reflections collected/unique) for 6640/9435
   Final R indices [*I* > 2σ(*I*)] R1 = 0.0597, wR2 = 0.1447
   R indices (all data) R1 = 0.0868, wR2 = 0.1676

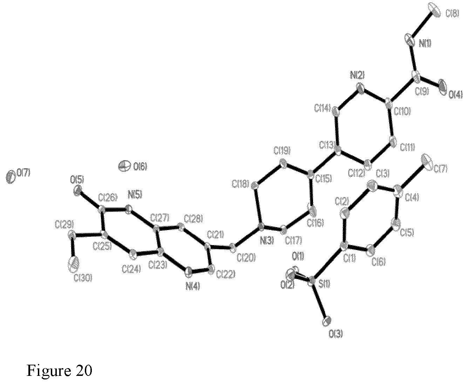

1.9.3.2 Experimental results: A single crystal structure as shown in FIG. 20 was obtained.

## Claims

1. An acid addition salt of a compound represented by general formula (I) or a stereoisomer thereof, 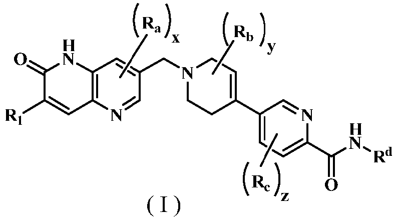 **characterized in that**
R₁ is selected from hydrogen, deuterium, halogen, nitro, hydroxyl, mercapto, cyano, C₁₋₆ alkyl, C₁₋₆ deuteroalkyl, C₁₋₆ haloalkyl, C₁₋₆ hydroxyalkyl, C₁₋₆ alkoxy, C₂₋₆ alkenyl, C₂₋₆ alkynyl, C₃₋₈ cycloalkyl, or 3- to 8-membered heterocyclyl; preferably C₁₋₃ alkyl, C₁₋₃ haloalkyl, C₂₋₄ alkynyl, or C₃₋₆ cycloalkyl;
R^{a} is independently selected from hydrogen, deuterium, halogen, nitro, hydroxyl, mercapto, cyano, C₁₋₆ alkyl, C₁₋₆ deuteroalkyl, C₁₋₆ haloalkyl, C₁₋₆ hydroxyalkyl, C₁₋₆ alkoxy, C₂₋₆ alkenyl, or C₂₋₆ alkynyl;
R^{b} is independently selected from hydrogen, deuterium, halogen, nitro, hydroxyl, mercapto, cyano, C₁₋₆ alkyl, C₁₋₆ deuteroalkyl, C₁₋₆ haloalkyl, C₁₋₆ hydroxyalkyl, C₁₋₆ alkoxy, C₂₋₆ alkenyl, or C₂₋₆ alkynyl; preferably hydrogen, deuterium, halogen, C₁₋₃ alkyl, C₁₋₃ deuteroalkyl, or C₁₋₃ haloalkyl;
R^{c} is independently selected from hydrogen, deuterium, halogen, nitro, hydroxyl, mercapto, cyano, C₁₋₆ alkyl, C₁₋₆ deuteroalkyl, C₁₋₆ haloalkyl, C₁₋₆ hydroxyalkyl, C₁₋₆ alkoxy, C₂₋₆ alkenyl, C₂₋₆ alkynyl, C₃₋₈ cycloalkyl, or 3- to 8-membered heterocyclyl; preferably selected from hydrogen, deuterium, halogen, cyano, C₁₋₃ alkyl, C₁₋₃ haloalkyl, C₁₋₃ alkoxy, C₂₋₄ alkynyl, or C₃₋₆ cycloalkyl;
R^{d} is selected from hydrogen, deuterium, halogen, nitro, hydroxyl, mercapto, cyano, C₁₋₆ alkyl, C₁₋₆ deuteroalkyl, C₁₋₆ haloalkyl, C₁₋₆ hydroxyalkyl, C₁₋₆ alkoxy, C₂₋₆ alkenyl, C₂₋₆ alkynyl, C₃₋₈ cycloalkyl, or 3- to 8-membered heterocyclyl, wherein the C₁₋₆ alkyl, C₁₋₆ deuteroalkyl, C₁₋₆ haloalkyl, C₁₋₆ hydroxyalkyl, C₁₋₆ alkoxy, C₂₋₆ alkenyl, C₂₋₆ alkynyl, C₃₋₈ cycloalkyl, or 3- to 8-membered heterocyclyl can be optionally further substituted with one or more of hydrogen, deuterium, halogen, nitro, hydroxyl, mercapto, cyano, C₁₋₃ alkyl, C₁₋₃ deuteroalkyl, C₁₋₃ haloalkyl, C₁₋₃ hydroxyalkyl, C₁₋₃ alkoxy, C₂₋₄ alkenyl, and C₂₋₄ alkynyl; R^{d} is preferably selected from cyano, C₁₋₃ alkyl, C₁₋₃ haloalkyl, C₁₋₃ alkoxy, cyano-substituted C₁₋₃ alkyl, cyano-substituted C₃₋₆ cycloalkyl, C₃₋₆ cycloalkyl, or 3- to 6-membered heterocyclyl;
x is 1, 2, or 3;
y is 1, 2, 3, or 4;
z is 1, 2, 3, or 4; and
the acid in the acid addition salt is an inorganic acid or organic acid; preferably, the inorganic acid is selected from hydrochloric acid, sulfuric acid, nitric acid, hydrobromic acid, hydrofluoric acid, hydroiodic acid, or phosphoric acid; the organic acid is selected from 2,5-dihydroxybenzoic acid, 1-hydroxy-2-naphthoic acid, acetic acid, ethanesulfonic acid, dichloroacetic acid, trichloroacetic acid, acetohydroxamic acid, adipic acid, benzenesulfonic acid, 4-chlorobenzenesulfonic acid, benzoic acid, 4-acetylaminobenzoic acid, 4-aminobenzoic acid, decanoic acid, hexanoic acid, octanoic acid, cinnamic acid, citric acid, cyclohexylsulfamic acid, camphorsulfonic acid, aspartic acid, camphanic acid, gluconic acid, glucuronic acid, glutamic acid, isoascorbic acid, lactic acid, malic acid, mandelic acid, pyroglutamic acid, tartaric acid, dodecylsulfuric acid, dibenzoyltartaric acid, ethane-1,2-disulfonic acid, ethanesulfonic acid, formic acid, fumaric acid, galactonic acid, gentisuric acid, glutaric acid, 2-ketoglutaric acid, glycolic acid, hippuric acid, hydroxyethylsulfonic acid, lactobionic acid, ascorbic acid, aspartic acid, lauric acid, camphanic acid, maleic acid, malonic acid, methanesulfonic acid, 1,5-naphthalenedisulfonic acid, naphthalene-2-sulfonic acid, nicotinic acid, oleic acid, orotic acid, oxalic acid, palmitic acid, pamoic acid, propanoic acid, salicylic acid, 4-aminosalicylic acid, sebacic acid, stearic acid, succinic acid, thiocyanic acid, undecylenoic acid, trifluoroacetic acid, benzenesulfonic acid, p-methylbenzenesulfonic acid, or L-malic acid.

2. The acid addition salt of the compound or a stereoisomer thereof according to claim 1, **characterized in that** the compound is as shown below: 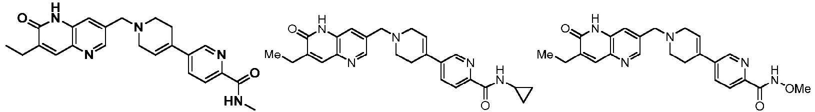 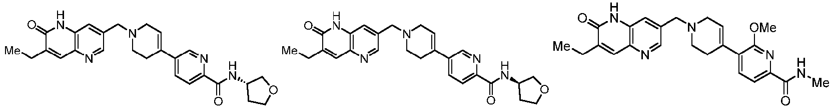 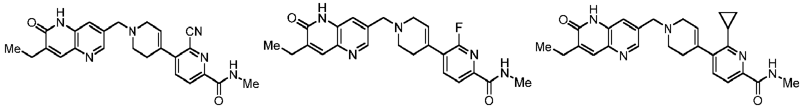 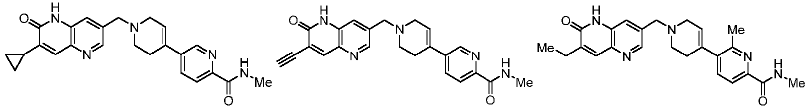 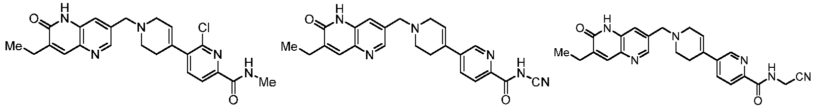 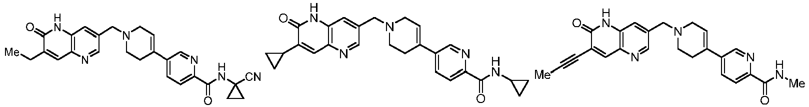 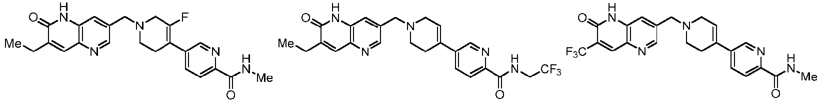 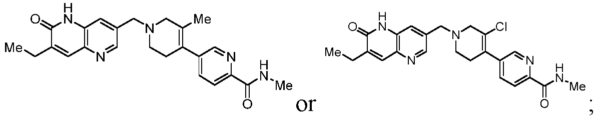 the acid in the acid addition salt is selected from hydroxyethylsulfonic acid, hydrochloric acid, sulfuric acid, 1,5-naphthalenedisulfonic acid, methanesulfonic acid, hydrobromic acid, ethanesulfonic acid, phosphoric acid, benzenesulfonic acid, oxalic acid, maleic acid, adipic acid, hydrochloric acid, citric acid, malonic acid, L-malic acid, pamoic acid, p-toluenesulfonic acid, or fumaric acid; preferably hydrochloric acid, sulfuric acid, methanesulfonic acid, hydrobromic acid, or p-toluenesulfonic acid.

3. The acid addition salt of the compound or a stereoisomer thereof according to any one of claims 1 to 2, **characterized in that** the number of the acid in the acid addition salt is 0.2-3; preferably 0.2, 0.5, 1, 1.5, 2, 2.5, or 3; more preferably 0.5, 1, 2, or 3, further preferably 1.

4. The acid addition salt of the compound or a stereoisomer thereof according to any one of claims 1 to 3, **characterized in that** the acid addition salt is a hydrate or an anhydrate; when the acid addition salt is a hydrate, the water is preferably crystal water or channel water; and the number of water molecules is 0.2-3; preferably 0.2, 0.5, 1, 1.5, 2, 2.5, or 3; more preferably 2.

5. A crystalline form of the acid addition salt of the compound or a stereoisomer thereof according to claim 4, **characterized in that**
preferably, the crystalline form is selected from:
a crystalline form of an acid addition salt of 1'-((7-ethyl-6-carbonyl-5,6-dihydro-1,5-diazanaphth-3-yl)methyl)-N-methyl-1',2',3',6'-tetrahydro-[3,4'-bipyridinyl]-6-carboxamide,
a crystalline form of an acid addition salt of N-cyclopropyl-1'-((7-ethyl-6-carbonyl-5,6-dihydro-1,5-diazanaphth-3-yl)methyl)-1',2',3',6'-tetrahydro-[3,4'-bipyridinyl]-6-carboxamide,
a crystalline form of an acid addition salt of 1'-((7-ethyl-6-carbonyl-5,6-dihydro-1,5-diazanaphth-3-yl)methyl)-2-methoxy-N-methyl-1',2',3',6'-tetrahydro-[3,4'-bipyridinyl]-6-carboxamide,
a crystalline form of an acid addition salt monohydrate of 1'-((7-ethyl-6-carbonyl-5,6-dihydro-1,5-diazanaphth-3-yl)methyl)-N-methyl-1',2',3',6'-tetrahydro-[3,4'-bipyridinyl]-6-carboxamide,
a crystalline form of an acid addition salt monohydrate of N-cyclopropyl-1'-((7-ethyl-6-carbonyl-5,6-dihydro-1,5-diazanaphth-3-yl)methyl)-1',2',3',6'-tetrahydro-[3,4'-bipyridinyl]-6-carboxamide,
a crystalline form of an acid addition salt monohydrate of 1'-((7-ethyl-6-carbonyl-5,6-dihydro-1,5-diazanaphth-3-yl)methyl)-2-methoxy-N-methyl-1',2',3',6'-tetrahydro-[3,4'-bipyridinyl]-6-carboxamide,
a crystalline form of an acid addition salt dihydrate of 1'-((7-ethyl-6-carbonyl-5,6-dihydro-1,5-diazanaphth-3-yl)methyl)-N-methyl-1',2',3',6'-tetrahydro-[3,4'-bipyridinyl]-6-carboxamide,
a crystalline form of an acid addition salt dihydrate of N-cyclopropyl-1'-((7-ethyl-6-carbonyl-5,6-dihydro-1,5-diazanaphth-3-yl)methyl)-1',2',3',6'-tetrahydro-[3,4'-bipyridinyl]-6-carboxamide, or
a crystalline form of an acid addition salt dihydrate of 1'-((7-ethyl-6-carbonyl-5,6-dihydro-1,5-diazanaphth-3-yl)methyl)-2-methoxy-N-methyl-1',2',3',6'-tetrahydro-[3,4'-bipyridinyl]-6-carboxamide;
more preferably, the acid addition salt in the crystalline form of the acid addition salt, the crystalline form of the acid addition salt monohydrate, or the crystalline form of the acid addition salt dihydrate is a hydroxyethylsulfonate, a sulfate, a hydrochloride, a 1,5-naphthalenedisulfonate, a methanesulfonate, an ethanesulfonate, a hydrobromide, a phosphate, a benzenesulfonate, an oxalate, a maleate, an adipate, a hydrochloride, a citrate, a malonate, an L-malate, a pamoate, a p-toluenesulfonate, or a fumarate.

6. The crystalline form according to claim 5, **characterized in that** the crystalline form of the acid addition salt of 1'-((7-ethyl-6-carbonyl-5,6-dihydro-1,5-diazanaphth-3-yl)methyl)-N-methyl-1',2',3',6'-tetrahydro-[3,4'-bipyridinyl]-6-carboxamide is hydrochloride crystalline Form A, sulfate crystalline Form A, methanesulfonate crystalline Form A, p-toluenesulfonate crystalline Form A, p-toluenesulfonate crystalline Form B, p-toluenesulfonate crystalline Form C, p-toluenesulfonate dihydrate crystalline Form A, and benzenesulfonate crystalline Form A;
preferably, wherein
the X-ray powder diffraction pattern of the hydrochloride crystalline Form A has a diffraction peak at 2θ of 4.6 ± 0.2°; or has a diffraction peak at 2θ of 7.0 ± 0.2°; or has a diffraction peak at 2θ of 9.2 ± 0.2°; or has a diffraction peak at 2θ of 13.8 ± 0.2°; or has a diffraction peak at 2θ of 15.0 ± 0.2°; or has a diffraction peak at 2θ of 16.1 ± 0.2°; or has a diffraction peak at 2θ of 18.2 ± 0.2°; or has a diffraction peak at 2θ of 20.8 ± 0.2°; or has a diffraction peak at 2θ of 22.4 ± 0.2°; or has a diffraction peak at 2θ of 25.2 ± 0.2°; or has a diffraction peak at 2θ of 28.1 ± 0.2°; preferably, the X-ray powder diffraction pattern comprises any 2-5, or 3-5, or 3-6, or 3-8, or 5-8, or 6-8, or 6-9, or 8-10, or 8-11 of said peaks, more preferably, comprises any 6, 7, 8, 9, 10, or 11 of said peaks;
the X-ray powder diffraction pattern of the sulfate crystalline Form A has a diffraction peak at 2θ of 6.5 ± 0.2°; or has a diffraction peak at 2θ of 9.7 ± 0.2°; or has a diffraction peak at 2θ of 14.3 ± 0.2°; or has a diffraction peak at 2θ of 16.1 ± 0.2°; or has a diffraction peak at 2θ of 18.6 ± 0.2°; or has a diffraction peak at 2θ of 19.3 ± 0.2°; or has a diffraction peak at 2θ of 19.7 ± 0.2°; or has a diffraction peak at 2θ of 22.0 ± 0.2°; or has a diffraction peak at 2θ of 22.5 ± 0.2°; or has a diffraction peak at 2θ of 23.6 ± 0.2°; or has a diffraction peak at 2θ of 25.5 ± 0.2°; or has a diffraction peak at 2θ of 25.9 ± 0.2°; or has a diffraction peak at 2θ of 29.2 ± 0.2°; preferably, the X-ray powder diffraction pattern comprises any 2-5, or 3-5, or 3-6, or 3-8, or 5-8, or 6-8, or 7-9, or 8-10, or 9-10, or 10-12, or 11-13 of said peaks, more preferably, comprises any 6, 7, 8, 9, 10, 11, 12, or 13 of said peaks;
the X-ray powder diffraction pattern of the methanesulfonate crystalline Form A has a diffraction peak at 2θ of 5.2 ± 0.2°; or has a diffraction peak at 2θ of 7.5 ± 0.2°; or has a diffraction peak at 2θ of 7.9 ± 0.2°; or has a diffraction peak at 2θ of 8.6 ± 0.2°; or has a diffraction peak at 2θ of 12.3 ± 0.2°; or has a diffraction peak at 2θ of 15.8 ± 0.2°; or has a diffraction peak at 2θ of 17.1 ± 0.2°; or has a diffraction peak at 2θ of 17.6 ± 0.2°; or has a diffraction peak at 2θ of 19.8 ± 0.2°; or has a diffraction peak at 2θ of 20.1 ± 0.2°; or has a diffraction peak at 2θ of 21.8 ± 0.2°; or has a diffraction peak at 2θ of 22.6 ± 0.2°; or has a diffraction peak at 2θ of 25.9 ± 0.2°; or has a diffraction peak at 2θ of 26.6 ± 0.2°; or has a diffraction peak at 2θ of 27.4 ± 0.2°; preferably, the X-ray powder diffraction pattern comprises any 2-5, or 3-5, or 3-6, or 3-8, or 5-8, or 6-8, or 7-9, or 8-10, or 9-10, or 10-12, or 11-13, or 12-14, or 14-15 of said peaks, more preferably, comprises any 6, 7, 8, 9, 10, 11, 12, 13, 14, or 15 of said peaks;
the X-ray powder diffraction pattern of the p-toluenesulfonate crystalline Form A has a diffraction peak at 2θ of 5.3 ± 0.2°; or has a diffraction peak at 2θ of 13.8 ± 0.2°; or has a diffraction peak at 2θ of 15.7 ± 0.2°; or has a diffraction peak at 2θ of 15.9 ± 0.2°; or has a diffraction peak at 2θ of 18.2 ± 0.2°; or has a diffraction peak at 2θ of 19.7 ± 0.2°; or has a diffraction peak at 2θ of 23.2 ± 0.2°; or has a diffraction peak at 2θ of 24.1 ± 0.2°; preferably, the X-ray powder diffraction pattern comprises any 2-5, or 3-5, or 3-6, or 3-8, or 5-8, or 6-8 of said peaks, more preferably, comprises any 6, 7, or 8 of said peaks;
the X-ray powder diffraction pattern of the p-toluenesulfonate crystalline Form B has a diffraction peak at 2θ of 4.7 ± 0.2°; or has a diffraction peak at 2θ of 5.2 ± 0.2°; or has a diffraction peak at 2θ of 13.8 ± 0.2°; or has a diffraction peak at 2θ of 14.3 ± 0.2°; or has a diffraction peak at 2θ of 18.1 ± 0.2°; or has a diffraction peak at 2θ of 18.7 ± 0.2°; or has a diffraction peak at 2θ of 23.1 ± 0.2°; or has a diffraction peak at 2θ of 25.3 ± 0.2°; preferably, the X-ray powder diffraction pattern comprises any 2-5, or 3-5, or 3-6, or 3-8, or 5-8, or 6-8 of said peaks, more preferably, comprises any 6, 7, or 8 of said peaks;
the X-ray powder diffraction pattern of the p-toluenesulfonate crystalline Form C has a diffraction peak at 2θ of 4.6 ± 0.2°; or has a diffraction peak at 2θ of 13.6 ± 0.2°; or has a diffraction peak at 2θ of 14.3 ± 0.2°; or has a diffraction peak at 2θ of 18.6 ± 0.2°; or has a diffraction peak at 2θ of 19.4 ± 0.2°; or has a diffraction peak at 2θ of 23.1 ± 0.2°; or has a diffraction peak at 2θ of 25.2 ± 0.2°; preferably, the X-ray powder diffraction pattern comprises any 2-5, or 3-5, or 3-6, or 3-7, or 5-7, or 6-7 of said peaks, more preferably, comprises any 6 or 7 of said peaks;
the X-ray powder diffraction pattern of the p-toluenesulfonate dihydrate crystalline Form A has a diffraction peak at 2θ of 3.3 ± 0.2°; or has a diffraction peak at 2θ of 6.6 ± 0.2°; or has a diffraction peak at 2θ of 9.9 ± 0.2°; or has a diffraction peak at 2θ of 13.3 ± 0.2°; or has a diffraction peak at 2θ of 13.7 ± 0.2°; or has a diffraction peak at 2θ of 14.2 ± 0.2°; or has a diffraction peak at 2θ of 16.9 ± 0.2°; or has a diffraction peak at 2θ of 23.3 ± 0.2°; or has a diffraction peak at 2θ of 24.9 ± 0.2°; preferably, the X-ray powder diffraction pattern comprises any 2-5, or 3-5, or 3-6, or 3-8, or 5-8, or 6-8, or 6-9, or 7-9 of said peaks, more preferably, comprises any 6, 7, 8, or 9 of said peaks;
the X-ray powder diffraction pattern of the benzenesulfonate crystalline Form A has a diffraction peak at 2θ of 5.3 ± 0.2°; or has a diffraction peak at 2θ of 9.1 ± 0.2°; or has a diffraction peak at 2θ of 13.9 ± 0.2°; or has a diffraction peak at 2θ of 15.7 ± 0.2°; or has a diffraction peak at 2θ of 18.9 ± 0.2°; or has a diffraction peak at 2θ of 23.3 ± 0.2°; or has a diffraction peak at 2θ of 23.9 ± 0.2°; or has a diffraction peak at 2θ of 26.1 ± 0.2°; preferably, the X-ray powder diffraction pattern comprises any 2-5, or 3-5, or 3-6, or 3-8, or 5-8, or 6-8 of said peaks, more preferably, comprises any 6, 7, or 8 of said peaks.

7. The crystalline form according to claim 6, **characterized in that**
the X-ray powder diffraction pattern of the hydrochloride crystalline Form A comprises at least one or more, preferably two, more preferably three of diffraction peaks at 2θ of 9.2 ± 0.2°, 13.8 ± 0.2°, and 15.0 ± 0.2°; and optionally further comprises at least one, preferably two or three of diffraction peaks at 2θ of 4.6 ± 0.2°, 7.0 ± 0.2°, and 20.8 ± 0.2°;
the X-ray powder diffraction pattern of the sulfate crystalline Form A comprises at least one or more, preferably two, more preferably three of diffraction peaks at 2θ of 6.5 ± 0.2°, 9.7 ± 0.2°, 16.1 ± 0.2°, and 23.6 ± 0.2°; and optionally further comprises at least one, preferably two or three of diffraction peaks at 2θ of 18.6 ± 0.2°, 19.3 ± 0.2°, 25.5 ± 0.2°, and 25.9 ± 0.2°;
the X-ray powder diffraction pattern of the methanesulfonate crystalline Form A comprises at least one or more, preferably two, more preferably three of diffraction peaks at 2θ of 5.2 ± 0.2°, 7.5 ± 0.2°, 15.8 ± 0.2°, 20.1 ± 0.2°, and 22.6 ± 0.2°; and optionally further comprises at least one, preferably two or three of diffraction peaks at 2θ of 7.9 ± 0.2°, 8.6 ± 0.2°, 12.3 ± 0.2°, 17.1 ± 0.2°, 17.6 ± 0.2°, 19.8 ± 0.2°, 25.9 ± 0.2°, 26.6 ± 0.2°, and 27.4 ± 0.2°;
the X-ray powder diffraction pattern of the p-toluenesulfonate crystalline Form A comprises at least one or more, preferably two, more preferably three of diffraction peaks at 2θ of 5.3 ± 0.2°, 15.9 ± 0.2°, and 18.2 ± 0.2°; and optionally further comprises at least one, preferably two or three of diffraction peaks at 2θ of 13.8 ± 0.2°, 15.7 ± 0.2°, 19.7 ± 0.2°, 23.2 ± 0.2°, and 24.1 ± 0.2°;
the X-ray powder diffraction pattern of the p-toluenesulfonate crystalline Form B comprises at least one or more, preferably two, more preferably three of diffraction peaks at 2θ of 4.7 ± 0.2°, 14.3 ± 0.2°, 23.1 ± 0.2°, and 25.3 ± 0.2°; and optionally further comprises at least one, preferably two or three of diffraction peaks at 2θ of 5.2 ± 0.2°, 13.8 ± 0.2°, 18.1 ± 0.2°, and 18.7 ± 0.2°;
the X-ray powder diffraction pattern of the p-toluenesulfonate crystalline Form C comprises at least one or more, preferably two, more preferably three of diffraction peaks at 2θ of 4.6 ± 0.2°, 14.3 ± 0.2°, 18.6 ± 0.2°, and 25.2 ± 0.2°; and optionally further comprises at least one, preferably two or three of diffraction peaks at 2θ of 9.1 ± 0.2°, 13.6 ± 0.2°, 16.3 ± 0.2°, 19.4 ± 0.2°, and 23.1 ± 0.2°;
the X-ray powder diffraction pattern of the p-toluenesulfonate dihydrate crystalline Form A comprises at least one or more, preferably two, more preferably three of diffraction peaks at 2θ of 3.3 ± 0.2°, 6.6 ± 0.2°, 9.9 ± 0.2°, and 13.3 ± 0.2°; and optionally further comprises at least one, preferably two, three, or four of diffraction peaks at 2θ of 13.7 ± 0.2°, 14.2 ± 0.2°, 16.9 ± 0.2°, 23.3 ± 0.2°, and 24.9 ± 0.2°; and
the X-ray powder diffraction pattern of the benzenesulfonate crystalline Form A comprises at least one or more, preferably two, more preferably three of diffraction peaks at 2θ of 5.3 ± 0.2°, 9.1 ± 0.2°, 13.9 ± 0.2°, 15.7 ± 0.2°, and 26.1 ± 0.2°; and optionally further comprises at least one, preferably two or three of diffraction peaks at 2θ of 18.9 ± 0.2°, 23.3 ± 0.2°, and 23.9 ± 0.2°.

8. The crystalline form according to claim 6 or 7, **characterized in that**
the X-ray powder diffraction pattern of the hydrochloride crystalline Form A optionally further comprises one or more of diffraction peaks at 2θ of 16.1 ± 0.2°, 18.2 ± 0.2°, 22.4 ± 0.2°, 25.2 ± 0.2°, and 28.1 ± 0.2°; preferably, at least comprises any 2-3, or 4-5 of said peaks; further preferably, comprises any 2, 3, 4, or 5 of said peaks; and
the X-ray powder diffraction pattern of the sulfate crystalline Form A optionally further comprises one or more of diffraction peaks at 2θ of 11.6 ± 0.2°, 12.9 ± 0.2°, 13.7 ± 0.2°, 20.4 ± 0.2°, and 20.9 ± 0.2°; preferably, at least comprises any 2-3, or 4-5 of said peaks; further preferably, comprises any 2, 3, 4, or 5 of said peaks; and
the X-ray powder diffraction pattern of the methanesulfonate crystalline Form A optionally further comprises one or more of diffraction peaks at 2θ of 4.6 ± 0.2°, 9.1 ± 0.2°, 10.8 ± 0.2°, 14.6 ± 0.2°, 15.1 ± 0.2°, 16.7 ± 0.2°, 20.6 ± 0.2°, 24.4 ± 0.2°, and 28.2 ± 0.2°; preferably, at least comprises any 2-3, or 4-5 of said peaks; further preferably, comprises any 2, 3, 4, or 5 of said peaks; and
the X-ray powder diffraction pattern of the p-toluenesulfonate crystalline Form A optionally further comprises one or more of diffraction peaks at 2θ of 11.3 ± 0.2°, 19.1 ± 0.2°, 21.6 ± 0.2°, 25.3 ± 0.2°, and 26.1 ± 0.2°; preferably, at least comprises any 2-3, or 4-5 of said peaks; further preferably, comprises any 2, 3, 4, or 5 of said peaks; and
the X-ray powder diffraction pattern of the p-toluenesulfonate crystalline Form B optionally further comprises one or more of diffraction peaks at 2θ of 9.2 ± 0.2°, 16.4 ± 0.2°, 21.7 ± 0.2°, 23.5 ± 0.2°, 25.7 ± 0.2°, and 28.1 ± 0.2°; preferably, at least comprises any 2-3, or 4-5 of said peaks; further preferably, comprises any 2, 3, 4, or 5 of said peaks; and
the X-ray powder diffraction pattern of the p-toluenesulfonate crystalline Form C optionally further comprises one or more of 2 diffraction peaks at θ of 17.7 ± 0.2°, 21.7 ± 0.2°, 23.5 ± 0.2°, 25.5 ± 0.2°, 26.7 ± 0.2°, and 28.1 ± 0.2°; preferably, at least comprises any 2-3, or 4-5 of said peaks; further preferably, comprises any 2, 3, 4, or 5 of said peaks; and
the X-ray powder diffraction pattern of the p-toluenesulfonate dihydrate crystalline Form A optionally further comprises one or more of diffraction peaks at 2θ of 11.4 ± 0.2°, 14.4 ± 0.2°, 19.2 ± 0.2°, 20.0 ± 0.2°, 22.7 ± 0.2°, 22.9 ± 0.2°, and 26.9 ± 0.2°; preferably, at least comprises any 2-3, or 4-5 of said peaks; further preferably, comprises any 2, 3, 4, or 5 of said peaks; and
the X-ray powder diffraction pattern of the benzenesulfonate crystalline Form A optionally further comprises one or more of diffraction peaks at 2θ of 10.5 + 0.2°, 11.3 ± 0.2°, 18.2 ± 0.2°, 22.8 ± 0.2°, 25.0 ± 0.2°, 28.2 ± 0.2°, and 32.2 ± 0.2°; preferably, at least comprises any 2-3, or 4-5 of said peaks; further preferably, comprises any 2, 3, 4, or 5 of said peaks.

9. The crystalline form according to claim 6, **characterized in that**
the X-ray powder diffraction pattern of the hydrochloride crystalline Form A comprises one or more of diffraction peaks at 2θ of 4.6 ± 0.2°, 7.0 ± 0.2°, 9.2 ± 0.2°, 13.8 ± 0.2°, 15.0 ± 0.2°, 16.1 ± 0.2°, 18.2 ± 0.2°, 20.8 ± 0.2°, 22.4 ± 0.2°, 25.2 ± 0.2°, and 28.1 ± 0.2°; preferably, comprises any 4, 5, 6, 8, or 10 of said peaks;
the X-ray powder diffraction pattern of the sulfate crystalline Form A comprises one or more of diffraction peaks at 2θ of 6.5 ± 0.2°, 9.7 ± 0.2°, 11.6 ± 0.2°, 12.9 ± 0.2°, 13.7 ± 0.2°, 14.3 ± 0.2°, 16.1 ± 0.2°, 18.6 ± 0.2°, 19.3 ± 0.2°, 19.7 ± 0.2°, 20.4 ± 0.2°, 20.9 ± 0.2°, 22.0 ± 0.2°, 22.5 ± 0.2°, 23.6 ± 0.2°, 25.5 ± 0.2°, 25.9 ± 0.2°, and 29.2 ± 0.2°; preferably, comprises any 4, 5, 6, 8, or 10 of said peaks;
the X-ray powder diffraction pattern of the methanesulfonate crystalline Form A comprises one or more of diffraction peaks at 2θ of 5.2 ± 0.2°, 7.5 ± 0.2°, 7.9 ± 0.2°, 8.6 ± 0.2°, 10.8 ± 0.2°, 12.3 ± 0.2°, 15.8 ± 0.2°, 17.1 ± 0.2°, 17.6 ± 0.2°, 19.8 ± 0.2°, 20.1 ± 0.2°, 20.6 ± 0.2°, 22.6 ± 0.2°, 24.4 ± 0.2°, 25.9 ± 0.2°, 26.6 ± 0.2°, 27.4 ± 0.2°, and 28.2 ± 0.2°; preferably, comprises any 4, 5, 6, 8, or 10 of said peaks;
the X-ray powder diffraction pattern of the p-toluenesulfonate crystalline Form A comprises one or more of diffraction peaks at 2θ of 5.3 ± 0.2°, 11.3 ± 0.2°, 13.8 ± 0.2°, 15.7 ± 0.2°, 15.9 ± 0.2°, 18.2 ± 0.2°, 19.1 ± 0.2°, 19.7 ± 0.2°, 21.6 ± 0.2°, 23.2 ± 0.2°, 25.3 ± 0.2°, and 26.1 ± 0.2°; preferably, comprises any 4, 5, 6, 8, or 10 of said peaks;
the X-ray powder diffraction pattern of the p-toluenesulfonate crystalline Form B comprises one or more of diffraction peaks at 2θ of 4.7 ± 0.2°, 5.2 ± 0.2°, 9.2 ± 0.2°, 13.8 ± 0.2°, 14.3 ± 0.2°, 16.4 ± 0.2°, 18.1 ± 0.2°, 18.7 ± 0.2°, 21.7 ± 0.2°, 23.1 ± 0.2°, 23.5 ± 0.2°, 25.3 ± 0.2°, 25.7 ± 0.2°, and 28.1 ± 0.2°; preferably, comprises any 4, 5, 6, 8, or 10 of said peaks;
the X-ray powder diffraction pattern of the p-toluenesulfonate crystalline Form C comprises one or more of diffraction peaks at 2θ of 4.6 ± 0.2°, 9.1 ± 0.2°, 13.6 ± 0.2°, 14.3 ± 0.2°, 16.3 ± 0.2°, 17.7 ± 0.2°, 18.6 ± 0.2°, 19.4 ± 0.2°, 21.7 ± 0.2°, 23.1 ± 0.2°, 23.5 ± 0.2°, 25.2 ± 0.2°, 25.5 ± 0.2°, 26.7 ± 0.2°, and 28.1 ± 0.2°; preferably, comprises any 4, 5, 6, 8, or 10 of said peaks;
the X-ray powder diffraction pattern of the p-toluenesulfonate dihydrate crystalline Form A comprises one or more of diffraction peaks at 2θ of 3.3 ± 0.2°, 6.6 ± 0.2°, 9.9 ± 0.2°, 11.4 ± 0.2°, 13.3 ± 0.2°, 13.7 ± 0.2°, 14.2 ± 0.2°, 14.4 ± 0.2°, 16.9 ± 0.2°, 19.2 ± 0.2°, 20.0 ± 0.2°, 22.7 ± 0.2°, 22.9 ± 0.2°, 23.3 ± 0.2°, 24.9 ± 0.2°, and 26.9 ± 0.2°; preferably, comprises any 4, 5, 6, 8, or 10 of said peaks;
the X-ray powder diffraction pattern of the benzenesulfonate crystalline Form A comprises one or more of diffraction peaks at 2θ of 5.3 ± 0.2°, 9.1 ± 0.2°, 10.5 ± 0.2°, 11.3 ± 0.2°, 13.9 ± 0.2°, 15.7 ± 0.2°, 18.2 ± 0.2°, 18.9 ± 0.2°, 22.8 ± 0.2°, 23.3 ± 0.2°, 23.9 ± 0.2°, 25.0 ± 0.2°, 26.1 ± 0.2°, 28.2 ± 0.2°, and 32.2 ± 0.2°; preferably, comprises any 4, 5, 6, 8, or 10 of said peaks.

10. The crystalline form according to claim 6, **characterized in that**
the X-ray powder diffraction pattern of the hydrochloride crystalline Form A has 2θ as shown in Table 1; or the X-ray powder diffraction pattern of the sulfate crystalline Form A has 2θ as shown in Table 2; or the X-ray powder diffraction pattern of the methanesulfonate crystalline Form A has 2θ as shown in Table 3; or the X-ray powder diffraction pattern of the p-toluenesulfonate crystalline Form A has 2θ as shown in Table 4; or the X-ray powder diffraction pattern of the p-toluenesulfonate crystalline Form B has 2θ as shown in Table 5; or the X-ray powder diffraction pattern of the p-toluenesulfonate crystalline Form C has 2θ as shown in Table 6; or the X-ray powder diffraction pattern of the p-toluenesulfonate dihydrate crystalline Form A has 2θ as shown in Table 7; or the X-ray powder diffraction pattern of the benzenesulfonate crystalline Form A has 2θ as shown in Table 8.

11. The crystalline form according to claim 6, **characterized in that**
the X-ray powder diffraction pattern of the hydrochloride crystalline Form A is substantially as shown in FIG. 3; or the X-ray powder diffraction pattern of the sulfate crystalline Form A is substantially as shown in FIG. 5; or the X-ray powder diffraction pattern of the methanesulfonate crystalline Form A is substantially as shown in FIG. 7; or the X-ray powder diffraction pattern of the p-toluenesulfonate crystalline Form A is substantially as shown in FIG. 9; or the X-ray powder diffraction pattern of the p-toluenesulfonate crystalline Form B is substantially as shown in FIG. 11; or the X-ray powder diffraction pattern of the p-toluenesulfonate crystalline Form C is substantially as shown in FIG. 13; or the X-ray powder diffraction pattern of the p-toluenesulfonate dihydrate crystalline Form A is substantially as shown in FIG. 15; or the X-ray powder diffraction pattern of the benzenesulfonate crystalline Form A is substantially as shown in FIG. 18;
or a DSC thermogram of the hydrochloride crystalline Form A is substantially as shown in FIG. 4; or a DSC thermogram of the sulfate crystalline Form A is substantially as shown in FIG. 6; or a DSC thermogram of the methanesulfonate crystalline Form A is substantially as shown in FIG. 8; or a DSC thermogram of the p-toluenesulfonate crystalline Form A is substantially as shown in FIG. 10; or a DSC thermogram of the p-toluenesulfonate crystalline Form B is substantially as shown in FIG. 12; or a DSC thermogram of the p-toluenesulfonate crystalline Form C is substantially as shown in FIG. 14; or a DSC thermogram of the p-toluenesulfonate dihydrate crystalline Form A is substantially as shown in FIG. 16; or a DSC thermogram of the benzenesulfonate crystalline Form A is substantially as shown in FIG. 19;
or a TGA thermogram of the p-toluenesulfonate dihydrate crystalline Form A is substantially as shown in FIG. 17.

12. The crystalline form according to claim 11, **characterized in that**
the 2θ positions of the top ten most intense diffraction peaks in the X-ray powder diffraction patterns of the hydrochloride crystalline Form A, the sulfate crystalline Form A, the methanesulfonate crystalline Form A, the p-toluenesulfonate **crystalline** Form A, the p-toluenesulfonate crystalline Form B, the p-toluenesulfonate crystalline Form C, the p-toluenesulfonate dihydrate crystalline Form A, and the benzenesulfonate crystalline Form A have a deviation of ±0.2° to ±0.5°, preferably ± 0.2° to ±0.3°, most preferably ±0.2° from the corresponding peak positions in the X-ray powder diffraction patterns.

13. A method for preparing the acid addition salt of the compound or a stereoisomer thereof according to any one of claims 1 to 4, **characterized in that** the method comprises the following steps:
1) weighing an appropriate amount of a free base and dissolving it in a benign solvent;
2) weighing an appropriate amount of a counter-ion acid and dissolving it in an organic solvent, wherein the amount of the counter-ion acid is preferably 1.2 equivalents;
3) combining the above two solutions, and stirring for precipitation, or adding a poor solvent dropwise with stirring for precipitation; and
4) obtaining the acid addition salt by rapid centrifugation or static drying,
wherein
the benign solvent is selected from one or more of methanol, acetone, ethyl acetate, acetonitrile, ethanol, 88% acetone, tetrahydrofuran, 2-methyltetrahydrofuran, dichloromethane, 1,4-dioxane, benzene, toluene, isopropanol, n-butanol, isobutanol, N,N-dimethylformamide, N,N-dimethylacetamide, n-propanol, tert-butanol, 2-butanone, 3-pentanone, or N-methylpyrrolidone; preferably one or more of N-methylpyrrolidone, methanol, dichloromethane, or anhydrous ethanol;
the organic solvent is selected from methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-butanone, 3-pentanone, heptane, methyl tert-butyl ether, isopropyl ether, 1,4-dioxane, tert-butyl alcohol, or N,N-dimethylformamide; preferably methanol, ethanol, or acetonitrile; the above good solvent and organic solvent are necessarily mutually soluble when used;
the poor solvent is selected from one or more of heptane, cyclohexane, n-hexane, n-pentane, water, ethyl acetate, methyl tert-butyl ether, toluene, or isopropyl ether; preferably one or more of water, heptane, methyl tert-butyl ether, or isopropyl ether; and
the counter-ion acid is selected from hydrochloric acid, sulfuric acid, nitric acid, hydrobromic acid, hydrofluoric acid, hydroiodic acid, phosphoric acid, the organic acid is selected from 2,5-dihydroxybenzoic acid, 1-hydroxy-2-naphthoic acid, acetic acid, dichloroacetic acid, trichloroacetic acid, acetohydroxamic acid, adipic acid, benzenesulfonic acid, 4-chlorobenzenesulfonic acid, benzoic acid, 4-acetylaminobenzoic acid, 4-aminobenzoic acid, decanoic acid, hexanoic acid, octanoic acid, cinnamic acid, citric acid, cyclohexylsulfamic acid, camphorsulfonic acid, aspartic acid, camphanic acid, gluconic acid, glucuronic acid, glutamic acid, isoascorbic acid, lactic acid, malic acid, mandelic acid, pyroglutamic acid, tartaric acid, dodecylsulfuric acid, dibenzoyltartaric acid, ethane-1,2-disulfonic acid, ethanesulfonic acid, formic acid, fumaric acid, galactonic acid, gentisuric acid, glutaric acid, 2-ketoglutaric acid, glycolic acid, hippuric acid, hydroxyethylsulfonic acid, lactobionic acid, ascorbic acid, aspartic acid, lauric acid, camphanic acid, maleic acid, malonic acid, methanesulfonic acid, 1,5-naphthalenedisulfonic acid, naphthalene-2-sulfonic acid, nicotinic acid, oleic acid, orotic acid, oxalic acid, palmitic acid, pamoic acid, propanoic acid, salicylic acid, 4-aminosalicylic acid, sebacic acid, stearic acid, succinic acid, thiocyanic acid, undecylenoic acid, trifluoroacetic acid, benzenesulfonic acid, p-methylbenzenesulfonic acid, or L-malic acid; preferably fumarate, p-toluenesulfonate, or succinate; most preferably p-toluenesulfonate.

14. A method for preparing the crystalline form of the acid addition salt of the compound or a stereoisomer thereof according to any one of claims 5 to 12, **characterized in that** the method is Method I, II, or III;
Method I comprises the following steps:
1) suspending the compound in a poor solvent;
2) adding a counter-ion acid, wherein the amount of the counter-ion acid is preferably 1.2 equivalents; and the counter-ion acid may be dissolved in an organic solvent;
3) stirring to dissolve, followed by continued stirring for precipitation or dropwise addition of a poor solvent with stirring to induce precipitation; and
4) isolating to obtain the anhydrous crystalline form;
Method II comprises the following step:
converting the anhydrous crystalline form obtained from Method I via crystal transformation ;
Method III comprises the following step:
suspending the anhydrous crystalline form obtained from Method I in water, and isolating to obtain the hydrate crystalline form,
wherein
the poor solvent is selected from one or more of acetone, ethyl acetate, isopropyl acetate, acetonitrile, ethanol, 88% acetone, tetrahydrofuran, 2-methyltetrahydrofuran, dichloromethane, 1,4-dioxane, benzene, toluene, isopropanol, n-butanol, isobutanol, N,N-dimethylformamide, N,N-dimethylacetamide, n-propanol, tert-butanol, 2-butanone or 3-pentanone, methyl tert-butyl ether, or water; preferably one or more of acetone, ethanol, tetrahydrofuran, acetonitrile, or toluene;
the organic solvent is selected from methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-butanone, 3-pentanone, heptane, methyl tert-butyl ether, isopropyl ether, 1,4-dioxane, tert-butyl alcohol, or N,N-dimethylformamide; preferably methanol, ethanol, or acetonitrile; and
the counter-ion acid is selected from hydrochloric acid, sulfuric acid, nitric acid, hydrobromic acid, hydrofluoric acid, hydroiodic acid, phosphoric acid, the organic acid is selected from 2,5-dihydroxybenzoic acid, 1-hydroxy-2-naphthoic acid, acetic acid, dichloroacetic acid, trichloroacetic acid, acetohydroxamic acid, adipic acid, benzenesulfonic acid, 4-chlorobenzenesulfonic acid, benzoic acid, 4-acetylaminobenzoic acid, 4-aminobenzoic acid, decanoic acid, hexanoic acid, octanoic acid, cinnamic acid, citric acid, cyclohexylsulfamic acid, camphorsulfonic acid, aspartic acid, camphanic acid, gluconic acid, glucuronic acid, glutamic acid, isoascorbic acid, lactic acid, malic acid, mandelic acid, pyroglutamic acid, tartaric acid, dodecylsulfuric acid, dibenzoyltartaric acid, ethane-1,2-disulfonic acid, ethanesulfonic acid, formic acid, fumaric acid, galactonic acid, gentisuric acid, glutaric acid, 2-ketoglutaric acid, glycolic acid, hippuric acid, hydroxyethylsulfonic acid, lactobionic acid, ascorbic acid, aspartic acid, lauric acid, camphanic acid, maleic acid, malonic acid, methanesulfonic acid, 1,5-naphthalenedisulfonic acid, naphthalene-2-sulfonic acid, nicotinic acid, oleic acid, orotic acid, oxalic acid, palmitic acid, pamoic acid, propanoic acid, salicylic acid, 4-aminosalicylic acid, sebacic acid, stearic acid, succinic acid, thiocyanic acid, undecylenoic acid, trifluoroacetic acid, benzenesulfonic acid, p-methylbenzenesulfonic acid, or L-malic acid; preferably fumarate, p-toluenesulfonate, or succinate; most preferably p-toluenesulfonate.

15. A pharmaceutical composition, **characterized by** comprising a therapeutically effective amount of the acid addition salt of the compound or a stereoisomer thereof according to any one of claims 1 to 4 and/or the crystalline form of the acid addition salt of the compound or a stereoisomer thereof according to any one of claims 5 to 12, and one or more pharmaceutically acceptable carriers, diluents or excipients.

16. A use of the acid addition salt of the compound or a stereoisomer thereof according to any one of claims 1 to 4, the crystalline form of the acid addition salt of the compound or a stereoisomer thereof according to any one of claims 5 to 12, or the pharmaceutical composition according to claim 15 in the manufacture of a PARP inhibitor medicament, **characterized in that** the PARP is preferably PARP1.

17. A use of the acid addition salt of the compound or a stereoisomer thereof according to any one of claims 1 to 4, the crystalline form of the acid addition salt of the compound or a stereoisomer thereof according to any one of claims 5 to 12, or the pharmaceutical composition according to claim 15 in the manufacture of a medicament for treating cancer, ischemic diseases, or neurodegenerative diseases, preferably wherein the cancer is selected from breast cancer, ovarian cancer, pancreatic cancer, prostate cancer, hematological cancer, gastric cancer, colorectal cancer, gastrointestinal cancer, and lung cancer.
